# EUROPEAN PATENT APPLICATION

(11) **EP 3 984 535 A1**
(43) Date of publication of application: **20.04.2022**
(21) Application number: 20202297.6
(22) Date of filing: 16.10.2020
(51) Int. Cl.: A61K 31/4245, A61K 31/00, A61K 31/17, A61K 31/341, A61K 31/47, A61K 31/4709, A61P 31/00, A61P 35/00, A61P 37/04

(54) **NLRP3 ACTIVATORS FOR USE IN THE TREATMENT OF INFECTIOUS DISEASES OR CANCER BY ACTIVATING NLRP3 INFLAMMASOME**

(71) Applicant: Albert-Ludwigs-Universität Freiburg, 79098 Freiburg (DE)
(72) Inventor: Groß, Olaf, 79341 Kenzingen (DE); Neuwirt, Emilia, 79114 Freiburg (DE)
(74) Representative: Grindl, Wolfgang

(57) **Abstract**

The present invention relates to a compound or pharmaceutically acceceptable salts thereof, that modulates NLRP3 in that the NLRP3 inflammasome is activated. This invention further relates to the compounds and/or compositions for use in the prevention and treatment of a condition, disease or a disorder by activating NLRP3 inflammasome, wherein the disease is selected from a group comprising infectious diseases, or cancer. Moreover, the invention relates to the use of the present compounds for analyzing the activity of NLRP3 activation.

## Description

### Technical Field

The present invention relates to compounds or pharmaceutically acceceptable salts thereof, that activate the NLRP3 inflammasome. Moreover, TLR7 and/or TLR8 is not activated by these compounds. Moreover, the mitochondrial respiratory chain is not inhibited by these compounds. This invention further relates to the compounds and/or compositions for use in the prevention and treatment of a condition, a disease or a disorder by activating the NLRP3 inflammasome, wherein the disease is selected from a group comprising infectious diseases or cancer. Moreover, the invention relates to the use of the present compounds as an adjuvant for vaccination. Moreover, the invention relates to the use of the present compounds for analyzing the mechanisms and consequences of NLRP3 activation.

### Background of the Invention

The present invention provides compounds useful as modulators of the NLRP3 inflammasome. NLRP (Nucleotide-binding oligomerization domain, Leucine rich Repeat and Pyrin domain containing) proteins, are a subfamily of NLRs (NOD-like receptor), which play a role in the formation of inflammasomes.

Inflammasomes are intracellular multiprotein oligomers of the innate immune system responsible for the activation of inflammatory responses. Moreover, inflammasomes control the bioactivity of potent pro-inflammatory cytokines of the interleukin-1 (IL-1) family members such as IL-1β and induce a form of lytic, proinflammatory cell death known as pyroptosis. These effects of inflammasome activity are mediated by one of its components, the protease caspase-1. It directly mediates maturation of inactive pro-IL-1β and controls IL-1 release and subsequent pyroptosis by its pore-forming substrate Gasdermin-D (GSDMD). The inflammasome/IL-1 axis promotes protective inflammatory responses in the context of infection, but its dysregulation can lead to pathological inflammation. The different inflammasomes respond to a variety of endogenous and exogenous danger signals and thus contribute significantly to the defense against pathogens. Most inflammasomes are triggered by the presence of specific pathogen components within the cytoplasm, either by direct binding or by detecting their activity. Instead, the NLRP3 inflammasome appears to respond to cellular stress signals triggered by structurally diverse pathogens, endogenous danger signals, and environmental irritants.

NLRP3 is expressed predominantly in certain immune cells such as in macrophages as well as in epithelial cells. NLRP3 is a cytoplasmic sensor protein responding to sterile endogenous danger signals that are typically products of damaged cells such as extracellular ATP and crystalline uric acid. Upon activation, it nucleates an inflammasome by connecting to the protease caspase-1 via the adaptor protein ASC (encoded by *PYCARD*), thereby triggering an immune response. While other inflammasome nucleating receptors mainly react to pathogen-derived signals, NLRP3 is the main contributor to sterile inflammation.

A targeted activation of NLRP3 can induce an inflammasome-dependent anti-cancer immune response, e.g. by increasing the activity of cytotoxic cells against tumor cells. Thus, NLRP3 activators may be used for the treatment of malignancies.

Moreover, many of the currently known NLRP3 activators have additional unspecific or unrelated activities that are unfavorable. For example, bacterial ionophore toxins such as nigericin that allow potassium efflux thought to be involved in NLRP3 activation integrate into cellular membranes of any cell type. Furthermore, known small molecule NLRP3 activators such as the imidazoquinoline compounds imiquimod (also known as R837) or CL097 also activate toll-like receptor-7 (TLR7) and/or TLR8. TLR7/8 activation mechanistically explains the production of interferons and other proinflammatory cytokines like TNF and IL-6 and the antiviral activity triggered by this class of molecules, and it is classically thought to account for their anti-tumor effect. It was discovered that imiquimod and the similar molecule CL097 are not only NLRP3 activators and TLR7/8 ligands, but that they also inhibit cellular respiration by inhibiting the mitochondrial electron transport chain (Christina J. Groß, Ritu Mishra, Katharina S. Schneider, Guillaume Médard, Jennifer Wettmarshausen, Daniela C. Dittlein, Hexin Shi, Oliver Gorka, Paul-Albert Koenig, Stephan Fromm, Giovanni Magnani, Tamara Cikovic, Lara Hartjes, Joachim Smollich, Avril A.B. Robertson, Matthew A. Cooper, Marc Schmidt-Supprian, Michael Schuster, Kate Schroder, Petr Broz, Claudia Traidl-Hoffmann, Bruce Beutler, Bernhard Kuster, Jürgen Ruland, Sabine Schneider, Fabiana Perocchi and Olaf Groß, "K+ Efflux-Independent NLRP3 Inflammasome Activation by Small Molecules Targeting Mitochondria", Immunity, volume 45, issue 4, October 18, 2016, pp. 761-773). Both TLR7/8 activation and electron transport chain inhibition by imidazoquinolines might contribute to their antiviral and anti-tumor activity, but are also known to have adverse effects. Thus, there is still a need to provide selective activators for NLRP3 inflammasome.

In summary, there is a need for NLRP3 activators, which are more selective than previous NLRP3 activators and can boost immune responses and trigger tumoricidal activity of the immune system.

In addition, new small molecule NLRP3 activators are useful to study the biology of the NLRP3 inflammasome and thus make an important contribution to the fundamental understanding of the activation mechanisms of the NLRP3 inflammasome and its function in health and disease and enable the targeted development of specific drugs.

The present invention addresses these needs. In particular, the present invention provides compounds and compositions comprising these compounds, which can activate NLRP3 without activating TLR7 and/or TLR8 and/or the mitochondrial electron transport chain. Therefore, these compounds are useful in the prevention or treatment of diseases such as cancer as well as infectious diseases or in the context of vaccination to boost specific immune responses as an adjuvant by activating NLRP3. Moreover, these compounds are useful in methods for analyzing the biology of NLRP3 activation and signaling mechanisms involved.

### Summary of the Invention

According to the first aspect, the present invention relates to a compound for use in the prevention or treatment of a disease by activating the NLRP3 inflammasome, wherein the disease is selected from a group comprising infectious diseases or cancer, and
wherein the compound is a compound of Formula (I) 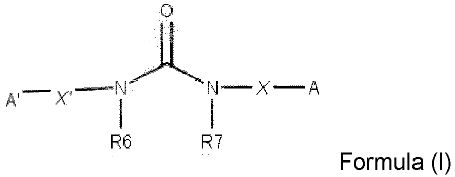 or a pharmaceutically acceceptable salt thereof, wherein:
A is an optionally substituted C₆-C₁₈ aryl; optionally substituted 5-20 membered heteroaryl; optionally substituted C₃-C₂₀ cycloalkyl; optionally substituted 3-20 membered cycloheteroalkyl,
and wherein X and X' are each independently present or not, wherein X and X' are each independently an optionally substituted C₁-C₁₀ alkyl group;
R⁶ and R⁷ are each independently hydrogen, an optionally substituted C₁-C₁₀ alkyl or R⁶ and R⁷ when taken together form an optionally substituted five- or six-membered heterocyclic ring.

In one embodiment, the compound of Formula (I) of the invention comprises X and X', wherein X and X' are each independently -(CH₂)ₘ-, wherein each m is independently an integer from 0 to 4; and wherein
R⁶ and R⁷ are each independently hydrogen, a C₁-C₁₀ alkyl, preferably wherein R⁶ and R⁷ are each hydrogen.

In one embodiment, the compound of Formula (I) of the invention comprises A and A', wherein A and A' are each independently optionally substituted benzene, imidazolidine, morpholine, piperazine, piperidine, pyrazolidine, pyrrolidone, quinuclidine, tetrahydrofuran, furan, pyrrolidine, pyridine, pyrazol, indolizine, quinoline, thiadiazol, oxadiazol, 1,2,4-oxadiazol, acridine, β-carbohne, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, cyclopropyl, cyclobutyl, cyclopentyl, cyclohexyl; cyclopropenyl, cyclobutenyl, cyclopentenyl, cyclohexenyl; cyclobutadienyl, cyclohexadienyl, carbazole, benzimidazole, imidazopyridine, benzoisoxazole, tetrahydrothiophene, benzofuran, benzoxazole, or benzthiozole,
preferably wherein A and A' are each independently optionally substituted quinoline, isoquinoline, indole, benzene, pyridine, pyrazole, pyrrol, furan or thiophene
more preferably wherein A and A' are each independently optionally substituted isoquinoline, quinoline or benzene.

In one embodiment, the compound of Formula (I) of the invention comprises X and X', wherein X and X' are each independently -(CH₂)ₘ-, wherein each m is independently an integer from 0 to 2, preferably 0 or 1, more preferably 0.

In one embodiment, the compound of Formula (I) of the invention comprises X and X', wherein X and X' are each independently -(CH₂)ₘ-, wherein each m is independently an integer from 0 to 2, preferably 0 or 1, more preferably 0,and R⁶ and R⁷ are each independently hydrogen, wherein A and A' are each independently optionally substituted isoquinoline, quinoline and benzene.

In one embodiment, the compound of Formula (I) of the invention is a compound of Formula (Ic): 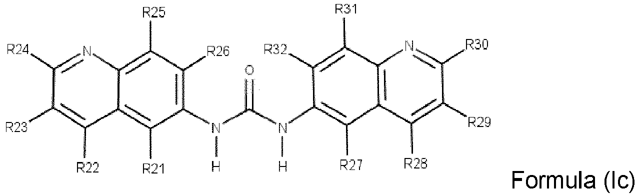 or 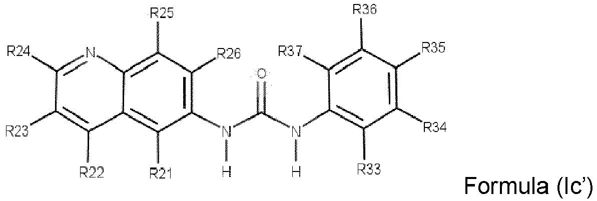
wherein R²¹ to R³⁷ are each independently selected from the group consisting of hydrogen, -F, -CI, -Br, -NO₂,-O-N=O, -N=O, -OH, -NH₂, -CH=NH, -N₃, -SH, -SO₃H, -SO₂H, -S(O)₂CH₃ -CN, - NC,-OCN, -NCO, -SCN, -NCS, -(P=O)(OH)₂, -O(P=O)(OH)₂, -(C=O)OH, -(C=O)NHOH, - NH(C=O)NH₂, -NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹² (SO₂)R¹¹, (SNO)R¹¹, (P=O)R¹¹R¹², (P=O)OR¹¹OR¹² or C(NR¹¹)R¹², -NHC(O)R¹¹, and - NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
preferably wherein R²¹ to R³⁷ are each independently selected from the group consisting of hydrogen, C₁-C₆ alkoxy, C₁-C₅ alkyl, -(C=O)CH₃, -CN, C₁-C₅ alkyl(SO₂)C₁-C₅ alkyl, preferably -CH₂(SO₂)CH₃, -CH₂(SO₂)CH₂CH₃, -CH₂CH₂(SO₂)CH₂CH₃; -(SO₂)C₁-C₅ alkyl, preferably - (SO₂)CH₃, -(SO₂)CH₂CH₃, -CF₃, -F, -CI, and -Br; or
more preferably wherein R²¹ to R³⁷ are each hydrogen.

In a second aspect the compound of Formula (I) of the invention is a compound of Formula (Ia), 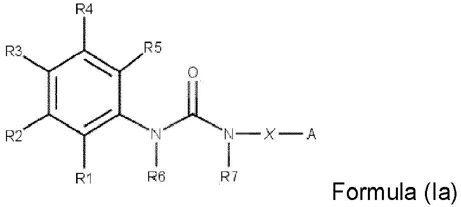 or a pharmaceutically acceceptable salt thereof, wherein:
A is preferably an optionally substituted C₆-C₁₈ aryl, and more preferably the following optionally substituted aryl group: 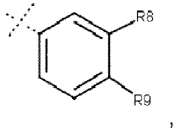 and X is not present;
or A is an optionally substituted five- or six-membered heterocyclic ring, and more preferably wherein A is 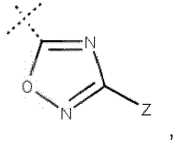
wherein Z is hydrogen, an optionally substituted C₁-C₁₀ alkyl, optionally substituted 2-20 membered heteroalkyl, optionally substituted C₃-C₂₀ cycloalkyl, optionally substituted 3-20 membered cycloheteroalkyl, optionally substituted C₆-C₁₈ aryl or optionally substituted 5-20 membered heteroaryl, preferably an optionally substituted C₆-C₁₄ aryl or 5-20 membered heteroaryl; and
wherein X is a C₁-C₁₀ alkyl group; and
wherein R¹, R², R³, R⁴, R⁵, R⁸ and R⁹ are each independently hydrogen, -F, -CI, -Br, -NO₂,-ON=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R¹ and R², R² and R³, R³ and R⁴, R⁴ and R⁵, or R⁸ and R⁹, when taken together form an optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring.

According to a third aspect, the present invention relates to a compound of Formula (II),

R²¹-B-CO-R²² Formula (II)

or a pharmaceutically acceceptable salt thereof, wherein:
B is an optionanlly substituted heteroaromatic bicycle selected from the group comprising 2-benzofuran, 2-benzothiophene, isoindol, indol, indolizine, and
wherein B is preferably indolizine 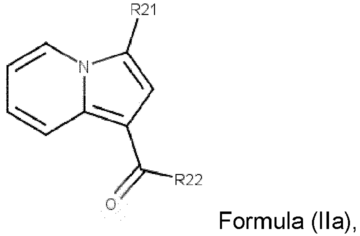
R²¹ is an optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, and
R²² is optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, -OR²³, -NR²³, and wherein
R²³ is hydrogen, optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl.

According to one aspect, the present invention relates to the present compounds for use as a medicament.

According to one aspect, the present invention relates to the present compounds for use in the prevention or treatment of a disease by activating NLRP3 inflammasome, wherein the disease is selected from a group comprising infectious diseases or cancer.

According to one aspect, the present invention relates to a pharmaceutical composition comprising a compound of the present invention and one or more pharmaceutically acceptable excipients.

According to one aspect, the present invention relates to a method for modulating NLRP3 activity, the method comprising engaging NLRP3 with a compound of the present invention.

According to one aspect, the present invention relates to a method of treating cancer, comprising administering to a subject in need of such treatment an effective amount of a compound of the present invention, or a pharmaceutical composition of the present invention According to one aspect, the present invention relates to a method for analyzing the mechanisms of NLRP3 activation.

According to one aspect, the present invention relates to the use of the compounds of the present invention for boosting immune responses or breaking immune evasion in cancer diseases in a subject.

According to one aspect, the present invention relates to the use of the compounds of the present invention for boosting immune responses as an adjuvant in a vaccine in a subject. Preferably, the compounds of the present invention may be used as an adjuvant in the context of vaccination to boost specific immune responses by activating NLRP3 inflammasome.

### Brief Description of the Drawing

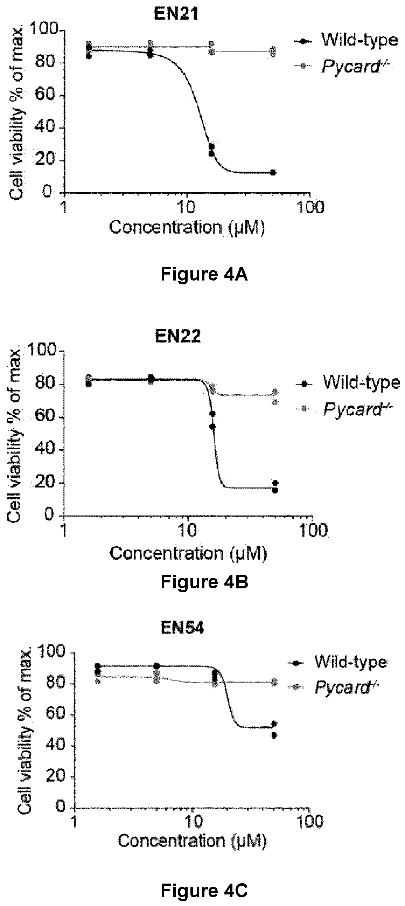

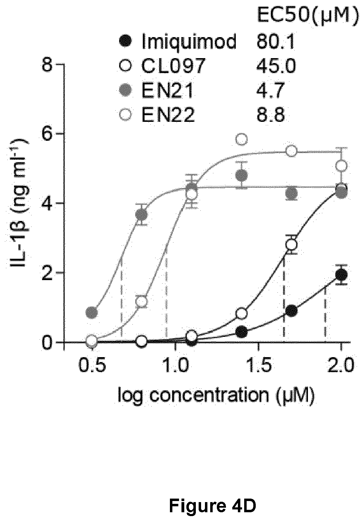

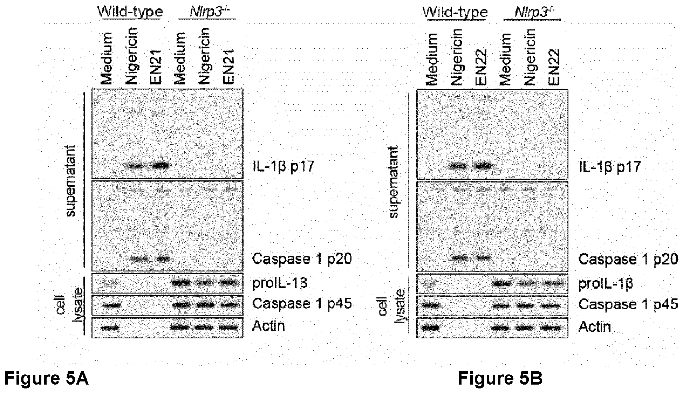

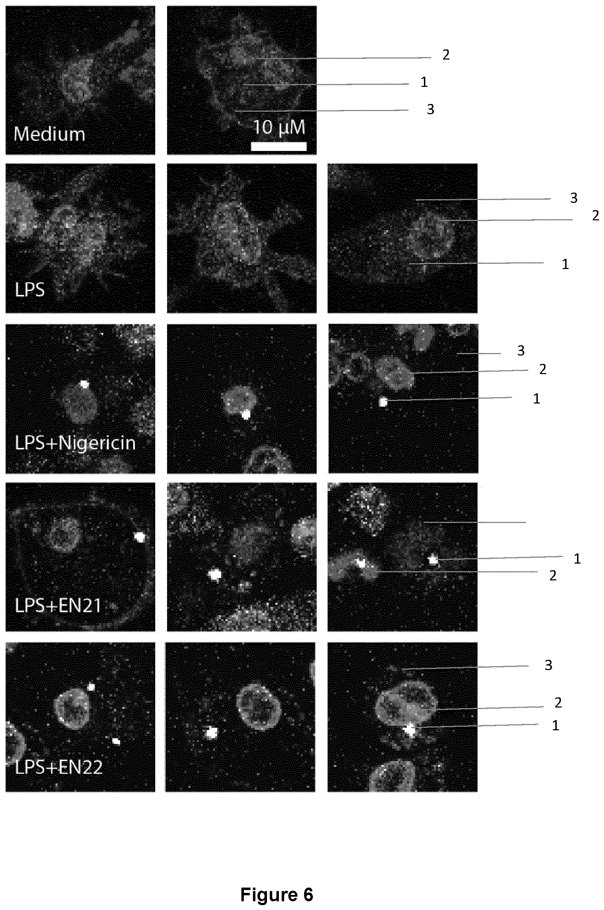

**Figure 1****:** Line diagram of the high-throughput screening of about 50 000 low molecular weight compounds. The diagram shows a partial data set for 39 440 compounds tested at 25 µM of the compounds after about 20 hours of incubation in LPS-primed murine bone marrow-derived dendritic cells (BMDCs). Primary identification of cell death inducing compounds.
**Figure 2****:** Dot diagram of the selection of NLRP3 activators for which cell death is dependent on the inflammasome adapter ASC (*Pycard*^{*-*/*-*}) in LPS-primed BMDCs after 16 hours incubation at 50 µM of the compounds, the framed dots represent the respective replicates of the tested compounds EN21, EN22 and EN54.
**Figure 3****:** Dot diagram of the selection of activators, which lead to the release of IL-1β as an indicator of inflammasome activation (from BMDCs after 20 hours incubation at 50 µM of the compounds). The cells were each pretreated for 3 hours with 50 ng ml⁻¹ LPS to prime for inflammasome activation.
**Figure 4A****:** Line diagram of the dose dependent cytotoxicity of compound EN21 in wild-type and inflammasome knockout (*Pycard*^{*-*/*-*}) BMDCs. Cytotoxicity was quantified by lactate dehydrogenase (LDH) release as determined by an enzymatic assay.
**Figure 4B****:** Line diagram of the dose dependent cytotoxicity of compound EN22 in wild-type and inflammasome knockout (*Pycard*^{*-*/*-*}) BMDCs.
**Figure 4C****:** Line diagram of the dose dependent cytotoxicity of compound EN54 in wild-type and inflammasome knockout (*Pycard*^{*-*/*-*}) BMDCs.
**Figure 4D****:** Line diagram of the EC50 for induction of IL-1β release from BMDCs for compounds EN21 and EN22 in comparison to the imidazoquinoline NLRP3 activators imiquimod and CL097. The presence of IL-1β in the cell culture supernatant was determined by enzyme-linked immunosorbent assay (ELISA) using a kit selective for the mature form of the cytokine.
**Figure 5A****:** Immunoblot analysis of the effects of compound EN21. Caspase-1 activation and release of the biologically active form of IL-1β in BMDCs after treatment with the present compounds at 50 µM for 3 hours. The cells were pretreated with 50 ng ml⁻¹ LPS.
**Figure 5B****:** Immunoblot analysis of the effects of compound EN22. Caspase-1 activation and release of the biologically active form of IL-1β in BMDCs after treatment with the present compounds at 50 µM for 3 hours. The cells were pretreated with 50 ng ml⁻¹ LPS.
**Figure** 6: Images of ASC speck formation in BMDCs of ASC^{citrine} mice expressing a fluorescent fusion protein of the inflammasome adapter ASC. ASC speck formation can be used as a readout for inflammasome activation. ASC specks 1 (green/bright grey, spots), nuclei 2 (blue/dark grey, bigger round structure), CD45 3 (red/dark grey, small spots). The cells were first treated with 50 ng ml⁻¹ LPS for 3 hours, then with 25 µM of the activators for 2 hours.
   The present compounds induce typical characteristics of inflammasome activation, including the activation of caspase-1 and the release of the biologically active forms of leaderless proinflammatory cytokines of the IL-1 family such as IL-1β (Figures 5A and B). Moreover, inflammasome-dependent lytic cell death (pyroptosis, Figure 4) and ASC speck formation are induced by the present compounds (Figure 6). For the induction a concentration dependence can be observed. An advantage of the present compounds are active *in vitro* at approximately 10-fold more lower concentrations than established imidazoquinolines.

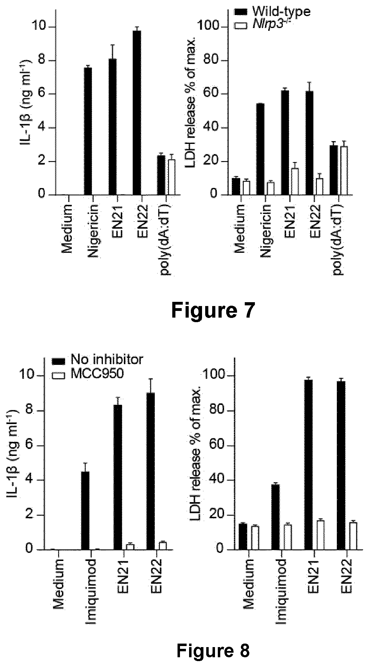

**Figure** 7: Bar diagram of IL-1β and lactate dehydrogenase release (as marker for lytic cell death and pyroptosis) from BMDCs from wild-type or NLRP3 knockout mice. The cells were each pretreated with 50 ng ml⁻¹ LPS.
**Figure** 8: Bar diagram of IL-1β and lactate dehydrogenase release from BMDCs from wild-type mice with or without pre-incubation with the NLRP3-specific inhibitor MCC950. The cells were each pretreated with 50 ng ml⁻¹ LPS.
   Without wishing to be bound to a theory, it is assumed that the compounds EN21 and EN22 activate the NLRP3 inflammasome, since the observed characteristics of inflammasome activation do not occur in cells isolated from NLRP3 knockout mice (Figure 7) and, in addition, the NLRP3-specific inhibitor MCC950 completely suppresses inflammasome activation (Figure 8).
   A further advantage of the present compounds is that the present compounds seem to activate NLRP3 rapidly. Especially with EN22, the inflammasome activation in BMDCs from wild-type mice without inhibitor is almost maximum after only 30 minutes.

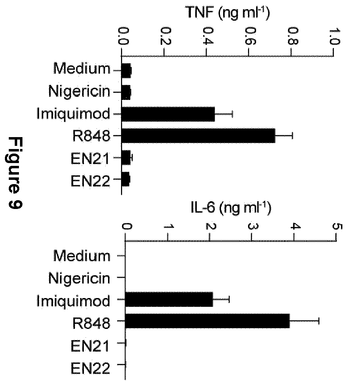

**Figure 9****:** Bar diagram of TNF and IL-6 release of BMDCs as readout for toll-like receptor activation.
   In contrast to the small molecule NLRP3 inflammasome activators imiquimod (patent US 2019/0127368 A1), the present compounds are more specific, since no simultaneous activation of Toll-like receptors (TLR7/ TLR8) occurs and thus the present compounds do not induce the release of further proinflammatory cytokines such as TNF or IL-6.

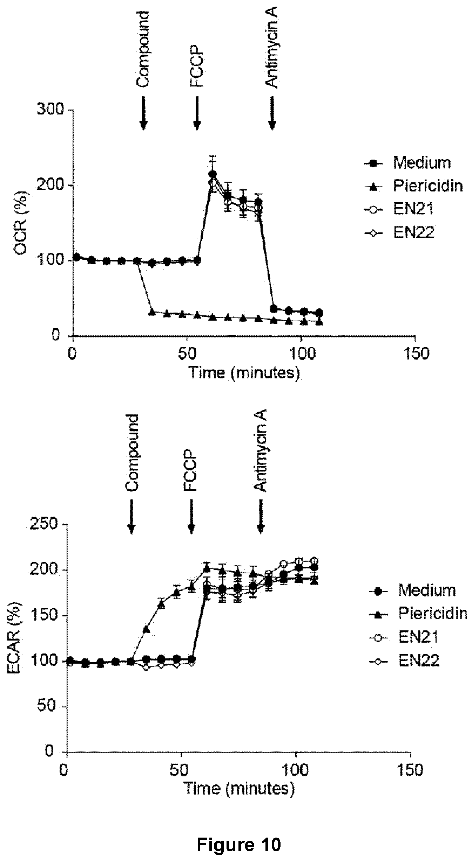

**Figure 10****:** Line diagram of extracellular flux analysis in BMDCs of inflammasome knockout mice. Upper panel: Oxygen consumption rate (OCR) from the medium, which shows the mitochondrial respiration over time. Lower panel: Acidification of the medium, which shows glycolytic activity of the cells over time (extracellular acidification rate, ECAR). The mitochondrial uncoupler FCCP was used to prove responsiveness of the cells and therefore functionality of the assay.
   In contrast to other known small molecule NLRP3 activators, the present compounds do not inhibit mitochondrial respiration and are therefore less susceptible to unspecific undesired effects.

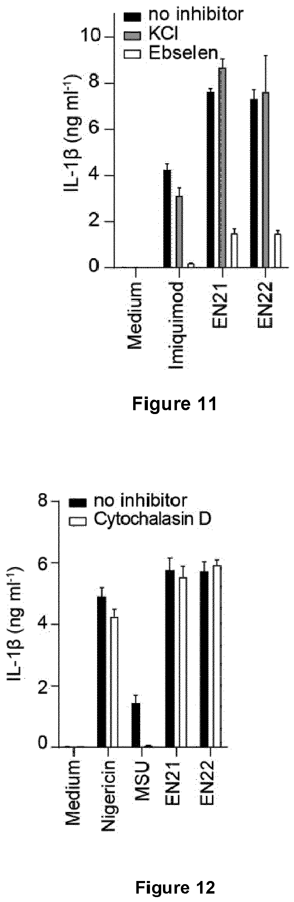

**Figure 11****:** Bar diagram of IL-1β release from BMDCs of wild-type mice either left without inhibitor or pretreated with 50 mM extracellular KCI to inhibit potassium efflux from the cells, or with 30 µM ebselen used as a ROS scavenger. The cells were pretreated with 50 ng ml⁻¹ LPS. Most of the known NLRP3 activators lead to substantial potassium efflux from the cell via different mechanisms, which precedes inflammasome activation. Without wishing to be bound to a theory, it is assumed that the present compounds such as EN21 and EN22 activate the NLRP3 inflammasome independently of potassium efflux from the cell. It is assumed that reactive oxygen species (ROS) are involved in the mechanism of action for NLRP3 activation of the present compounds, since the ROS scavenger ebselen can inhibit inflammasome activation. It might be also possible that ROS influences a further mechanism that is required for NLRP3 activation in parallel to the mechanism activated by EN21 and/or EN22.
**Figure 12****:** Bar diagram of IL-1β release from LPS-primed BMDCs from wild-type mice either left without inhibitor or pretreated with the endocytosis inhibitor cytochalasin D.
   The endocytosis inhibitor cytochalasin D prevents MSU-induced NLRP3 activation, but has no effect on NLRP3 activation by EN21 and EN22. Without wishing to be bound to a theory, it is assumed that contrary to particulate NLRP3 activators such as uric acid crystals (mono sodium urate, MSU) or aluminum hydroxide, the present compounds are not endocyted by immune cells as a prerequisite for NLRP3 inflammasome activation.

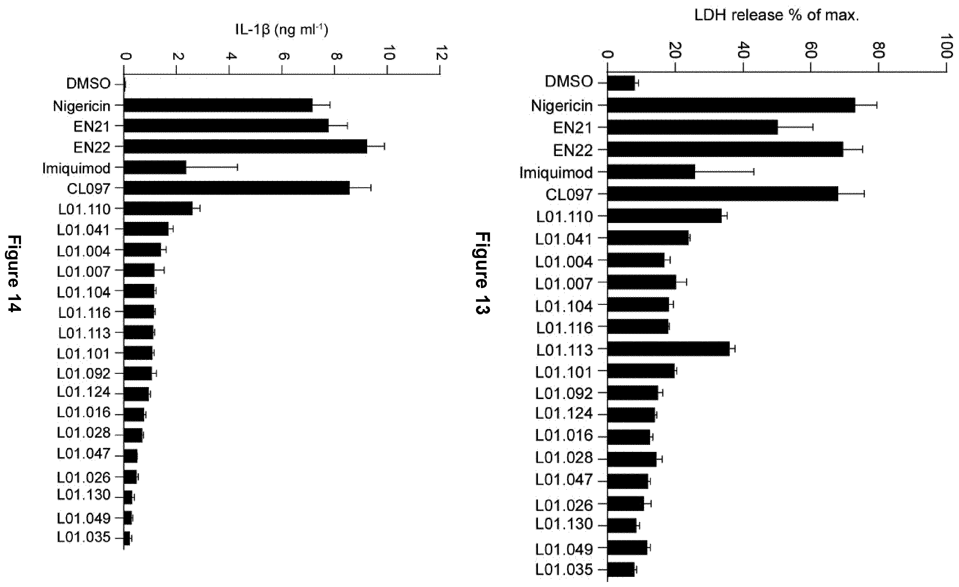

**Figure 13****:** Bar diagram of lactate dehydrogenase release (as marker for lytic cell death and pyroptosis) from BMDCs of wild-type mice treated with 50 µM of the present compounds for 22 hours in comparison to the solvent only (DMSO), and the known activators nigericin (5 µM), imiquimod (R837) and CL097 (each 100 µM). The cells were each pretreated for 3 hours with 50 ng ml⁻¹ LPS.
**Figure 14****:** Bar diagram of IL-1β release from BMDCs from wild-type mice treated with 50 µM of the present compounds for 22 hours in comparison to the solvent only (DMSO) and the known activators nigericin (5 µM), imiquimod (R837) and CL097 (each 100 µM). The cells were each pretreated for 3 hours with 50 ng ml⁻¹ LPS.

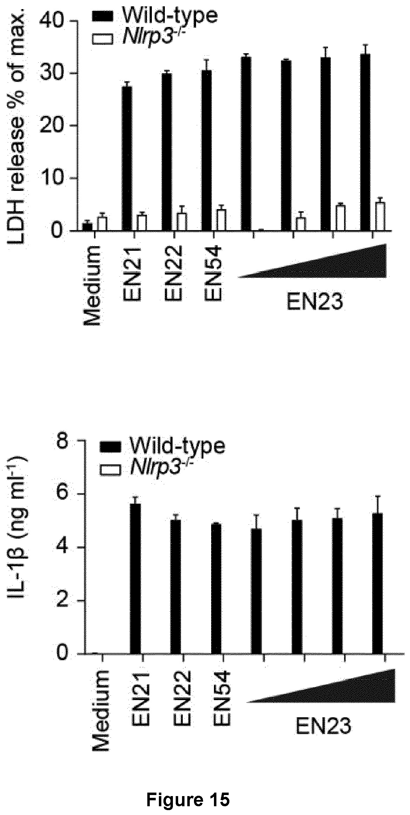

**Figure 15****:** Bar diagram of IL-1β (lower figure) and lactate dehydrogenase (upper figure) release of BMDCs from wild-type and NLRP3 knockout mice. The cells were primed with 50 ng ml⁻¹ LPS for 3 hours and then stimulated with the indicated compounds for 3 hours as follows: EN21 12.5 µM, EN22 25 µM, EN54 50 µM, EN23 25 µM, 50 µM, 100 µM, 200 µM (concentrations increasing from left to right, indicated by the wedge below).
   Without wishing to be bound to a theory, it is assumed that the compound EN23 acts similarly as EN21 and EN22 activates the NLRP3 inflammasome, since the observed characteristics of inflammasome activation do not occur in cells isolated from NLRP3 knockout mice (Figures 7 and 15).

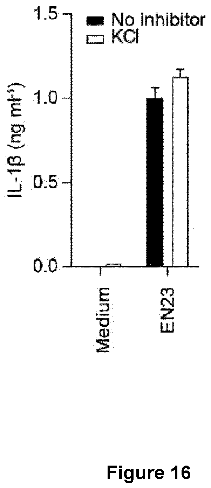

**Figure 16****:** Bar diagram of IL-1β release of wild-type BMDCs. The cells were primed with 50 ng ml⁻¹ LPS for 3 hours, incubated with 50 mM extracellular KCI to prevent potassium efflux from the cells for 30 minutes and then stimulated with 12.5 µM EN23 for 3 hours.
   Without wishing to be bound to a theory, it is assumed that the present compounds such as EN23 activate the NLRP3 inflammasome independently of potassium efflux from the cell.

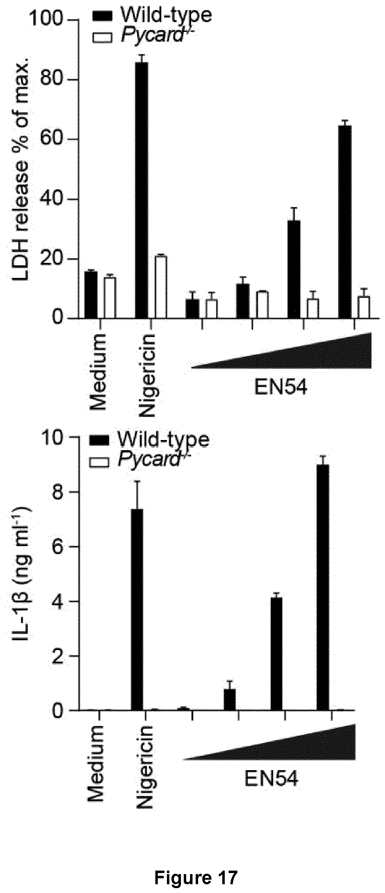

**Figure 17****:** Bar diagram of IL-1β and lactate dehydrogenase release of BMDCs from wild-type and ASC knockout mice. The cells were primed with 50 ng ml⁻¹ LPS for 3 hours and then stimulated with the indicated compounds for 3 hours as follows: nigericin 5 µM, EN54 10 µM, 20 µM, 40 µM, 80 µM (concentrations increasing from left to right, indicated by the wedge below).

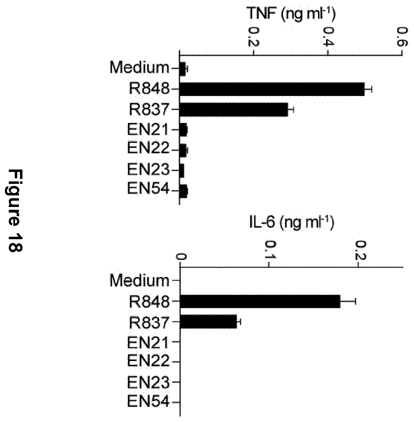

**Figure 18****:** Bar diagram of TNF and IL-6 release of BMDCs as readout for toll-like receptor activation.

### Detailed description of the invention

The terms "a" and "an" do not denote a limitation of quantity, but rather denote the presence of at least one of the referenced item. The term "or" or "and/or" is used as a function word to indicate that two words or expressions are to be taken together or individually. The terms "comprising", "having", "including", and "containing" are to be construed as open-ended terms (i.e., meaning "including, but not limited to"). The endpoints of all ranges directed to the same component or property are inclusive and independently combinable.

The term "present compound(s)" or "compound(s) of the present invention" refers to compounds encompassed by a structural formula disclosed herein, such as Formula (I), and Formula (II), Formula (la), Formual (lb), Formula (IIa), Formual (IIb) and includes any subgenus and specific compounds within the formula whose structure is disclosed herein. Compounds may be identified either by their chemical structure and/or chemical name. When the chemical structure and chemical name conflict, the chemical structure is determinative of the identity of the compound. The compounds described herein may contain one or more chiral centers and/or double bonds and therefore, may exist as stereoisomers, such as double-bond isomers (*i.e*., geometric isomers), enantiomers or diastereomers. Accordingly, the chemical structures depicted herein encompass all possible enantiomers and stereoisomers of the illustrated compounds including the stereoisomerically pure form (*e.g*., geometrically pure, enantiomerically pure or diastereomerically pure) and enantiomeric and stereoisomeric mixtures. Enantiomeric and stereoisomeric mixtures can be resolved into their component enantiomers or stereoisomers using separation techniques or chiral synthesis techniques well known to the skilled artisan. The compounds may also exist in several tautomeric forms including the enol form, the keto form and mixtures thereof. Accordingly, the chemical structures depicted herein encompass all possible tautomeric forms of the illustrated compounds. The compounds described also include isotopically labeled compounds where one or more atoms have an atomic mass different from the atomic mass conventionally found in nature. Examples of isotopes that may be incorporated into the compounds of the invention include, but are not limited to, ²H, ³H, ¹³C, ¹⁴C, ¹⁵N, ¹⁸O, ¹⁷O, *etc.* Compounds may exist in unsolvated forms as well as solvated forms, including hydrated forms and as N-oxides. In general, compounds may be hydrated, solvated or N-oxides. Certain compounds may exist in multiple crystalline or amorphous forms. In general, all physical forms are equivalent for the uses contemplated herein and are intended to be within the scope of the present invention. Further, it should be understood, when partial structures of the compounds are illustrated, that brackets indicate the point of attachment of the partial structure to the rest of the molecule. The term "tautomer" as used herein refers to isomers that change into one another with great ease so that they can exist together in equilibrium.

"Alkyl," by itself or as part of another substituent, refers to a saturated branched, or straight-chain hydrocarbon radical derived by the removal of one hydrogen atom from a single carbon atom of a parent alkane. Typical alkyl groups include, but are not limited to, methyl; ethyl; propyls such as propan-1-yl, propan-2-yl (isopropyl), *etc.;* butanyls such as butan-1-yl, butan-2-yl (sec-butyl), 2-methyl-propan-1-yl (isobutyl), 2-methyl-propan-2-yl (*t*-butyl); and the like. In some embodiments, an alkyl group comprises from 1 to 20 carbon atoms (C₁-C₂₀ alkyl). In other embodiments, an alkyl group comprises from 1 to 10 carbon atoms (C₁-C₁₀ alkyl). In still other embodiments, an alkyl group comprises from 1 to 6 carbon atoms (C₁-C₆ alkyl) or 1 to 4 carbon atoms (C₁-C₄ alkyl). In still other embodiments, an alkyl group comprises from 1 to 3 carbon atoms (C₁-C₃ alkyl). C₁-C₆ alkyl is also known as "lower alkyl".

"Cycloalkyl," by itself or as part of another substituent, refers to a saturated cycloalkyl hydrocarbon radical derived by the removal of one hydrogen atom from a single carbon atom of a parent cycloalkane. Cycloalkyl further includes cycloalkenyls as defined herein. Typical cycloalkyl groups include, but are not limited to, cyclopropyl, cyclobutyl, cyclopentyl, cyclohexyl; cyclopropenyl, cyclobutenyl, cyclopentenyl, cyclohexenyl; cyclobutadienyl, cyclohexadienyl; and the like. In some embodiments, a cycloalkyl group comprises from 3 to 20 carbon atoms (C₃-C₂₀ cycloalkyl). In other embodiments, a cycloalkyl group comprises from 3 to 10 carbon atoms (C₃-C₁₀ cycloalkyl). In a preferred embodiment, a cycloalkyl group comprises from 3 to 6 carbon atoms (C₃-C₆ cycloalkyl). In a more preferred embodiment, a cycloalkyl group comprises from 3 to 5 carbon atoms (C₃-C₅ cycloalkyl).

"Cycloalkyl alkyl," by itself or as part of another substituent, refers to an acyclic alkyl group in which one of the hydrogen atoms bonded to a carbon atom, typically a terminal or *sp*³ carbon atom, is replaced with a cycloalkyl group as defined herein. That is, cycloalkyl alkyl can also be considered as an alkyl substituted by cycloalkyl. Typical cycloalkyl alkyl groups include, but are not limited to, cyclopropylmethyl, 2-cyclopropylethan-1-yl, 2-cyclopropylethen-1-yl, pentylmethyl, 2-pentylethan-1-yl, 2-pentylethen-1-yl, and the like. Where specific alkyl moieties are intended, the nomenclature cycloalkyl alkanyl, cycloalkyl alkenyl and/or cycloalkyl alkynyl is used. In some embodiments, a cycloalkyl alkyl group is (C₃-C₃₀) cycloalkyl alkyl, e.g., the alkanyl, alkenyl or alkynyl moiety of the cycloalkyl alkyl group is (C₁-C₁₀) alkyl and the cycloalkyl moiety is (C₃-C₂₀) cycloalkyl. In other embodiments, a cycloalkyl alkyl group is (C₄-C₂₀) cycloalkyl alkyl, e.g., the alkanyl, alkenyl or alkynyl moiety of the cycloalkyl alkyl group is (C₁-C₈) alkyl and the cycloalkyl moiety is (C₃-C₁₂) cycloalkyl. In still other embodiments, a cycloalkyl alkyl group is (C₄-C₁₀) cycloalkyl alkyl, e.g., the alkanyl, alkenyl or alkynyl moiety of the cycloalkyl alkyl group is (C₁-C₄) alkyl and the cycloalkyl moiety is (C₃-C₆) cycloalkyl.

It is noted that when an alkyl group is further connected to another atom, it becomes an "alkylene" group. In other words, the term "alkylene" refers to a divalent alkyl. For example, - CH₂CH₃ is an ethyl, while -CH₂CH₂- is an ethylene. That is, "Alkylene," by itself or as part of another substituent, refers to a saturated or unsaturated, branched, straight-chain or cyclic divalent hydrocarbon radical derived by the removal of two hydrogen atoms from a single carbon atom or two different carbon atoms of a parent alkane, alkene or alkyne. The term "alkylene" is specifically intended to include groups having any degree or level of saturation, *i.e.,* groups having exclusively single carbon-carbon bonds, groups having one or more double carbon-carbon bonds, groups having one or more triple carbon-carbon bonds and groups having mixtures of single, double and triple carbon-carbon bonds. In some embodiments, an alkylene group comprises from 1 to 20 carbon atoms (C₁-C₂₀ alkylene). In other embodiments, an alkylene group comprises from 1 to 10 carbon atoms (C₁-C₁₀ alkylene). In still other embodiments, an alkylene group comprises from 1 to 6 carbon atoms (C₁-C₆ alkylene).

"Alkenyl," by itself or as part of another substituent, refers to an unsaturated branched or straight-chain monovalent hydrocarbon radical having at least one carbon-carbon double bond derived by the removal of one hydrogen atom from a single carbon atom of a parent alkene. The group may be in either the *cis* or *trans* conformation about the double bond(s). Typical alkenyl groups include, but are not limited to, ethenyl; propenyls such as prop-1-en-1-yl, prop-1-en-2-yl, prop-2-en-1-yl (allyl), prop-2-en-2-yl, butenyls such as but-1-en-1-yl, but-1-en-2-yl, 2-methyl-prop-1-en-1-yl, but-2-en-1-yl, but-2-en-1-yl, but-2-en-2-yl, buta-1,3-dien-1-yl, buta-1,3-dien-2-yl, *etc.;* and the like. In some embodiments, an alkenyl group comprises from 2 to 20 carbon atoms (C₂-C₂₀ alkenyl). In other embodiments, an alkenyl group comprises from 2 to 10 carbon atoms (C₂-C₁₀ alkenyl). In still other embodiments, an alkenyl group comprises from 2 to 6 carbon atoms (C₂-C₆ alkenyl) or 1 to 4 carbon atoms (C₂-C₄ alkenyl). C₂-C₆ alkenyl is also known as "lower alkenyl".

"Cycloalkenyl," by itself or as part of another substituent, refers to an unsaturated cycloalkenyl hydrocarbon radical derived by the removal of one hydrogen atom from a single carbon atom of a parent cycloalkene. Typical cycloalkenyl groups include, but are not limited to, cyclopropenyls such as clycloprop-1-en-1-yl, cycloprop-2-en-1-yl, cyclobutenyls such as cyclobut-1-en-1-yl, cyclobut-1-en-3-yl, cyclobuta-1,3-dien-1-yl, *etc.;* and the like. In some embodiments, a cycloalkenyl group comprises from 3 to 20 carbon atoms (C₃-C₂₀ cycloalkenyl). In other embodiments, a cycloalkenyl group comprises from 3 to 10 carbon atoms (C₃-C₁₀ cycloalkenyl). In a preferred embodiment, a cycloalkenyl group comprises from 3 to 6 carbon atoms (C₃-C₆ cycloalkenyl). In a more preferred embodiment, a cycloalkenyl group comprises from 3 to 5 carbon atoms (C₃-C₅ cycloalkenyl).

"Cycloalkenyl alkyl," by itself or as part of another substituent, refers to an acyclic alkyl group in which one of the hydrogen atoms bonded to a carbon atom, typically a terminal or *sp*³ carbon atom, is replaced with an cycloalkenyl group as defined herein. That is, cycloalkenyl alkyl can also be considered as an alkyl substituted by cycloalkenyl. Typical cycloalkenyl alkyl groups include, but are not limited to, cyclopropenylmethyl, 2-cyclopropenylethan-1-yl, 2-cyclopropenylethen-1-yl, pentylmethyl, 2-pentenylethan-1-yl, 2-pentenylethen-1-yl, and the like. Where specific alkyl moieties are intended, the nomenclature cycloalkenyl alkanyl, cycloalkenyl alkenyl and/or cycloalkenyl alkynyl is used. In some embodiments, a cycloalkenyl alkyl group is (C₃-C₃₀) cycloalkenyl alkyl, e.g., the alkanyl, alkenyl or alkynyl moiety of the cycloalkenyl alkyl group is (C₁-C₁₀) alkyl and the cycloalkenyl moiety is (C₃-C₂₀) cycloalkenyl. In other embodiments, a cycloalkenyl alkyl group is (C₄-C₂₀) cycloalkenyl alkyl, e.g., the alkanyl, alkenyl or alkynyl moiety of the cycloalkenyl alkyl group is (C₁-C₈) alkyl and the cycloalkenyl moiety is (C₃-C₁₂) cycloalkenyl. In still other embodiments, a cycloalkenyl alkyl group is (C₄-C₁₀) cycloalkenyl alkyl, e.g., the alkanyl, alkenyl or alkynyl moiety of the cycloalkenyl alkyl group is (C₁-C₄) alkyl and the cycloalkenyl moiety is (C₃-C₆) cycloalkenyl.

"Alkynyl," by itself or as part of another substituent refers to an unsaturated branched, or straight-chain monovalent hydrocarbon radical having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl, prop-2-yn-1-yl, *etc.;* butynyls such as but-1-yn-1-yl, but-1-yn-3-yl, but-3-yn-1-yl, *etc.;* and the like. In some embodiments, an alkynyl group comprises from 1 to 20 carbon atoms (C₂-C₂₀ alkynyl). In other embodiments, an alkynyl group comprises from 1 to 10 carbon atoms (C₂-C₁₀ alkynyl). In still other embodiments, an alkynyl group comprises from 1 to 6 carbon atoms (C₂-C₆ alkynyl) or 1 to 4 carbon atoms (C₂-C₄ alkynyl). C₂-C₆ alkynyl is also known as "lower alkynyl".

"Alkoxy," by itself or as part of another substituent, refers to a radical of the formula -O-R¹⁹⁹, where R¹⁹⁹ is alkyl or substituted alkyl as defined herein. In some embodiments, R¹⁹⁹ is a C₁-C₃ alkyl substituent. In some embodiments, R¹⁹⁹ is a C₁-C₂ alkyl substituent. In some embodiments, R¹⁹⁹ is a C₁ alkyl substituent.

"Acyl" by itself or as part of another substituent refers to a radical -C(O)R²⁰⁰, where R²⁰⁰ is hydrogen, alkyl, substituted alkyl, aryl, substituted aryl, arylalkyl, substituted arylalkyl, heteroalkyl, substituted heteroalkyl, heteroarylalkyl or substituted heteroarylalkyl as defined herein. Representative examples include, but are not limited to formyl, acetyl, cyclohexylcarbonyl, cyclohexylmethylcarbonyl, benzoyl, benzylcarbonyl and the like.

"Aryl," by itself or as part of another substituent, refers to a monovalent aromatic hydrocarbon group derived by the removal of one hydrogen atom from a single carbon atom of a parent aromatic ring system, as defined herein. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene and the like. In some embodiments, an aryl group comprises from 6 to 18 carbon atoms (C₆-C₁₈ aryl). In other embodiments, an aryl group comprises from 6 to 15 carbon atoms (C₆-C₁₅ aryl). In still other embodiments, an aryl group comprises from 6 to 10 carbon atoms (C₆-C₁₀ aryl).

"Arylalkyl," by itself or as part of another substituent, refers to an acyclic alkyl group in which one of the hydrogen atoms bonded to a carbon atom, typically a terminal or *sp*³ carbon atom, is replaced with an aryl group, as defined herein. That is, arylalkyl can also be considered as an alkyl substituted by aryl. Typical arylalkyl groups include, but are not limited to, benzyl, 2-phenylethan-1-yl, 2-phenylethen-1-yl, naphthylmethyl, 2-naphthylethan-1-yl, 2-naphthylethen-1-yl, naphthobenzyl, 2-naphthophenylethan-1-yl and the like. Where specific alkyl moieties are intended, the nomenclature arylalkanyl, arylalkenyl and/or arylalkynyl is used. In some embodiments, an arylalkyl group is (C₆-C₃₀) arylalkyl, e.g., the alkanyl, alkenyl or alkynyl moiety of the arylalkyl group is (C₁-C₁₀) alkyl and the aryl moiety is (C₆-C₁₈) aryl. In other embodiments, an arylalkyl group is (C₆-C₁₈) arylalkyl, e.g., the alkanyl, alkenyl or alkynyl moiety of the arylalkyl group is (C₁-C₈) alkyl and the aryl moiety is (C₆-C₁₂) aryl. In still other embodiments, an arylalkyl group is (C₆-C₁₄) arylalkyl, e.g., the alkanyl, alkenyl or alkynyl moiety of the arylalkyl group is (C₁-C₅) alkyl and the aryl moiety is (C₆-C₁₀) aryl.

"Carbocyclic," or "Carbocyclyl," by itself or as part of another substituent, refers to a saturated or partially saturated, aromatic, cyclic monovalent hydrocarbon radical, including cycloalkyl, cycloalkenyl, and cycloalkynyl as defined herein. Typical carbocyclyl groups include, but are not limited to, groups derived from cyclopropane, cyclobutane, cyclopentane, cyclohexane, and the like. In some embodiments, the cycloalkyl group comprises from 3 to 10 ring atoms (C₃-C₁₀ cycloalkyl). In other embodiments, the cycloalkyl group comprises from 3 to 7 ring atoms (C₃-C₇ cycloalkyl). The carbocyclyl may be further substituted by one or more heteroatoms including, but not limited to, N, P, O, S, and Si, which attach to the carbon atoms of the cycloalkyl via monovalent or multivalent bond.

"Heteroalkyl," by themselves or as part of other substituents, refer to alkyl groups, in which one or more of the carbon atoms, are each, independently of one another, replaced with the same or different heteroatoms or heteroatomic groups. Each atom in the linear heteroalkyl forms a member of the heteroalkyl. For example -CH₂-O-CH₂-CH₂-O-CH₃ is a six-membered heteroalkyl. In some embodiments, the heteroalkyl group comprises from 2 to 20 members. In other embodiments, the heteroalkyl group comprises from 2 to 15 members. In some embodiments, the heteroalkyl group comprises from 2 to 10 members. In other embodiments, the heteroalkyl group comprises from 2 to 5 members. Typical heteroatoms or heteroatomic groups, which can replace the carbon atoms include, but are not limited to, -O-, -S-, -N-, -Si-, -NH-, -S(O)-, -S(O)₂-, -S(O)NH-, -S(O)₂NH- and the like and combinations thereof. The heteroatoms or heteroatomic groups may be placed at any interior position of the alkyl group. Typical heteroatomic groups which can be included in these groups include, but are not limited to, -O-, -S-, -O-O-, -S-S-, -O-S-, -NR²⁰¹R²⁰²-, =N-N=, -N=N-, -N=N-NR²⁰³R²⁰⁴, -PR²⁰⁵-, -P(O)₂-, -POR²⁰⁶-, -O-P(O)₂-, -SO-, -SO₂-, -SnR²⁰⁷R^{2 08}- and the like, where R²⁰¹, R²⁰², R²⁰³, R²⁰⁴, R²⁰⁵, R²⁰⁶, R²⁰⁷ and R²⁰⁸ are independently hydrogen, alkyl, substituted alkyl, aryl, substituted aryl, arylalkyl, substituted arylalkyl, cycloalkyl, substituted cycloalkyl, cycloheteroalkyl, substituted cycloheteroalkyl, heteroalkyl, substituted heteroalkyl, heteroaryl, substituted heteroaryl, heteroarylalkyl or substituted heteroarylalkyl. Preferably the heteroatoms, which can replace the carbon atoms include -O-, -S-, -N-, or -NH-.

"Heterocyclic ring" or "Heterocyclyl" by itself or as part of another substituent, refers to a carbocyclic radical in which one or more carbon atoms are independently replaced with the same or different heteroatom. The heterocyclyl may be a cycloheteroaryl or a cycloheteroalkyl. The heterocyclyl may be further substituted by one or more heteroatoms including, but not limited to, N, P, O, S, and Si, which attach to the carbon atoms of the heterocyclyl via monovalent or multivalent bond. Typical heteroatoms to replace the carbon atom(s) include, but are not limited to, N, P, O, S, Si, *etc.* Typical heterocyclyl groups include, but are not limited to, groups derived from epoxides, azirines, thiiranes, imidazolidine, morpholine, piperazine, piperidine, pyrazolidine, pyrrolidone, quinuclidine, and the like. In preferrred embodiments, heterocyclyl groups include, but are not limited to tetrahydrofuran, furan, pyrrolidine, pyridine, pyrazol, indolizine, quinoline, thiadiazol, oxadiazol and 1,2,4-oxadiazol. In some embodiments, the heterocyclyl group comprises from 3 to 20 ring atoms (3-20 membered heterocyclyl). In preferred embodiments, the heterocyclyl group comprises from 3 to 14 ring atoms (3-14 membered heterocyclyl). In more preferred embodiments, the heterocyclyl group comprises from 3 to 10 ring atoms (3-10 membered heterocyclyl). In still more preferred embodiments, the heterocyclyl group comprise from 5 to 7 ring atoms (5-7 membered heterocyclyl). A cycloheteroalkyl group may be substituted at a heteroatom, for example, a nitrogen atom, with a (C₁-C₆) alkyl group. As specific examples, N-methyl-imidazolidinyl, N-methyl-morpholinyl, N-methyl-piperazinyl, N-methyl-piperidinyl, N-methyl-pyrazolidinyl and N-methyl-pyrrolidinyl are included within the definition of "heterocyclyl." A heterocyclyl group may be attached to the remainder of the molecule *via* a ring carbon atom or a ring heteroatom.

"Halo," by itself or as part of another substituent refers to a radical -F, -CI, -Br or -I.

"Heteroaryl," by itself or as part of another substituent, refers to a monovalent heteroaromatic radical derived by the removal of one hydrogen atom from a single atom of a parent heteroaromatic ring systems, as defined herein. Typical heteroaryl groups include, but are not limited to, groups derived from acridine, β-carbohne, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, and the like. In some embodiments, the heteroaryl group comprises from 5 to 20 ring atoms (5-20 membered heteroaryl). In other embodiments, the heteroaryl group comprises from 5 to 10 ring atoms (5-10 membered heteroaryl). Exemplary heteroaryl groups include those derived from furan, thiophene, pyrrole, benzothiophene, benzofuran, benzimidazole, indole, pyridine, pyrazole, quinoline, imidazole, oxazole, isoxazole and pyrazine.

"Heteroarylalkyl" by itself or as part of another substituent refers to an acyclic alkyl group in which one of the hydrogen atoms bonded to a carbon atom, typically a terminal or *sp*³ carbon atom, is replaced with a heteroaryl group. Where specific alkyl moieties are intended, the nomenclature heteroarylalkanyl, heteroarylakenyl and/or heteroarylalkynyl is used. In some embodiments, the heteroarylalkyl group is a 4-30 membered heteroarylalkyl, e.g., the alkanyl, alkenyl or alkynyl moiety of the heteroarylalkyl is (C₁-C₁₀) alkyl and the heteroaryl moiety is a 5-20-membered heteroaryl. In other embodiments, the heteroarylalkyl is a 5-15 membered heteroarylalkyl, e.g., the alkanyl, alkenyl or alkynyl moiety is (C₁-C₅) alkyl and the heteroaryl moiety is a 5-10 membered heteroaryl.

"Salt" refers to a salt of a compound, which possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound is replaced by a metal ion, e.g., an alkali metal ion, an alkaline earth ion, or an aluminum ion; or coordinates with an organic base such as ethanolamine, diethanolamine, triethanolamine, N-methylglucamine and the like.

"Solvate" means a compound formed by solvation (the combination of solvent molecules with molecules or ions of the solute), or an aggregate that consists of a solute ion or molecule, i.e., a compound of the present invention, with one or more solvent molecules. When water is the solvent, the corresponding solvate is "hydrate". In one embodiment, each compound of the present invention is a solvate.

"Substituted," when used to modify a specified group or radical, means that one or more hydrogen atoms of the specified group or radical are each, independently of one another, replaced with the same or different substituent(s). Substituent groups useful for substituting saturated carbon atoms in the specified group or radical include, but are not limited to -R^{a}, halo, -O⁻, =O, -OR^{b}, -SR^{b}, -S⁻, =S, -NR^{c}R^{c}, =NR^{b}, =N-OR^{b}, trihalomethyl, -CF₃, -CN, -OCN, -SCN, -NO, -NO₂, =N₂, -N₃, -S(O)₂R^{b}, -S(O)₂NR^{b}, -S(O)₂O-, -S(O)₂OR^{b}, -OS(O)₂R^{b}, -OS(O)₂O⁻, -OS(O)₂OR^{b}, -P( O)(O⁻)₂, -P(O)(OR^{b})(O⁻), -P(O)(OR^{b})(OR^{b}), -C(O)R^{b}, -C(S)R^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S)R^{b}, -OC(O)O⁻, -OC(O)OR^{b}, -OC(S) OR^{b}, -NR^{b}C(O)R^{b}, -NR^{b}C(S)R^{b}, -NR^{b}C(O)O⁻, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a} is selected from the group consisting of alkyl, cycloalkyl, heteroalkyl, cycloheteroalkyl, aryl, arylalkyl, heteroaryl and heteroarylalkyl; each R^{b} is independently hydrogen or R^{a}; and each R^{c} is independently R^{b} or alternatively, the two R^{c}s may be taken together with the nitrogen atom to which they are bonded form a 4-, 5-, 6- or 7-membered cycloheteroalkyl which may optionally include from 1 to 4 of the same or different additional heteroatoms selected from the group consisting of O, N and S. As specific examples, -NR^{c}R^{c} is meant to include -NH₂, -NH-alkyl, N-pyrrolidinyl and N-morpholinyl. As another specific example, a substituted alkyl is meant to include -alkylene-O-alkyl, -alkylene-heteroaryl, -alkylene-cycloheteroalkyl, -alkylene-C(O)OR^{b}, -alkylene-C(O)NR^{b}R^{b}, and -CH₂-CH₂-C(O)-CH₃. The one or more substituent groups, taken together with the atoms to which they are bonded, may form a cyclic ring including cycloalkyl and cycloheteroalkyl.

Similarly, substituent groups useful for substituting unsaturated carbon atoms in the specified group or radical include, but are not limited to, -R^{a}, halo, -O⁻, -OR^{b}, -SR^{b}, -S-, -NR^{c}R^{c}, trihalomethyl, -CF₃, -CN, -OCN, -SCN, -NO, -NO₂, -N₃, -S(O)₂R^{b}, -S(O)₂O-, -S(O)₂OR^{b}, -OS(O )₂R^{b}, -OS(O)₂O⁻, -OS(O)₂OR^{b}, -P(O)(O⁻)₂, -P(O)(OR^{b})(O⁻), -P(O)(OR^{b})(OR^{b}), -C(O)R^{b}, -C(S)R ^{b}, -C(NR^{b})R^{b}, -C(O)O⁻, -C(O)OR^{b}, -C(S)OR^{b}, -C(O)NR^{c}R^{c}, -C(NR^{b})NR^{c}R^{c}, -OC(O)R^{b}, -OC(S) R^{b}, -OC(O)O⁻, -OC(O)OR^{b}, -OC(S)OR^{b}, -NR^{b}C(O)R^{b}, -NR^{b}C(S)R^{b}, -NR^{b}C(O)O- -NR^{b}C(O)OR ^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups useful for substituting nitrogen atoms in heteroalkyl and cycloheteroalkyl groups include, but are not limited to, -R^{a}, -O⁻, -OR^{b}, -SR^{b}, -S-, -_{NR}^{e}_{R}^{e}, trihalomethyl, -CF₃, -CN, -NO, -NO₂, -S(O)₂R^{b}, -S(O)₂O-, -S(O)₂OR^{b}, -OS(O)₂R ^{b}, -OS(O)₂O⁻, - OS(0)₂0R ^{b}, -P(O)(O⁻)₂, -P(O)(OR^{b})(O⁻), -P(O)(OR^{b})(OR^{b}), -C(O)R^{b}, -C(S)R^{b}, -C(NR^{b})R^{b}, -C( O)OR^{b}, -C(S)OR^{b}, -C(O)NR^{c}R^{c}, -C(NR^{b})NR^{c}R^{c}, -OC(O)R^{b}, -OC(S)R^{b}, -OC(O)OR^{b}, -OC(S)OR ^{b}, -NR^{b}C(O)R^{b}, -NR^{b}C(S)R^{b}, -NR^{b}C(O)OR^{b}, -NR^{b}C(S)OR^{b}, -NR^{b}C(O)NR^{c}R^{c}, -NR^{b}C(NR^{b})R^{b} and -NR^{b}C(NR^{b})NR^{c}R^{c}, where R^{a}, R^{b} and R^{c} are as previously defined.

Substituent groups from the above lists useful for substituting other specified groups or atoms will be apparent to those of skill in the art.

The term "substituted" specifically envisions and allows for one or more substitutions that are common in the art. However, it is generally understood by those skilled in the art that the substituents should be selected so as to not adversely affect the useful characteristics of the compound or adversely interfere with its function. Suitable substituents may include, for example, halogen groups, perfluoroalkyl groups, perfluoroalkoxy groups, alkyl groups, alkenyl groups, alkynyl groups, hydroxy groups, oxo groups, mercapto groups, alkylthio groups, alkoxy groups, aryl or heteroaryl groups, aryloxy or heteroaryloxy groups, arylalkyl or heteroarylalkyl groups, arylalkoxy or heteroarylalkoxy groups, amino groups, alkyl- and dialkylamino groups, carbamoyl groups, alkylcarbonyl groups, carboxyl groups, alkoxycarbonyl groups, alkylaminocarbonyl groups, dialkylamino carbonyl groups, arylcarbonyl groups, aryloxycarbonyl groups, alkylsulfonyl groups, arylsulfonyl groups, cycloalkyl groups, cyano groups, C₁-C₆ alkylthio groups, arylthio groups, nitro groups, keto groups, acyl groups, boronate or boronyl groups, phosphate or phosphonyl groups, sulfamyl groups, sulfonyl groups, sulfinyl groups, and combinations thereof. In the case of substituted combinations, such as "substituted arylalkyl," either the aryl or the alkyl group may be substituted, or both the aryl and the alkyl groups may be substituted with one or more substituents. Additionally, in some cases, suitable substituents may combine to form one or more rings as known to those of skill in the art.

In a preferred embodiment, a "substituted" cycloalkyl, heterocyclic ring or aryl group includes the substitution with one or more substituent each substituent independently selected from the group comprising -F, -CI, -Br, -NO₂,-O-N=O, -N=O, -OH, -NH₂, -CH=NH, -N₃, -SH, -SO₃H, - SO₂H, -S(O)₂CH₃ -CN, -NC,-OCN, -NCO, -SCN, -NCS, -(P=O)(OH)₂, -O(P=O)(OH)₂, - (C=O)OH, -(C=O)NHOH, -NH(C=O)NH₂, -NH(C=S)NH₂, -(C=O)NH₂,-CHO,-CHS, -OCHO, - NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)1R¹¹, (C-O)OR¹¹ (C=-O=SR¹¹ (C=O)NR¹¹R¹², (SO₂)R¹¹, (SNO)R¹¹, (P=O)R¹¹R¹² (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃.

More preferably, a "substituted" alkyl, heteroalkyl, cycloalkyl, heterocyclic ring or aryl group includes the substitution with one or more substituent each substituent independently selected from the group comprising -F, -CI, -Br, -NO₂,-O-N=O, -N=O, -OH, -NH₂, -CH=NH, -N₃, -SH, - SO₃H, -SO₂H, -S(O)₂CH₃ -CN, -NC,-OCN, -NCO, -SCN, -NCS, -(P=O)(OH)₂, -O(P=O)(OH)₂, - (C=O)OH, -(C=O)NHOH, -NH(C=O)NH₂, -NH(C=S)NH₂, -(C=O)NH₂,-CHO,-CHS, -OCHO, - NCHO, -B(OH)₂, C₁-C₄ alkyl, C₁-C₄ alkoxy, C₂-C₄ alkenyl, C₃-C_{1O} cycloalkyl, C₃-C₁₀ cycloalkyl C₁-C₂ alkyl, 3-10 membered cycloheteroalkyl or 3-10 membered cycloheteroalkyl C₁-C₂ alkyl, C₆-C₁₀ aryl, C₆-C₁₀ aryl C₁-C₂ alkyl, 5-10 membered heteroaryl, 5-10 membered heteroaryl C₁-C₂ alkyl, (C=O)1R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of C₁-C₄ alkyl, C₂-C4 alkenyl, C₃-C₁₀ cycloalkyl, C₆-C₁₀ aryl, 5-10 membered heteroaryl, 2-10 membered heteroalkyl, -CHF₂, -CF₃, -CHCl₂, and -CCl₃.

Preferably, a "substituted" cycloalkyl, heterocyclic ring or aryl group includes the substitution with one, two or three substituents, still more preferably with one or two substituents.

The term "optionally substituted" denotes the presence or absence of the substituent group(s). That is, it means "substituted or unsubstituted". For example, optionally substituted alkyl includes both unsubstituted alkyl and substituted alkyl. The substituents used to substitute a specified group can be further substituted, typically with one or more of the same or different groups selected from the various groups specified above.

"Carrier" refers to a diluent, excipient or vehicle with which a compound is administered.

"NLRP3" is meant to include, without limitation, nucleic acids, polynucleotides, oligonucleotides, sense and antisense polynucleotide strands, complementary sequences, peptides, polypeptides, proteins, homologous and/or orthologous NLRP3 molecules, isoforms, precursors, mutants, variants, derivatives, splice variants, alleles, different species, and active fragments thereof.

A "modulator" herein refers to a compound that can regulate the activity of NLRP3. Such regulation includes activating NLRP3, blocking NLRP3, or potentiating/reducing the activation of NLRP3. That is, the modulators include agonists, antagonists, enhancers, etc.

An "activator" or an "agonist" of NLRP3 herein refers to a compound that can modulate the activity of NLRP3. Such modulation includes activating NLRP3, or increasing the activation of NLRP3. An "activator" or an "agonist" includes compounds that, at the protein level, directly bind or modify NLRP3 such that an activity of NLRP3 is increased, e.g., by activation, stabilization, altered distribution, or otherwise.

"Treating" or "treatment" of any condition, disease or disorder refers to ameliorating the condition, disease or disorder (*i.e*., arresting or reducing the development of the condition, disease or disorder or at least one of the clinical symptoms thereof). In other embodiments "treating" or "treatment" refers to ameliorating at least one physical parameter, which may not be discernible by the patient. In yet other embodiments, "treating" or "treatment" refers to inhibiting the condition, disease or disorder, either physically, (*e.g*., stabilization of a discernible symptom), physiologically, (*e.g*., stabilization of a physical parameter) or both. In yet other embodiments, "preventing," "prevention," "treating" or "treatment" refers to delaying the onset of the condition, disease or disorder.

"Therapeutically effective amount" means the amount of the present compound, a pharmaceutically acceptable salt and/or hydrate and/or cocrystal thereof; e.g., a compound, such as niclosamide or a pharmaceutically acceptable salt and/or hydrate and/or cocrystal thereof that, when administered to a subject for treating a condition, disease or disorder, is sufficient to effect such treatment for the condition, disease or disorder. The "therapeutically effective amount" will vary depending on the compound, the condition, disease or disorder and its severity and the age, weight, *etc*., of the subject to be treated. The result includes reduction and/or alleviation of the signs, symptoms, or causes of a disease, or any other desired alteration of a biological system.

"Subject" refers to an animal, including, but not limited to, a primate (e.g., human), monkey, cow, pig, sheep, goat, horse, dog, cat, rabbit, rat, or mouse. The terms "subject" and "patient" are used interchangeably herein in reference, for example, to a mammalian subject, such as a human.

"Pharmaceutical composition" refers to a mixture of a compound described herein with other chemical components (referred to collectively herein as "excipients"), such as carriers, stabilizers, diluents, dispersing agents, suspending agents, and/or thickening agents. The pharmaceutical composition facilitates administration of the compound to an organism. Multiple techniques of administering a compound exist in the art including, but not limited to: rectal, oral, intravenous, aerosol, parenteral, ophthalmic, pulmonary, and topical administration. A further technique of administering is the intratumoral injection.

"Excipient" or "pharmaceutically acceptable excipient" means a pharmaceutically-acceptable material, composition, or vehicle, such as a liquid or solid filler, diluent, carrier, solvent, or encapsulating material. In one embodiment, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. See, e.g., Remington: The Science and Practice of Pharmacy, 21st ed.; Lippincott Williams & Wilkins: Philadelphia, PA, 2005; Handbook of Pharmaceutical Excipients, 6th ed. ; Rowe et al, Eds.; The Pharmaceutical Press and the American Pharmaceutical Association: 2009; Handbook of Pharmaceutical Additives, 3rd ed. ; Ash and Ash Eds.; Gower Publishing Company: 2007; Pharmaceutical Preformulation and Formulation, 2nd ed. ; Gibson Ed.; CRC Press LLC: Boca Raton, FL, 2009.

### Embodiments of the Compounds

According to the first aspect, the present invention relates to a compound for use in the prevention or treatment of a disease by activating NLRP3 inflammasome, wherein the disease is selected from a group comprising infectious diseases or cancer, and
wherein the compound is a compound of Formula (I) 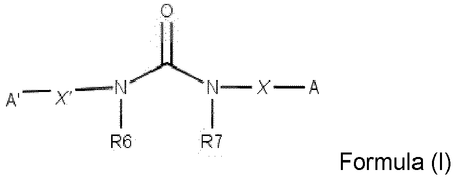 or a pharmaceutically acceceptable salt thereof, wherein:
A is an optionally substituted C₆-C₁₈ aryl; optionally substituted 5-20 membered heteroaryl; optionally substituted C₃-C₂₀ cycloalkyl; optionally substituted 3-20 membered cycloheteroalkyl,
and wherein X and X' are each independently present or not, wherein X and X' are each independently an optionally substituted C₁-C₁₀ alkyl group;
R⁶ and R⁷ are each independently hydrogen, an optionally substituted C₁-C₁₀ alkyl or R⁶ and R⁷ when taken together form an optionally substituted five- or six-membered heterocyclic ring.

X and X' are each independently a linker group which bonds the nitrogen of the urea to the A or A' respectively. If X or X' is not present, the nitrogen of the urea is directly bound to the A or A', respectively.

In one embodiment, the compound of Formula (I) of the invention comprises X and X', wherein X and X' are each independently -(CH₂)ₘ-, wherein each m is independently an integer from 0 to 4; and wherein
R⁶ and R⁷ are each independently hydrogen, a C₁-C₁₀ alkyl, preferably wherein R⁶ and R⁷ are each hydrogen.

In one embodiment, the compound of Formula (I) of the invention comprises A and A', wherein A and A' are each independently optionally substituted benzene, imidazolidine, morpholine, piperazine, piperidine, pyrazolidine, pyrrolidone, quinuclidine, tetrahydrofuran, furan, pyrrolidine, pyridine, pyrazol, indolizine, quinoline, thiadiazol, oxadiazol, 1,2,4-oxadiazol, acridine, β-carbohne, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, cyclopropyl, cyclobutyl, cyclopentyl, cyclohexyl; cyclopropenyl, cyclobutenyl, cyclopentenyl, cyclohexenyl; cyclobutadienyl, cyclohexadienyl, carbazole, benzimidazole, imidazopyridine, benzoisoxazole, tetrahydrothiophene, benzofuran, benzoxazole, or benzthiozole,
preferably wherein A and A' are each independently optionally substituted quinoline, isoquinoline, indole, benzene, pyridine, pyrazole, pyrrol, furan or thiophene;
more preferably wherein A and A' are each independently optionally substituted quinoline or benzene.

In one embodiment, the compound of Formula (I) of the invention comprises X and X', wherein X and X' are each independently -(CH₂)ₘ-, wherein each m is independently an integer from 0 to 2, preferably 0 or 1, more preferably 0.

In one embodiment, the compound of Formula (I) of the invention is a compound of Formula (Ic): 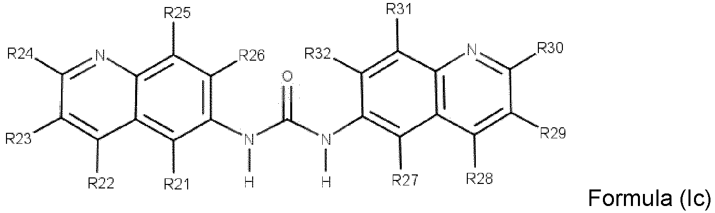 or 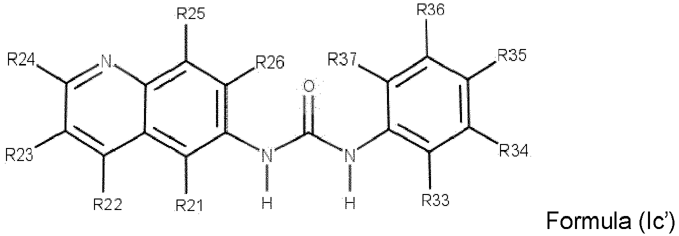
wherein R²¹ to R³⁷ are each independently selected from the group consisting of hydrogen, -F, -CI, -Br, -NO₂,-O-N=O, -N=O, -OH, -NH₂, -CH=NH, -N₃, -SH, -SO₃H, -SO₂H, -S(O)₂CH₃ -CN, - NC,-OCN, -NCO, -SCN, -NCS, -(P=O)(OH)₂, -O(P=O)(OH)₂, -(C=O)OH, -(C=O)NHOH, - NH(C=O)NH₂, -NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=0)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or - NHC(O)NHR",
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃.

In a preferred embodiment, the compound of Formula (Ic) or (Ic') is as defined above, wherein R²¹ to R³⁷ are each independently selected from the group consisting of hydrogen, C₁-C₆ alkoxy, C₁-C₅ alkyl, -(C=O)CH₃, -CN, C₁-C₅ alkyl(SO₂)C₁-C₅ alkyl, preferably -CH₂(SO₂)CH₃, - CH₂(SO2)CH₂CH₃, -CH₂CH₂(SO₂)CH₂CH₃; -(SO₂)C₁-C₅ alkyl, preferably -(SO₂)CH₃, - (SO₂)CH₂CH₃, -CF₃, -F, -CI, or-Br.

In a preferred embodiment, the compound of Formula (Ic) is as defined above, wherein R²¹, R²⁶, R²⁷ and R³² is preferably hydrogen.

In a preferred embodiment, the compound of Formula (Ic') is as defined above, wherein R²¹, R²⁶, R³³ and R³⁷ is preferably hydrogen.

In a preferred embodiment, the compound of Formula (Ic) or (Ic') is as defined above, wherein R²⁴ is C₁-C₆ alkoxy, C₁-C₅ alkyl, -(C=O)CH₃, -CN, C₁-C₅ alkyl(SO₂)C₁-C₅ alkyl, preferably - CH₂(SO2)CH₃, -CH₂(SO₂)CH₂CH₃, -CH₂CH₂(SO₂)CH₂CH₃; -(SO₂)C₁-C₅ alkyl, preferably - (SO₂)CH₃, -(SO₂)CH₂CH₃, -CF₃, -F, -CI, or-Br. and wherein R²¹ to R²³ and R²⁵ to R³⁷ are each hydrogen.

In a preferred embodiment, the compound of Formula (Ic) or (Ic') is as defined above, wherein R²³ is C₁-C₆ alkoxy, C₁-C₅ alkyl, -(C=O)CH₃, -CN, C₁-C₅ alkyl(SO₂)C₁-C₅ alkyl, preferably - CH₂(SO2)CH₃, -CH₂(SO₂)CH₂CH₃, -CH₂CH₂(SO₂)CH₂CH₃; -(SO₂)C₁-C₅ alkyl, preferably - (SO₂)CH₃, -(SO₂)CH₂CH₃, -CF₃, -F, -CI, or-Br. and wherein R²¹, R²² and R²⁴ to R³⁷ are each hydrogen.

In a preferred embodiment, the compound of Formula (Ic') is as defined above, wherein R³⁵ is C₁-C₆ alkoxy, C₁-C₅ alkyl, -(C=O)CH₃, -CN, C₁-C₅ alkyl(SO₂)C₁-C₅ alkyl, preferably - CH₂(SO2)CH₃, -CH₂(SO₂)CH₂CH₃, -CH₂CH₂(SO₂)CH₂CH₃; -(SO₂)C₁-C₅ alkyl, preferably - (SO₂)CH₃, -(SO₂)CH₂CH₃, -CF₃, -F, -CI, or -Br. and wherein R²¹ to R²⁶, R³³, R³⁴ and R³⁶, R³⁷ are each hydrogen.

In a preferred embodiment, the compound of Formula (Ic') is as defined above, wherein R³⁶ is C₁-C₆ alkoxy, C₁-C₅ alkyl, -(C=O)CH₃, -CN, C₁-C₅ alkyl(SO₂)C₁-C₅ alkyl, preferably - CH₂(SO2)CH₃, -CH₂(SO₂)CH₂CH₃, -CH₂CH₂(SO₂)CH₂CH₃; -(SO₂)C₁-C₅ alkyl, preferably - (SO₂)CH₃, -(SO₂)CH₂CH₃, -CF₃, -F, -CI, or -Br. and wherein R²¹ to R²⁶, R³³, R³⁴ and R³⁵, R³⁷ are each hydrogen.

In a preferred embodiment, the compound of Formula (Ic) or (Ic') is as defined above, wherein R²² is C₁-C₆ alkoxy, C₁-C₅ alkyl, -(C=O)CH₃, -CN, C₁-C₅ alkyl(SO₂)C₁-C₅ alkyl, preferably - CH₂(SO2)CH₃, -CH₂(SO₂)CH₂CH₃, -CH₂CH₂(SO₂)CH₂CH₃; -(SO₂)C₁-C₅ alkyl, preferably - (SO₂)CH₃, -(SO₂)CH₂CH₃, -CF₃, -F, -CI, or -Br. and wherein R²¹ and R²³ to R³⁷ are each hydrogen.

In a preferred embodiment, the compound of Formula (Ic) or (Ic') is as defined above, wherein R²¹ to R³⁷ are each hydrogen.

In a preferred embodiment, the compound of Formula (Ic) is the following structure:

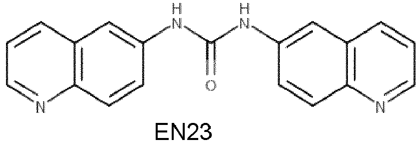

In a second aspect, the compound of Formula (I) of the invention is a compound of Formula (la), 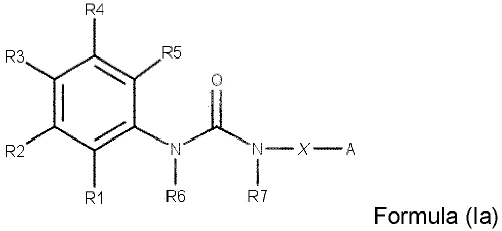 or a pharmaceutically acceceptable salt thereof, wherein:

A is preferably an optionally substituted C₆-C₁₈ aryl, and more preferably the following optionally substituted aryl group: 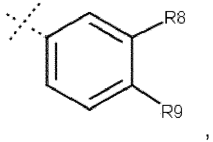
wherein X is a C₁-C₁₀ alkyl group or preferably X is not present;
or A is an optionally substituted five- or six-membered heterocyclic ring, and more preferably wherein A is

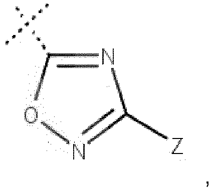

wherein Z is hydrogen, an optionally substituted C₁-C₁₀ alkyl, optionally substituted 2-20 membered heteroalkyl, optionally substituted C₃-C₂₀ cycloalkyl, optionally substituted 3-20 membered cycloheteroalkyl, optionally substituted C₆-C₁₈ aryl or optionally substituted 5-20 membered heteroaryl, preferably an optionally substituted C₆-C₁₄ aryl or 5-20 membered heteroaryl; and
wherein X is a C₁-C₁₀ alkyl group; and
wherein R¹, R², R³, R⁴, R⁵, R⁸ and R⁹are each independently hydrogen, -F, -CI, -Br, -NO₂,-ON=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R¹ and R², R² and R³, R³ and R⁴, R⁴ and R⁵, or R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring. In one embodiment of the invention, in Formula (la), X is a C₁-C₅ alkyl group and A is an optionally substituted five- or six-membered heterocyclic ring, wherein the heterocyclic ring is selected from the group comprising pyridine, pyrimidine, oxazole, thiazole, pyrazole, imidazole, triazole, furan, thiophene, pyrrole, tetrazole or oxadiazole. In one embodiment of the invention, in Formula (la), A is oxadiazole.

In one embodiment, the compound of Formula (Ia) of the invention comprises X, wherein X is -(CH₂)ₘ-, wherein each m is independently an integer from 0 to 2, preferably 0 or 1, more preferably 0

In one embodiment of the invention, in Formula (la), A is an optionally substituted C₆ aryl and X is not present or a C₁-C₂ alky, preferably X is not present.

In one embodiment of the invention, in Formula (la), A is an optionally substituted C₆ aryl.

In one embodiment of the invention, in Formula (la), A is an optionally substituted 6 to 10 membered heteroaryl, wherein the 6 to 10 membered heteroaryl is pyridine, pyrimidine, pyrazine, quinazoline, benzoxazine, isoquinoline, or quinoline.

In one embodiment of the invention, in Formula (la), A is quinoline.

According to one aspect, the present invention relates to a compound of Formula (la), or a pharmaceutically acceceptable salt thereof, wherein:
A is the following optionally substituted aryl group: 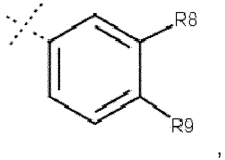
and X is not present;
or wherein A is 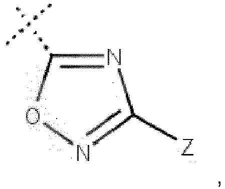
wherein Z is hydrogen, an optionally substituted C₁-C₁₀ alkyl, optionally substituted 2-20 membered heteroalkyl, optionally substituted C₃-C₂₀ cycloalkyl, optionally substituted 3-20 membered cycloheteroalkyl, optionally substituted C₆-C₁₈ aryl or optionally substituted 5-20 membered heteroaryl, preferably an optionally substituted C₆-C₁₄ aryl or 5-20 membered heteroaryl; and X is a C₁-C₁₀ alkyl group
   R¹, R², R³, R⁴, R⁵, R⁸ and R⁹ are each independently hydrogen, -F, -CI, -Br, -NO₂,-O-N=O, - N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, -NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R¹ and R², R² and R³, R³ and R⁴, R⁴ and R⁵, or R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring.

In one embodiment of Formula (la), R⁸ and R⁹ are each independently hydrogen, -F, -CI, -Br, - NO₂,-O-N=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R³ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring and
wherein R¹, R², R³, R⁴ and R⁵ are each independently hydrogen, C₁-C₆ alkoxy, C₁-C₅ alkyl, -F, -CI, or-Br.

In one embodiment of Formula (la), R⁸ and R⁹ are each independently hydrogen, -F, -CI, -Br, - NO₂,-O-N=O, -N=O, -OH, -NH₂, -CH=NH, -N₃, -SH, -SO₃H, -SO₂H, -S(O)₂CH₃-CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring and
wherein R¹, R⁴ and R⁵ are each independently hydrogen, C₁-C₆ alkoxy, C₁-C₅ alkyl, -F, -Cl, or -Br and wherein R² and R³ are each hydrogen.

In one embodiment of Formula (Ia), R¹, R², R³, R⁴, R⁵, R⁸ and R⁹ are each independently hydrogen, -F, -CI, -Br, -NO₂,-O-N=O, -N=O, -OH, -NH₂, -CH=NH, -N₃, -SH, -SO₃H, -SO₂H, - S(O)₂CH₃ -CN, -NC,-OCN, -NCO, -SCN, -NCS, -(P=O)(OH)₂, -O(P=O)(OH)₂, -(C=O)OH, - (C=O)NHOH, -NH(C=O)NH₂, -NH(C=S)NH₂, -(C=O)NH₂,-CHO,-CHS, -OCHO, -NCHO, - B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹² (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or

R¹ and R², R² and R³, R³ and R⁴, R⁴ and R⁵, or R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
wherein R⁶ and R⁷ are each independently hydrogen, or R⁶ and R⁷ when taken together form a heterocyclic ring selected from:

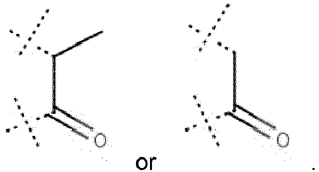

In one embodiment of Formula (Ia), R⁸ and R⁹ are each independently hydrogen, -F, -CI, -Br, - NO₂,-O-N=O, -N=O, -OH, -NH₂, -CH=NH, -N₃, -SH, -SO₃H, -SO₂H, -S(O)₂CH₃-CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring and
wherein
- R¹: is hydrogen, C₁-C₂ alkoxy, C₁-C₃ alkyl,
- R²: is hydrogen;
- R³: is hydrogen or methyl;
- R⁴: is hydrogen or -F, -Cl, or -Br; and
- R⁵: is hydrogen, methyl or ethyl.

In one embodiment of Formula (la), R¹ to R⁵ and R⁶ and R⁹ are as previously defined wherein, R² and R³; or R², R³ and R⁵; or R², R³, R⁴ and R⁵ are each hydrogen.

According to one embodiment, the present invention relates to a compound of Formula (Ia), 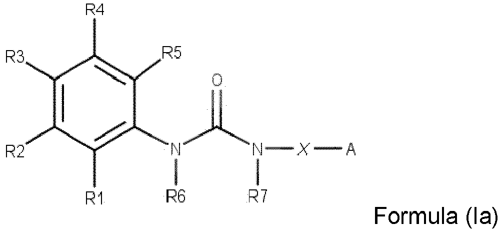 or a pharmaceutically acceceptable salt thereof, wherein:
or A is the following optionally substituted aryl group: 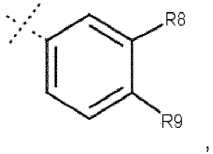 and X is not present; and wherein
R¹, R², R³, R⁴, R⁵, R⁸ and R⁹ are each independently hydrogen, -F, -CI, -OH, -NH₂, -S(O)₂CH₃, -(C=O)OH, -NH(C=O)NH₂, -(C=O)NH₂,-CHO,- -OCHO, -NCHO optionally substituted C₁-C₅ alkyl, optionally substituted C₁-C₅ alkoxy, optionally substituted C₂-C₆ alkenyl, optionally substituted C₃-C₇ cycloalkyl, optionally substituted C₃-C₇ cycloalkyl C₁-C₅ alkyl, optionally substituted C₂-C₁₀ cycloheteroalkyl or C₂-C₁₄ cycloheteroalkyl C₁-C₅ alkyl, optionally substituted C₆-C₁₄ aryl, optionally substituted C₆-C₁₄ aryl C₁-C₅ alkyl, optionally substituted C₂-C₁₄ heteroaryl, optionally substituted C₂-C₁₄ heteroaryl C₁-C₅ alkyl, (C=O)R¹¹, (C=O)OR¹¹, (C=O)NR¹¹R¹², (SO₂)R¹¹, C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring.

In one embodiment, the present invention relates to a compound of Formula (la), wherein wherein X is a C₁-C₁₀ alkyl group; and wherein A is 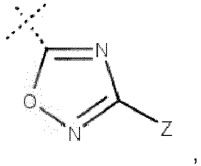 wherein Z is an optionally substituted C₁-C₅ alkyl, optionally substituted C₁-C₅ heteroalkyl, optionally substituted C₃-C₁₀ cycloalkyl, optionally substituted 3-10 membered cycloheteroalkyl, optionally substituted C₆-C₁₀ aryl or optionally substituted 5-10 membered heteroaryl. In one embodiment, Z is an optionally substituted C₆-C₁₀ aryl, preferably an optionally substituted C₆ aryl. In one embodiment Z is optionally substituted 5-10 membered heteroaryl, preferably 5-6 membered heteroaryl, and more preferably pyridine.

R¹, R², R³, R⁴, are R⁵, are each independently hydrogen, -F, -CI, -Br, -NO₂,-O-N=O, -N=O, - OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, -NH(C=S)NH₂, - (C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R¹ and R², R² and R³, R³ and R⁴, R⁴ and R⁵, or R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring.

In one embodiment, the present invention relates to a compound of Formula (la), wherein the compound is a compound of Formula (Ia') 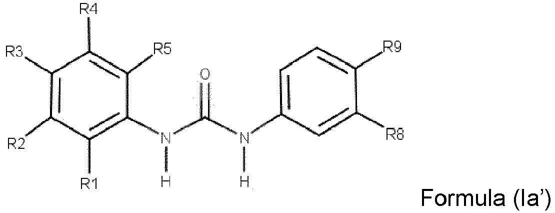 wherein R¹, R², R³, R⁴, R⁵, R⁸ and R⁹ are each independently hydrogen, -F, -Cl, -Br, -NO₂,-ON=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R¹ and R², R² and R³, R³ and R⁴, R⁴ and R⁵, or R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl;
or wherein R¹, R², R³, R⁴ and R⁵ are each hydrogen;
or wherein R⁸ and R⁹ are each independently hydrogen, substituted C₁-C₁₀ alkyl, substituted 2-10 membered heteroalkyl, substituted C₂-C₁₄ aryl, substituted 5-10 membered heteroaryl, substituted five- or six-membered cycloheteroalkyl, -S(O)₂CH₃, -F, -Cl, -Br, -NHC(O)R¹¹, or - NHC(O)NHR¹¹,
wherein R¹¹ is selected from C₁-C₅ alkyl, C₃-C₆ cycloalkyl, -CHF₂, -CF₃, -CHCl₂, -CCl₃; or five-membered heterocyclic ring;
with the proviso that R⁸ is not identical to R⁹, or
wherein R³ and R⁹, when taken together form an optionally substituted five- or six-membered cycloalkyl, optionally substituted five- or six-membered cycloheteroalkyl or optionally substituted five or six-membered aryl, optionally substituted five- or six-membered heteroaryl, preferably the heterocyclic ring is selected from: 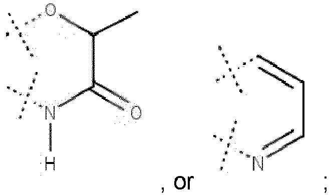 or or wherein R⁸ and R⁹ are each independently defined as above, with the provisio that if one of R⁸ or R⁹ is -F, -Cl, or -Br, the other R⁸ or R⁹ is neither -F, -Cl, -Br nor hydrogen.

In one embodiment, the present invention relates to a compound of Formula (la), wherein the compound is a compound of Formula (Ia')
wherein R¹, R², R³, R⁴, R⁵, and R⁸ are each independently hydrogen, -F, -Cl, -Br, -NO₂,-ON=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; and
wherein R⁹ is selected from C₁-C₃ alkyl, five-membered heterocyclic ring, -OCH₃, -S(O)₂CH₃, -NHC(O)R¹¹, or -NHC(O)NHR¹¹,
wherein R¹¹ is selected from C₂-C₅ alkyl, C₃-C₆ cycloalkyl, -CHF₂, -CF₃, -CHCl₂, -CCl₃; or five-membered heterocyclic ring.

In preferred embodiment, R⁹ is selected from C₁-C₃ alkyl, pyrrolidine, -OCH₃, -S(O)₂CH₃, - NHC(O)R¹¹, or -NHC(O)NHR¹¹,
wherein R¹¹ is selected from C₂-C₅ alkyl, C₃-C₆ cycloalkyl, -CHF₂, -CF₃, -CHCl₂, -CCl₃; or tetrahydrofuran or furan.

In more preferred embodiment, R⁹ is selected from C₁ alkyl, C₃ alkyl, pyrrolidine, -OCH₃, - S(O)₂CH₃, -NHC(O)R¹¹, or -NHC(O)NHR¹¹,
wherein R¹¹ is selected from C₅ cycloalkyl, -CHF₂, or tetrahydrofuran or furan.

In one embodiment, the present invention relates to a compound of Formula (la), wherein the compound is a compound of Formula (Ia')
wherein R¹, R², R³, R⁴, R⁵, and R⁹ are each independently hydrogen, -F, -CI, -Br, -NO₂,-ON=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; and
R⁸ is -S(O)₂CH₃, -NHC(O)R¹¹, or -NHC(O)NHR¹¹,
wherein R¹¹ is selected from C₂-C₅ alkyl, C₃-C₅ cycloalkyl, -CHF₂; or five-membered heterocyclic ring.

In a preferred embodiment R⁸ is -S(O)₂CH₃, -NHC(O)R¹¹, or -NHC(O)NHR¹¹,
wherein R¹¹ is selected from C₃ alkyl, C₃ cycloalkyl, -CHF₂.

In one embodiment, the present invention relates to a compound of Formula (Ia), wherein the compound is a compound of Formula (Ia')
wherein R¹, R², R³, R⁴, and R⁵, are each independently hydrogen, -F, -Cl, -Br, -NO₂,-O-N=O, - N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, -NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; and,
wherein R⁸ and R⁹, when taken together form a heterocyclic ring is selected from:

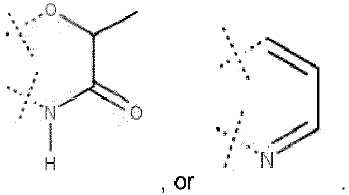

In one embodiment, the present invention relates to a compound of Formula (la), wherein the -X-A group is 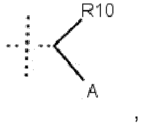 wherein R¹⁰ is C₁-C₃ alkyl, preferably -CH(CH₃)₂ or -(CH₂)ₙ-A, wherein n is an integer from 1-3 or in one embodiment, n is 2; in another embodiment, n is 1; and wherein A is 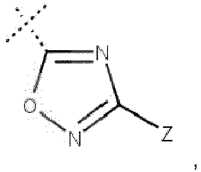 forming Formula (Ib) 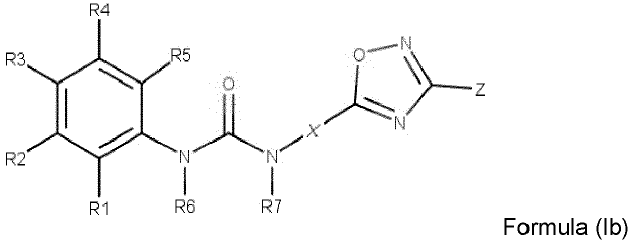 wherein Z is hydrogen, an optionally substituted C₁-C₁₀ alkyl, optionally substituted 2-20 membered heteroalkyl, optionally substituted C₆-C₁₈ aryl or optionally substituted 5-20 membered heteroaryl, preferably an optionally substituted C₆-C₁₄ aryl or 5-20 membered heteroaryl.

R¹, R², R³, R⁴, and R⁵, are each independently hydrogen, -F, -Cl, -Br, -NO₂,-O-N=O, -N=O, - OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, -NH(C=S)NH₂, - (C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R⁶ and R⁷ are each independently hydrogen, an optionally substituted C₁-C₁₀ alkyl or R⁶ and R⁷ when taken together form an optionally substituted five- or six-membered heterocyclic ring.

In one embodiment of the invention, in Formula (Ia) or (Ib), A is an optionally substituted oxadiazole. In one embodiment, A is 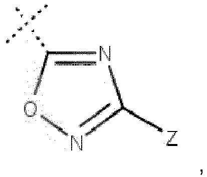 wherein Z is an optionally substituted C₆-C₁₄ aryl or 5-10 membered heteroaryl, preferably Z is pyridyl or substituted C₆ aryl, more preferably 3-pyridyl or para substituted phenyl, wherein the substituent is selected from the group consisting of -F, -Cl, and -CF₃.

In one embodiment, the present invention relates to a compound of Formula (lb), wherein R² and R⁵ are each hydrogen,
R¹ is hydrogen or methoxy,
R³, is hydrogen or methyl and
R⁴ is hydrogen or -F, wherein at least one of R¹ to R⁵ is not hydrogen.

In one embodiment, the present invention relates to a compound wherein the compound is selected from the compounds delineated in Table 1.

**Table 1:**

| No. | Structure Formula (Ia') |
|---|---|
| L01.110 | 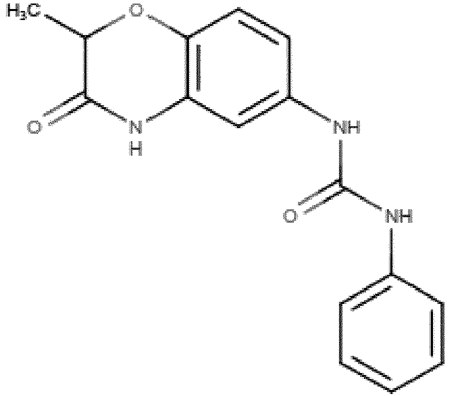 |
| L01.041 | 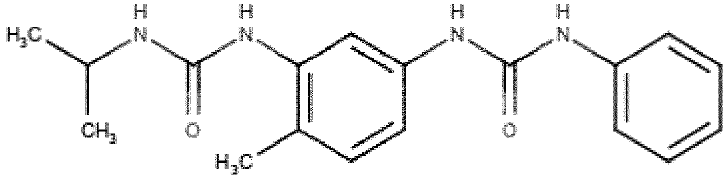 |
| L01.004 | 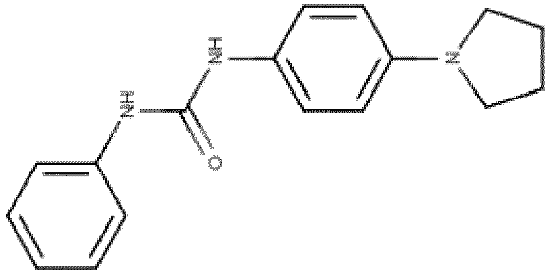 |
| L01.104 | 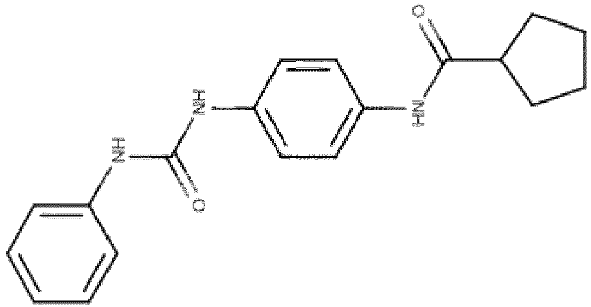 |
| L01.116 | 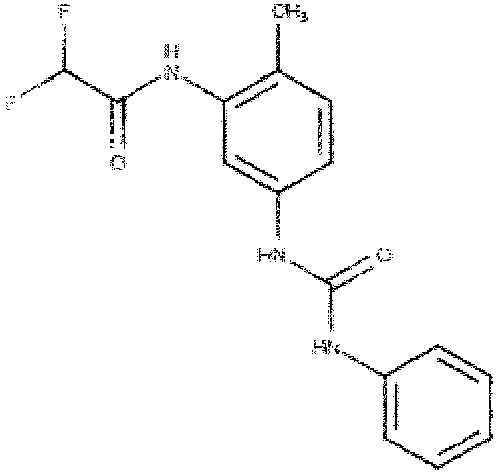 |
| L01.101 | 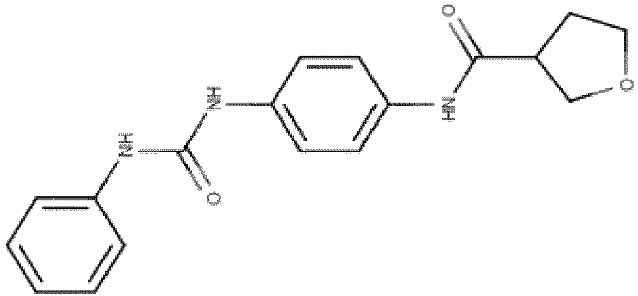 |
| L01.092 | 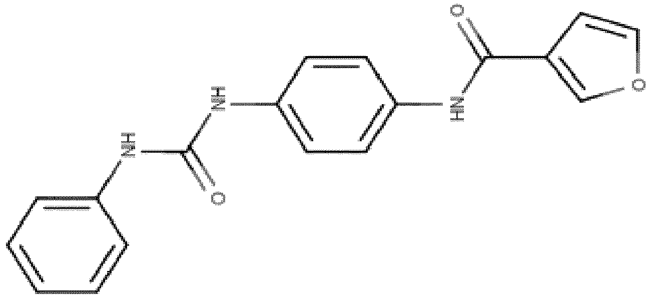 |
| L01.124 | 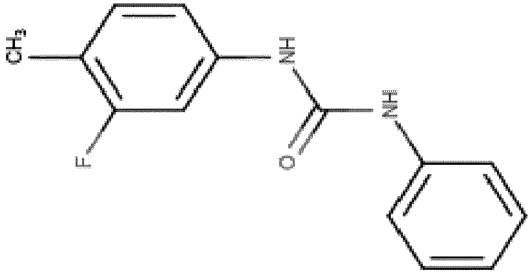 |
| L01.016 | 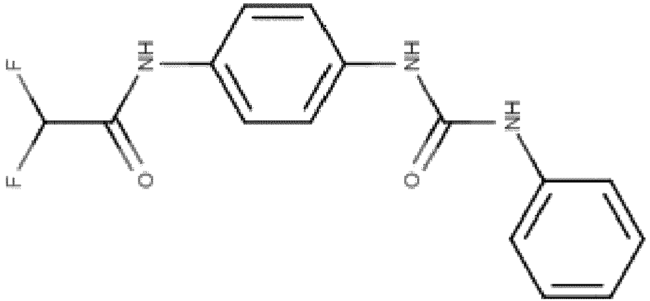 |
| L01.028 | 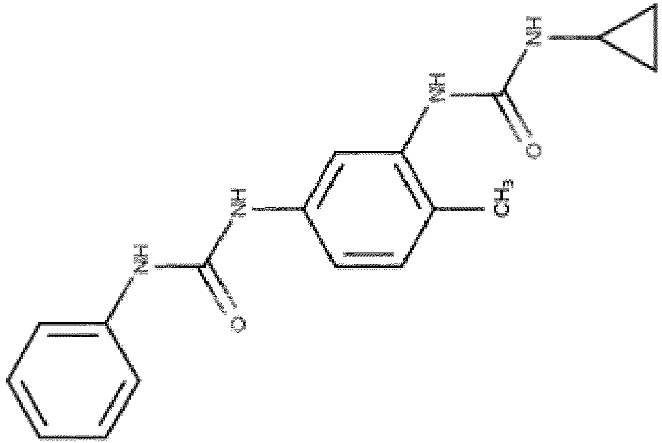 |
| L01.047 | 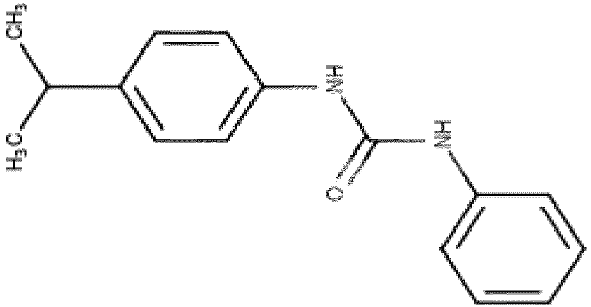 |
| L01.026 | 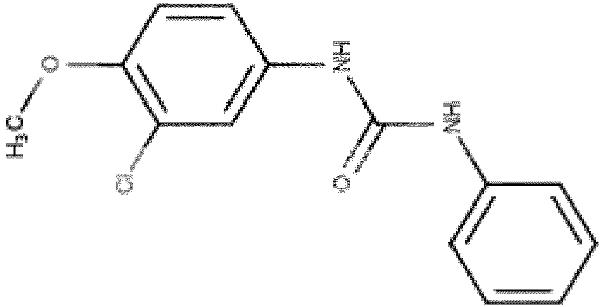 |
| L01.007 | 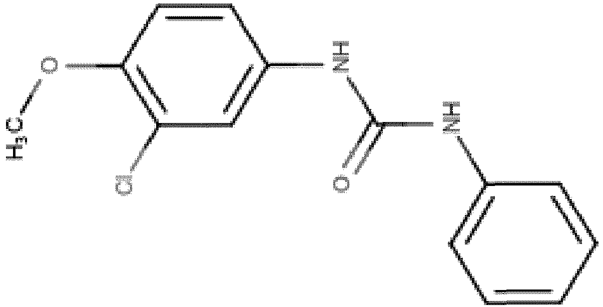 |
| EN22 | 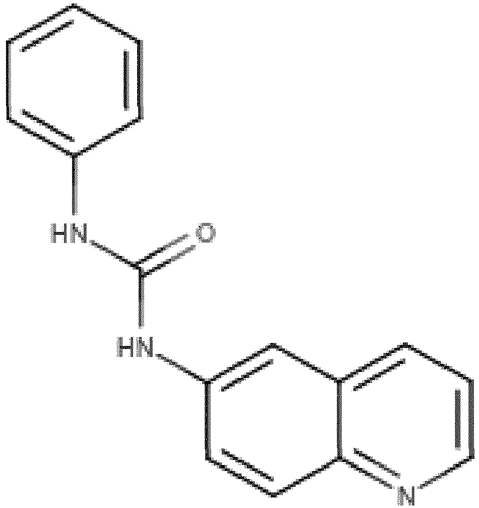 |
| L01.035 | 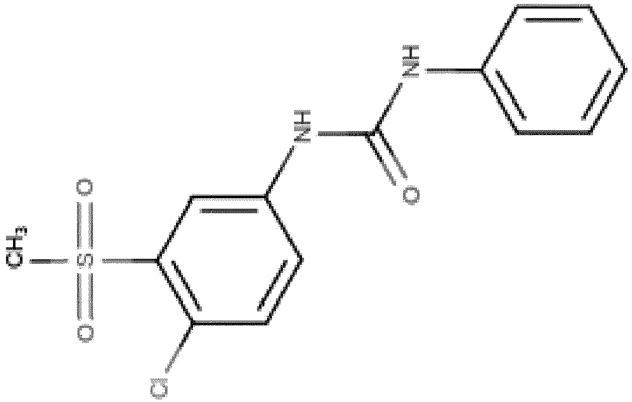 |
| EN23 | 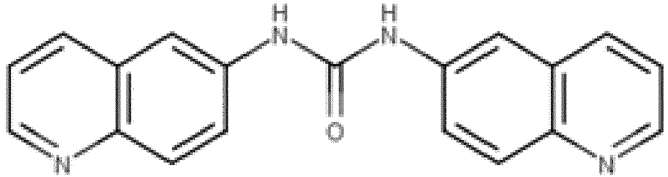 |

| No. | Structure Formula (Ib) |
|---|---|
| L01.113 | 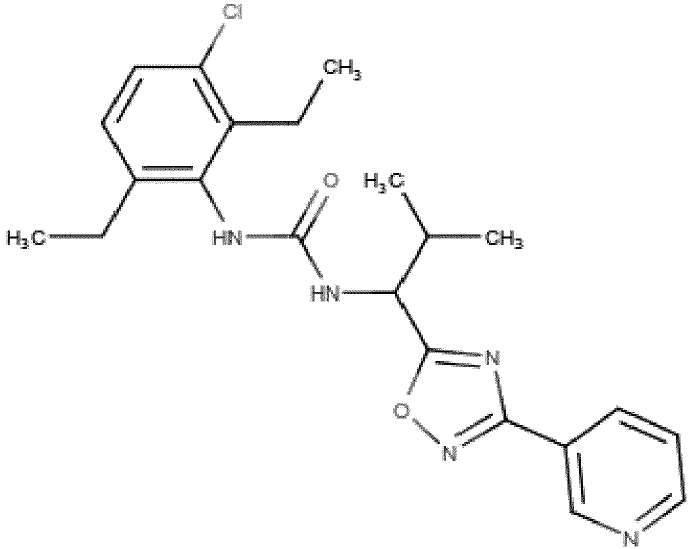 |
| L01.130 | 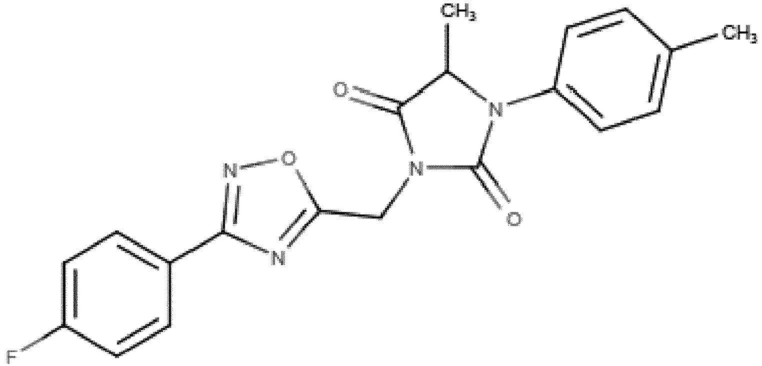 |
| L01.049 | 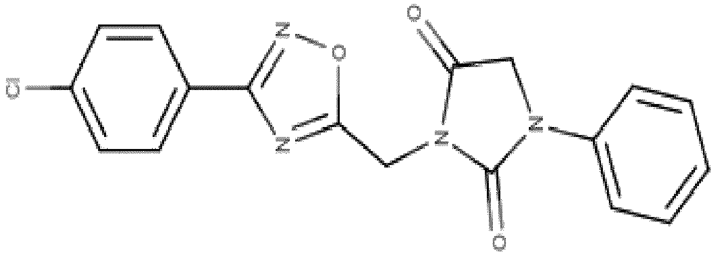 |
| EN21 | 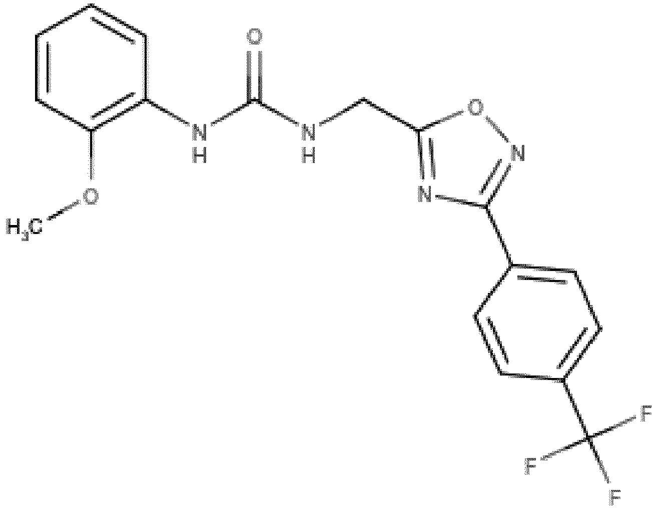 |

According to the one aspect, the present invention relates to a compound of Formula (II),

R²¹-B-CO-R²²

or a pharmaceutically acceceptable salt thereof, wherein:
B is an optionanlly substituted heteroaromatic bicycle selected from the group comprising 2-benzofuran, 2-benzothiophene, isoindol, indol, indolizine and purine and
R²¹ is an optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, and
R²² is optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, -OR²³, -NR²³, and wherein
R²³ is hydrogen, optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl.

In one embodiment of Formula II, B is isoindol, indol, or preferably indolizine. In one embodiment, the present invention relates to a compound of Formula (II), wherein the compound is a compound of Formula (IIa):

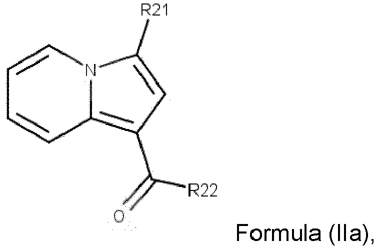

In one embodiment, the present invention relates to a compound of Formula (II) or (IIa), wherein R²² is -OR²³, -NR²³, wherein R²² is preferably -NR²³ and wherein
R²³ is hydrogen, optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl.

In one embodiment, the present invention relates to a compound of Formula (IIa), wherein Formula (IIa) is represented by Formula (IIb) 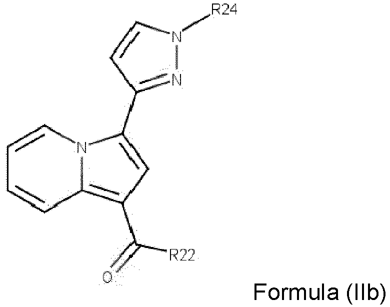 wherein R²² is optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, -OR²³, -NR²³, and
R²³ is hydrogen, optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl and
wherein R²⁴ is hydrogen, optionally substituted C₁-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, and wherein R²⁴ is preferably optionally substituted C₃-C₂₀ cycloalkyl or C₃-C₂₀ cycloalkyl C₁-C₁₀ alkyl.

In one embodiment, the present invention relates to a compound of Formula (IIb), wherein R²² is -OR²³, -NR²³, wherein R²² is preferably -NR²³ and wherein
R²³ is, optionally substituted C₂-C₂₀ alkyl, optionally substituted C₂-C₂₀ alkenyl, optionally substituted 2-20 membered heteroalkyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, and
wherein R²⁴ is hydrogen, optionally substituted C₁-C₂₀ alkyl, optionally substituted C₂-C₂₀ alkenyl, optionally substituted C₆-C₁₈ aryl or C₆-C₁₈ aryl C₁-C₁₀ alkyl; optionally substituted 5-20 membered heteroaryl or 5-20 membered heteroaryl C₁-C₁₀ alkyl; optionally substituted C₃-C₂₀ cycloalkyl or C₃-C₂₀ cycloalkyl C₁-C₁₀ alkyl; optionally substituted 3-20 membered cycloheteroalkyl or 3-20 membered cycloheteroalkyl C₁-C₁₀ alkyl, and wherein R²⁴ is preferably optionally substituted C₃-C₂₀ cycloalkyl or C₃-C₂₀ cycloalkyl C₁-C₁₀ alkyl.

In one embodiment, the present invention relates to a compound of Formula (IIb), wherein the compound is

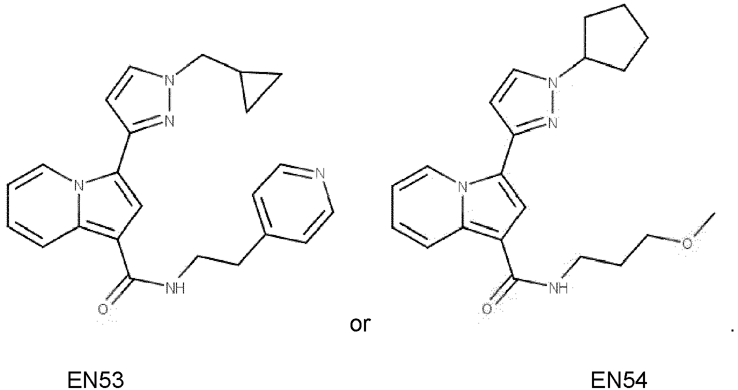

In one embodiment, the present invention relates to the present compounds, wherein the present compound induces NLRP3 inflammasome activation, thereby increasing the release of IL-1β of 0.1x10⁶ BMDCs upon administering the compound in a concentration of 50 µM of the compound and incubation of 2 hours. The increase of the release of the amount of IL-1β corresponds preferably to at least a twofold increase, at least a threefold increase and an even more preferably at least a tenfold increase of the amounts of IL-1β in comparison to BMDCs that are not administered the active compound. In one embodiment, the present invention relates to the present compounds, wherein the present compound induces NLRP3 inflammasome activation, thereby preferably increasing the release of IL-1β of 0.1x10⁶ BMDCs upon administering the compound in a concentration of 50 µM of the compound and incubation of 2 hours, wherein the increase is at least 0.1 ng ml⁻¹, 0.5 ng ml⁻¹, preferably 2 ng ml⁻¹, 10 ng ml⁻¹, 25 ng ml⁻¹, 50 ng ml⁻¹ and more preferably 4 ng ml⁻¹ or more. The increase of the release of IL-1β of 0.1x10⁶ BMDCs can be measured with the method described in Example 1.8.

### Pharmaceutical Compositions and Administration

### General

In some embodiments, a compound of the present invention (e.g., a compound that modulates e.g. agonizes) NLRP3, or a pharmaceutically acceptable salt, and/or hydrate, and/or cocrystal, and/or drug combination thereof is administered as a pharmaceutical composition that includes the compound of the invention and one or more pharmaceutically acceptable excipients, and optionally one or more additional therapeutic agents as described herein.

In some embodiments, the present compounds can be administered in combination with one or more conventional pharmaceutical excipients. Pharmaceutically acceptable excipients include, but are not limited to, ion exchangers, alumina, aluminum stearate, lecithin, self-emulsifying drug delivery systems (SEDDS) such as d-a-tocopherol polyethylene glycol 1000 succinate, surfactants used in pharmaceutical dosage forms such as Tweens, poloxamers or other similar polymeric delivery matrices, serum proteins, such as human serum albumin, buffer substances such as phosphates, tris, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium-chloride, zinc salts, colloidal silica, magnesium trisilicate, polyvinyl pyrrolidone, cellulose-based substances, polyethylene glycol, sodium carboxymethyl cellulose, polyacrylates, waxes, polyethylene-polyoxypropylene-block polymers, and wool fat. Cyclodextrins such as α-, β, and γ-cyclodextrin, or chemically modified derivatives such as hydroxyalkyl cyclodextrins, including 2- and 3- hydroxypropyl-P-cyclodextrins, or other solubilized derivatives can also be used to enhance delivery of compounds described herein. Dosage forms or compositions containing a compound of the invention as described herein in the range of 0.005% to 100% with the balance made up from non-toxic excipient may be prepared. The contemplated compositions may contain 0.001%-100% of a compound of the invention provided herein, in one embodiment 0.1-95%, in another embodiment 75-85%, in a further embodiment 20-80%. Actual methods of preparing such dosage forms are known, or will be apparent, to those skilled in this art; for example, see Remington: The Science and Practice of Pharmacy, 22nd Edition (Pharmaceutical Press, London, UK. 2012).

### Routes of Administration and Composition Components

In some embodiments, the present compounds described herein or a pharmaceutical composition thereof can be administered to subject in need thereof by any accepted route of administration. Acceptable routes of administration include, but are not limited to, buccal, cutaneous, endocervical, endosinusial, endotracheal, enteral, epidural, interstitial, intra-abdominal, intra-arterial, intrabronchial, intrabursal, intracerebral, intraci sternal, intracoronary, intradermal, intraductal, intraduodenal, intradural, intraepidermal, intraesophageal, intragastric, intragingival, intraileal, intralymphatic, intramedullary, intrameningeal, intramuscular, intraovarian, intraperitoneal, intraprostatic, intrapulmonary, intrasinal, intraspinal, intrasynovial, intratesticular, intrathecal, intratubular, intratumoral, intrauterine, intravascular, intravenous, nasal, nasogastric, oral, parenteral, percutaneous, peridural, rectal, respiratory (inhalation), subcutaneous, sublingual, submucosal, topical, transdermal, transmucosal, transtracheal, ureteral, urethral and vaginal. In certain embodiments, a preferred route of administration is parenteral (e.g., intratumoral). In certain embodiments, a preferred route of administration is systemic.

Compositions can be formulated for parenteral administration, e.g., formulated for injection via the intravenous, intramuscular, sub-cutaneous, or even intraperitoneal routes. Typically, such compositions can be prepared as injectables, either as liquid solutions, emulsions or suspensions; solid forms suitable for use to prepare solutions, emulsions or suspensions upon the addition of a liquid prior to injection can also be prepared; and the preparations can also be emulsified. The preparation of such formulations will be known to those of skill in the art in light of the present disclosure.

The pharmaceutical forms suitable for injectable use include sterile aqueous solutions or dispersions; formulations including sesame oil, peanut oil, or aqueous propylene glycol; and sterile powders for the extemporaneous preparation of sterile injectable solutions or dispersions. In all cases the form must be sterile and must be fluid to the extent that it may be easily injected. It also should be stable under the conditions of manufacture and storage and must be preserved against the contaminating action of microorganisms, such as bacteria and fungi.

The carrier also can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating, such as lecithin, by the maintenance of the required particle size in the case of dispersion, and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compounds in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques, which yield a powder of the active ingredient, plus any additional desired ingredient from a previously sterile-filtered solution thereof. Intratumoral injections are discussed, e.g., in Lammers, et al., "Effect of Intratumoral Injection on the Biodistribution and the Therapeutic Potential of HPMA Copolymer-Based Drug Delivery Systems" Neoplasia. 2006, 10, 788-795.

Pharmacologically acceptable excipients usable in the rectal composition as a gel, cream, enema, or rectal suppository, include, without limitation, any one or more of cocoa butter glycerides, synthetic polymers such as polyvinylpyrrolidone, PEG (like PEG ointments), glycerine, glycerinated gelatin, hydrogenated vegetable oils, poloxamers, mixtures of polyethylene glycols of various molecular weights and fatty acid esters of polyethylene glycol Vaseline, anhydrous lanolin, shark liver oil, sodium saccharinate, menthol, sweet almond oil, sorbitol, sodium benzoate, anoxid SBN, vanilla essential oil, aerosol, parabens in phenoxyethanol, sodium methyl p-oxybenzoate, sodium propyl p- oxybenzoate, diethylamine, carbomers, carbopol, methyloxybenzoate, macrogol cetostearyl ether, cocoyl caprylocaprate, isopropyl alcohol, propylene glycol, liquid paraffin, xanthan gum, carboxy-metabisulfite, sodium edetate, sodium benzoate, potassium metabi sulfite, grapefruit seed extract, methyl sulfonyl methane (MSM), lactic acid, glycine, vitamins, such as vitamin A and E and potassium acetate.

In certain embodiments, suppositories can be prepared by mixing the present compound described herein with suitable non-irritating excipients or carriers such as cocoa butter, polyethylene glycol or a suppository wax which are solid at ambient temperature but liquid at body temperature and therefore melt in the rectum and release the active compound. In other embodiments, compositions for rectal administration are in the form of an enema.

In other embodiments, the compounds described herein or a pharmaceutical composition thereof are suitable for local delivery to the digestive or GI tract by way of oral administration (e.g., solid or liquid dosage forms.).

Solid dosage forms for oral administration include capsules, tablets, pills, powders, and granules. In such solid dosage forms, the acompound of the invention is mixed with one or more pharmaceutically acceptable excipients, such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like.

In one embodiment, the compositions will take the form of a unit dosage form such as a pill or tablet and thus the composition may contain, along with a compound of the invention provided herein, a diluent such as lactose, sucrose, dicalcium phosphate, or the like; a lubricant such as magnesium stearate or the like; and a binder such as starch, gum acacia, polyvinylpyrrolidine, gelatin, cellulose, cellulose derivatives or the like. In another solid dosage form, a powder, marume, solution or suspension (e.g., in propylene carbonate, vegetable oils, PEG's, poloxamer 124 or triglycerides) is encapsulated in a capsule (gelatin or cellulose base capsule). Unit dosage forms in which one or more of the present compounds provided herein or additional active agents are physically separated are also contemplated; e.g., capsules with granules (or tablets in a capsule) of each drug; two-layer tablets; two- compartment gel caps, etc. Enteric coated or delayed release oral dosage forms are also contemplated.

Other physiologically acceptable compounds include wetting agents, emulsifying agents, dispersing agents or preservatives that are particularly useful for preventing the growth or action of microorganisms. Various preservatives are well known and include, for example, phenol and ascorbic acid. In certain embodiments the excipients are sterile and generally free of undesirable matter. These compositions can be sterilized by conventional, well-known sterilization techniques. For various oral dosage form excipients such as tablets and capsules sterility is not required. The USP/NF standard is usually sufficient.

In certain embodiments, solid oral dosage forms can further include one or more components that chemically and/or structurally predispose the composition for delivery of the compound of the invention to the stomach or the lower GI; e.g., the ascending colon and/or transverse colon and/or distal colon and/or small bowel. Exemplary formulation techniques are described in, e.g., Filipski, K.J., et al., Current Topics in Medicinal Chemistry, 2013, 13, 776-802, which is incorporated herein by reference in its entirety.

Examples include upper-GI targeting techniques, e.g., Accordion Pill (Intec Pharma), floating capsules, and materials capable of adhering to mucosal walls.

Other examples include lower-GI targeting techniques. For targeting various regions in the intestinal tract, several enteric/pH-responsive coatings and excipients are available. These materials are typically polymers that are designed to dissolve or erode at specific pH ranges, selected based upon the GI region of desired drug release. These materials also function to protect acid labile drugs from gastric fluid or limit exposure in cases where the active ingredient may be irritating to the upper GI (e.g., hydroxypropyl methylcellulose phthalate series, Coateric (polyvinyl acetate phthalate), cellulose acetate phthalate, hydroxypropyl methylcellulose acetate succinate, Eudragit series (methacrylic acid-methyl methacrylate copolymers), and Marcoat). Other techniques include dosage forms that respond to local flora in the GI tract, Pressure-controlled colon delivery capsule, and Pulsincap. Ocular compositions can include, without limitation, one or more of any of the following: viscogens (e.g., Carboxymethylcellulose, Glycerin, Polyvinylpyrrolidone, Polyethylene glycol); Stabilizers (e.g., Pluronic (triblock copolymers), Cyclodextrins); Preservatives (e.g., Benzalkonium chloride, EDTA, SofZia (boric acid, propylene glycol, sorbitol, and zinc chloride; Alcon Laboratories, Inc.), Purite (stabilized oxychloro complex; Allergan, Inc.)). Topical compositions can include ointments and creams. Ointments are semisolid preparations that are typically based on petrolatum or other petroleum derivatives. Creams containing the selected active agent are typically viscous liquid or semisolid emulsions, often either oil-in-water or water-in-oil. Cream bases are typically water-washable, and contain an oil phase, an emulsifier and an aqueous phase. The oil phase, also sometimes called the "internal" phase, is generally comprised of petrolatum and a fatty alcohol such as cetyl or stearyl alcohol; the aqueous phase usually, although not necessarily, exceeds the oil phase in volume, and generally contains a humectant. The emulsifier in a cream formulation is generally a nonionic, anionic, cationic or amphoteric surfactant. As with other carriers or vehicles, an ointment base should be inert, stable, nonirritating and non- sensitizing.

In any of the foregoing embodiments, pharmaceutical compositions described herein can include one or more one or more of the following: lipids, interbilayer crosslinked multilamellar vesicles, biodegradeable poly(D,L-lactic-co-glycolic acid) [PLGA]-based or poly anhydride-based nanoparticles or microparticles, and nanoporous particle-supported lipid bilayers.

### Dosages

The dosages may be varied depending on the requirement of the patient, the severity of the condition being treating and the particular compound being employed. Determination of the proper dosage for a particular situation can be determined by one skilled in the medical arts. The total daily dosage may be divided and administered in portions throughout the day or by means providing continuous delivery.

In some embodiments, the compounds described herein are administered at a dosage of from about 0.0001 mg kg⁻¹ to about 1000 mg kg⁻¹ (e.g., from about 0.001 mg kg⁻¹ to about 200 mg kg⁻¹; from about 0.01 mg kg⁻¹ to about 200 mg kg⁻¹; from about 0.01 mg kg⁻¹ to about 150 mg kg⁻¹; from about 0.01 mg kg⁻¹ to about 100 mg kg⁻¹; from about 0.01 mg kg⁻¹ to about 50 mg kg⁻¹; from about 0.01 mg kg⁻¹ to about 10 mg kg⁻¹; from about 0.01 mg kg⁻¹ to about 5 mg kg⁻¹; from about 0.01 mg kg⁻¹ to about 1 mg kg⁻¹; from about 0.01 mg kg⁻¹ to about 0.5 mg kg⁻¹; from about 0.01 mg kg⁻¹ to about 0.1 mg kg⁻¹; from about 0.1 mg kg⁻¹ to about 200 mg kg⁻¹; from about 0.1 mg kg⁻¹ to about 150 mg kg⁻¹; from about 0.1 mg kg⁻¹ to about 100 mg kg⁻¹; from about 0.1 mg kg⁻¹ to about 50 mg kg⁻¹; from about 0.1 mg kg⁻¹ to about 10 mg kg⁻¹; from about 0.1 mg kg⁻¹ to about 5 mg kg⁻¹; from about 0.1 mg kg⁻¹ to about 1 mg kg⁻¹; from about 0.1 mg kg⁻¹ to about 0.5 mg kg⁻¹).

### Regimens

The foregoing dosages can be administered on a daily basis (e.g., as a single dose or as two or more divided doses) or non-daily basis (e.g., every other day, every two days, every three days, once weekly, twice weeks, once every two weeks, once a month).

In some embodiments, the period of administration of a compound described herein is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 1 1 months, 12 months, or more. In a further embodiment, a period of during which administration is stopped is for 1 day, 2 days, 3 days, 4 days, 5 days, 6 days, 7 days, 8 days, 9 days, 10 days, 1 1 days, 12 days, 13 days, 14 days, 3 weeks, 4 weeks, 5 weeks, 6 weeks, 7 weeks, 8 weeks, 9 weeks, 10 weeks, 1 1 weeks, 12 weeks, 4 months, 5 months, 6 months, 7 months, 8 months, 9 months, 10 months, 11 months, 12 months, or more. In an embodiment, a therapeutic compound is administered to an individual for a period of time followed by a separate period of time. In another embodiment, a therapeutic compound is administered for a first period and a second period following the first period, with administration stopped during the second period, followed by a third period where administration of the therapeutic compound is started and then a fourth period following the third period where administration is stopped. In an aspect of this embodiment, the period of administration of a therapeutic compound followed by a period where administration is stopped is repeated for a determined or undetermined period of time. In a further embodiment, a period of administration is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more. In a further embodiment, a period of during which administration is stopped is for 1 day, 2 days, 3 days, 4 days, 5 days, 6 days, 7 days, 8 days, 9 days, 10 days, 11 days, 12 days, 13 days, 14 days, 3 weeks, 4 weeks, 5 weeks, 6 weeks, 7 weeks, 8 weeks, 9 weeks, 10 weeks, 11 weeks, 12 weeks, 4 months, 5 months, 6 months, 7 months, 8 months, 9 months, 10 months, 11 months, 12 months, or more.

### Methods of Treatment

In some embodiments, methods for treating a subject having condition, disease or disorder in which a decrease or increase in NLRP3 activity (e.g., a decrease, e.g., repressed or impaired NLRP3 signaling) contributes to the pathology and/or symptoms and/or progression of the condition, disease or disorder (e.g., cancer) are provided.

### Indications

In any of the methods described herein, the subject can have a cancer. In some examples of any of the methods described herein, the mammal has been identified as having a cancer, or has been diagnosed as having a cancer.

Non-limiting examples of cancer include: acute myeloid leukemia, adrenocortical carcinoma, Kaposi sarcoma, lymphoma, anal cancer, appendix cancer, teratoid/rhabdoid tumor, basal cell carcinoma, bile duct cancer, bladder cancer, bone cancer, brain cancer, breast cancer, bronchial tumor, carcinoid tumor, cardiac tumor, cervical cancer, chordoma, chronic lymphocytic leukemia, chronic myeloproliferative neoplasm, colon cancer, colorectal cancer, craniopharyngioma, bile duct cancer, endometrial cancer, ependymoma, esophageal cancer, esthesioneuroblastoma, Ewing sarcoma, eye cancer, fallopian tube cancer, gallbladder cancer, gastrointestinal carcinoid tumor, gastrointestinal stromal tumor, germ cell tumor, hairy cell leukemia, head and neck cancer, heart cancer, liver cancer, hypopharngeal cancer, pancreatic cancer, kidney cancer, laryngeal cancer, chronic myelogenous leukemia, lip and oral cavity cancer, lung cancer, melanoma, Merkel cell carcinoma, mesothelioma, mouth cancer, oral cancer, osteosarcoma, ovarian cancer, penile cancer, pharyngeal cancer, prostate cancer, rectal cancer, salivary gland cancer, skin cancer, small intestine cancer, soft tissue sarcoma, testicular cancer, throat cancer, thyroid cancer, urethral cancer, uterine cancer, vaginal cancer, and vulvar cancer.

Methods for diagnosing a subject as having a cancer or identifying a mammal as having a cancer are well known in the art. For example, a medical professional (e.g., a physician, a physician's assistant, or a technician) can diagnose cancer in a mammal by observing one or more symptoms of cancer in a mammal. Non-limiting examples of symptoms of cancer include: fatigue, lump or area of thickening felt under the skin, weight change, jaundice, darkening or redness of the skin, sores that won't heal, changes to existing moles, changes in bowel or bladder habits, persistent cough or trouble breathing, difficulty swallowing, hoarseness, persistent indigestion or discomfort after eating, persistent, unexplained muscle or joint pain, persistent, unexplained fevers or night sweats, and unexplained bleeding or bruising. Methods of diagnosing a subject as having a cancer or identifying a subject as having a cancer can further include performing one or more diagnostic tests (e.g., performing one or more diagnostic tests on a biopsy or a blood sample).

In some examples of any of the methods described herein, a subject can be a subject having a cancer, a subject diagnosed as having a cancer, or a subject identified as having a cancer that has been unresponsive to a previously administered treatment for cancer. Diagnostic tests for diagnosing a subject as having a cancer or identifying a mammal as having a cancer are known in the art.

In any of the methods described herein, the subject can have an infectious disease. In some examples of any of the methods described herein, the subject has been identified as having an infectious disease, or has been diagnosed as having an infectious disease. For example, an infectious disease can be caused by a bacterium, virus, fungus, or a parasite.

Non-limiting examples of infectious disease include: Acinetoobacter infection, actinomycosis, African sleeping sickness, acquired immunodeficiency syndrome, amebiasis, anaplasmosis, anthrax, Arcanobacterium haemolyticum infection, Argentine hemorrhagic fever, ascariasis, aspergillosis, astrovirus infection, babesiosis, Bacillus cereus infection, bacterial pneumonia, bacterial vaginosis, Bacteroides infection, balantidiasis, Baylisascaris infection, BK virus infection, black piedra, Blastocystic hominis infection, blastomycosis, Bolivian hemorrhagic fever, botulism, Brazilian hemorrhagic fever, brucellosis, bubonic plaque, Burkholderi infection, Buruli ulcer, Calicivirus infection, camptobacteriosis, candidiasis, cat-scratch disease, cellulitis, Chagas disease, chancroid, chickenpox, chikungunya, chlamydia, Chlamydophila pneumoniae infection, cholera, chromoblastomycosis, clonorchiasis, Clostridium difficile infection, coccidioidomycosis, Colorado tick fever, common cold, Creutzfeldt-Jakob disease, Crimean-Congo hemorrhagic fever, crytococcosis, cryptosporidiosis, cutaneous larva migrans, cyclosporiasis, cysticercosis, cytomegalovirus infection, dengue fever, Desmodesmus infection, deintamoebiasis, diphtheria, diphyllobothriasis, dracunculiasis, ebola hemorrhagic fever, echinococcosis, ehrlichiosis, enterobiasis, Enterococcus infection, Enterovirus infection, epidemic typhus, erythema infection, exanthema subitum, fasciolopsiasis, fasciolosis, fatal familial insomnia, filariasis, food poisoning by Clostridium myonecrosis, free-living amebic infection, Fusobacterium infection, gas gangrene, geotrichosis, Gerstmann-Straussler-Scheinker syndrome, giardiasis, glanders, gnathostomiasis, gonorrhea, granuloma inguinale, Group A streptococcal infection, Group B streptococcal infection, Haemophilus influenzae infection, hand foot and mouth disease, hantavirus pulmonary syndrome, Heartland virus disease, Heliobacter pylori infection, hemolytic-uremic syndrome, hemorrhagic fever with renal syndrome, hepatitis A, hepatitis B, hepatitis C, hepatitis D, hepatitis E, herpes simplex, histoplasmosis, hookworm infection, human bocavirus infection, human ewingii ehrlichiosis, human granulocyte anaplasmosis, human metapneuomovirus infection, human monocytic ehrlichiosis, human papillomavirus infection, human parainfluenza virus infection, hymenolepiasis, Epstein- Barr virus infectious mononucleosis, influenza, isosporiasis, Kawasaki disease, keratitis, Kingella kingae infection, kuru, lassa fever, Legionnaires' disease, Pontiac fever, leishmaniasis, leprosy, leptospirosis, listeriosis, lyme disease, lymphatic filariasis, lymphocytic choriomeningitis, malaria, Marburg hemorrhagic fever, measles, Middle East respiratory syndrome, melioidosis, meningitis, meningococcal disease, metagonimiasis, microsporidiosis, molluscum contagiosum, monkeypox, mumps, murine typhus, mycoplasma pneumonia, mycetoma, myiasis, neonatal conjunctivitis, variant Creutzfeldt- Jakob disease, nocardiosis, onchocerciasis, paracoccidioidomycosis, paragonimiasis, pasteurellosis, pediculosis capitis, pediculosis corporis, pediculosis pubis, pelvic inflammatory disease, pertussis, plague, pneumonia, poliomyelitis, Prevotella infection, primary amoebic meningoencephalitis, progressive multifocal leukoencephalopathy, psittacosis, Q fever, rabies, relapsing fever, respiratory syncytial virus infection, rhinosporidiosis, rhinovirus infection, rickettsial infection, rickettsialpox, Rift Valley Fever, Rocky Mountain spotted fever, rotavirus infection, rubella, salmonellosis, severe acute respiratory syndrome, scabies, schistosomiasis, sepsis, shigellosis, shingles, smallpox, sporothrichosis, staphylococcal food poisoning, staphylococcal infection, staphylococcal infection, strongyloidiasis, subacute sclerosing panencephalitis, syphilis, taeniasis, tetanus, tinea barabe, tinea capitis, tinea corporis, tinea cruris, tinea manum, tinea nigra, tinea pedis, tinea unguium, tinea versicolor, toxocariasis, trachoma, toxoplasmosis, trichinosis, trichomoniasis, trichuriasis, tuberculosis, tularemia, typhoid fever, Ureaplasma urealyticum infection, valley fever, Venezuelan hemorrhagic fever, viral pneumonia, West Nile fever, white piedra, Yersinia psuedotuberculosis infection, yersiniosis, yellow fever, and zygomycosis.

Methods for diagnosing a subject as having an infectious disease, or identifying a subject as having an infectious disease are well known in the art. For example, a medical professional (e.g., a physician, a physician's assistant, or a technician) can diagnose infectious disease in a subject by observing one or more symptoms of infectious disease in a subject. Non-limiting examples of symptoms of infectious disease include: fever, diarrhea, fatigue, and muscle aches. Methods of diagnosing a mammal as having an infectious disease or identifying a subject as having an infectious disease can further include performing one or more diagnostic tests (e.g., performing one or more diagnostic tests on a biopsy or a blood sample). Diagnostic tests for diagnosing a subject as having an infectious disease or identifying a subject as having an infectious disease are known in the art. With regard to the combination therapy, this disclosure contemplates both monotherapy regimens as well as combination therapy regimens.

In some embodiments, the methods described herein can further include administering one or more additional therapies (e.g., one or more additional therapeutic agents and/or one or more therapeutic regimens) in combination with administration of the compounds described herein.

In certain embodiments, the methods described herein can further include administering one or more additional cancer therapies.

The one or more additional cancer therapies can include, without limitation, surgery, radiotherapy, chemotherapy, toxin therapy, immunotherapy, cryotherapy, cancer vaccines (e.g., HPV vaccine, hepatitis B vaccine, Oncophage, Provenge) and gene therapy, as well as combinations thereof. Immunotherapy, including, without limitation, adoptive cell therapy, the derivation of stem cells and/or dendritic cells, blood transfusions, lavages, and/or other treatments, including, without limitation, freezing a tumor.

In some embodiments, the one or more additional cancer therapies is chemotherapy, which can include administering one or more additional chemotherapeutic agents. In certain embodiments, the additional chemotherapeutic agent is an immunomodulatory moiety, e.g., an immune checkpoint inhibitor. In certain of these embodiments, the immune checkpoint inhibitor targets an immune checkpoint receptor selected from the group consisting of CTLA-4, PD-1, PD-L1, PD-1 - PD-L1, PD-1 - PD- L2, T cell immunoglobulin and mucin 3 (TEVI3 or HAVCR2), Galectin 9 - TEVI3, Phosphatidylserine - TIM3, lymphocyte activation gene 3 protein (LAG3), MHC class II, - LAG3, 4- 1BB-4- 1BB ligand, OX40-OX40 ligand, GITR, GITR ligand - GITR, CD27, CD70-CD27, T FRSF25, TNFRSF25-TL1 A, CD40L, CD40-CD40 ligand, HVEM- LIGHT-LTA, HVEM, HVEM - BTLA, HVEM - CD 160, HVEM - LIGHT, HVEM- BTLA-CD160, CD80, CD80 - PD-L1, PD-L2 - CD80, CD244, CD48 - CD244, CD244, ICOS, ICOS-ICOS ligand, B7-H3, B7-H4, VISTA, TMIGD2, HHLA2-TMIGD2, Butyrophilins, including BTNL2, Siglec family, TIGIT and PVR family members, K Rs, ILTs and LIRs, NKG2D and NKG2A, MICA and MICB, CD244, CD28, CD86 - CD28, CD86 - CTLA, CD80 - CD28, Phosphatidylserine, TIM3, Phosphatidylserine - TIM3, SIRPA-CD47, VEGF, Neuropilin, CD160, CD30, and CD155 (e.g., CTLA-4 or PD-1 or PD-L1) and other immunomodulatory agents, such as interleukin-2 (IL-2), indoleamine 2,3-dioxygenase (IDO), IL- 10, transforming growth factor-β (TGFp), CD39, CD73, Adenosine-CD39-CD73, and CXCR4-CXCL 12. See, e.g., Postow, M. J. Clin. Oncol. 2015, 33, 1. In certain of these embodiments, the immune checkpoint inhibitor is selected from the group consisting of: Urelumab, PF-05082566, MEDI6469, TRX518, Varlilumab, CP-870893, Pembrolizumab (PD-1), Nivolumab (PD-1), Atezolizumab (formerly MPDL3280A) (PD-L1), MEDI4736 (PD-L1), Avelumab (PD-L1), PDR001 (PD-1), BMS-986016, MGA271, Lirilumab, IPH2201, Emactuzumab, INCB024360, Galunisertib, Ulocuplumab, BKT140, Bavituximab, CC-90002, Bevacizumab, and MNRP1685A, and MGA271.

In certain embodiments, the additional chemotherapeutic agent is a STING agonist. For example, the STING agonist can include cyclic di-nucleotides, such as cAMP, cGMP, and cGAMP as well as modified cyclic di-nucleotides that include one or more of the following modification features (2'-0/3'-0 linkage, phosphorothioate linkage, adenine and/or guanine analogue, 2'-OH modification (e.g., -OCH3 or replacement, e.g., -F or N3). See, e.g., WO 2014/189805. In certain embodiments, the additional chemotherapeutic agent is an alkylating agent. Alkylating agents are so named because of their ability to alkylate many nucleophilic functional groups under conditions present in cells, including, but not limited to cancer cells. In a further embodiment, an alkylating agent includes, but is not limited to, Cisplatin, carboplatin, mechlorethamine, cyclophosphamide, chlorambucil, ifosfamide and/or oxaliplatin. In an embodiment, alkylating agents can function by impairing cell function by forming covalent bonds with the amino, carboxyl, sulfhydryl, and phosphate groups in biologically important molecules or they can work by modifying a cell's DNA. In a further embodiment an alkylating agent is a synthetic, semisynthetic or derivative.

In certain embodiments, the additional chemotherapeutic agent is an anti- metabolite. Antimetabolites masquerade as purines or pyrimidines, the building-blocks of DNA and in general, prevent these substances from becoming incorporated in to DNA during the "S" phase (of the cell cycle), stopping normal development and division. Antimetabolites can also affect RNA synthesis. In an embodiment, an antimetabolite includes, but is not limited to azathioprine and/or mercaptopurine. In a further embodiment an anti- metabolite is a synthetic, semisynthetic or derivative.

In certain embodiments, the additional chemotherapeutic agent is a plant alkaloid and/or terpenoid. These alkaloids are derived from plants and block cell division by, in general, preventing microtubule function. In an embodiment, a plant alkaloid and/or terpenoid is a vinca alkaloid, a podophyllotoxin and/or a taxane. Vinca alkaloids, in general, bind to specific sites on tubulin, inhibiting the assembly of tubulin into microtubules, generally during the M phase of the cell cycle. In an embodiment, a vinca alkaloid is derived, without limitation, from the Madagascar periwinkle, Catharanthus roseus (formerly known as Vinca rosea). In an embodiment, a vinca alkaloid includes, without limitation, Vincristine, Vinblastine, Vinorelbine and/or Vindesine. In an embodiment, a taxane includes, but is not limited, to Taxol, Paclitaxel and/or Docetaxel. In a further embodiment a plant alkaloid or terpernoid is a synthetic, semisynthetic or derivative. In a further embodiment, a podophyllotoxin is, without limitation, an etoposide and/or teniposide. In an embodiment, a taxane is, without limitation, docetaxel and/or ortataxel. In an embodiment, a cancer therapeutic is a topoisomerase. Topoisomerases are essential enzymes that maintain the topology of DNA. Inhibition of type I or type II topoisom erases interferes with both transcription and replication of DNA by upsetting proper DNA supercoiling. In a further embodiment, a topoisomerase is, without limitation, a type I topoisomerase inhibitor or a type II topoisomerase inhibitor. In an embodiment a type I topoisomerase inhibitor is, without limitation, a camptothecin. In another embodiment, a camptothecin is, without limitation, exatecan, irinotecan, lurtotecan, topotecan, BNP 1350, CKD 602, DB 67 (AR67) and/or ST 1481. In an embodiment, a type II topoisomerase inhibitor is, without limitation, epipodophyllotoxin. In a further embodiment an epipodophyllotoxin is, without limitation, an amsacrine, etoposid, etoposide phosphate and/or teniposide. In a further embodiment a topoisomerase is a synthetic, semisynthetic or derivative, including those found in nature such as, without limitation, epipodophyllotoxins, substances naturally occurring in the root of American Mayapple (Podophyllum peltatum).

In certain embodiments, the additional chemotherapeutic agent is a stilbenoid. In a further embodiment, a stilbenoid includes, but is not limited to, Resveratrol, Piceatannol, Pinosylvin, Pterostilbene, Alpha- Viniferin, Ampelopsin A, Ampelopsin E, Diptoindonesin C, Diptoindonesin F, Epsilon- Vinferin, Flexuosol A, Gnetin H, Hemsleyanol D, Hopeaphenol, Trans-Diptoindonesin B, Astringin, Piceid and Diptoindonesin A. In a further embodiment a stilbenoid is a synthetic, semisynthetic or derivative.

In certain embodiments, the additional chemotherapeutic agent is a cytotoxic antibiotic. In an embodiment, a cytotoxic antibiotic is, without limitation, an actinomycin, an anthracenedione, an anthracycline, thalidomide, dichloroacetic acid, nicotinic acid, 2- deoxyglucose and/or chlofazimine. In an embodiment, an actinomycin is, without limitation, actinomycin D, bacitracin, colistin (polymyxin E) and/or polymyxin B. In another embodiment, an antracenedione is, without limitation, mitoxantrone and/or pixantrone. In a further embodiment, an anthracycline is, without limitation, bleomycin, doxorubicin (Adriamycin), daunorubicin (daunomycin), epirubicin, idarubicin, mitomycin, plicamycin and/or valrubicin. In a further embodiment a cytotoxic antibiotic is a synthetic, semisynthetic or derivative.

In certain embodiments, the additional chemotherapeutic agent is selected from endostatin, angiogenin, angiostatin, chemokines, angioarrestin, angiostatin (plasminogen fragment), basement-membrane collagen-derived anti-angiogenic factors (tumstatin, canstatin, or arrestin), anti-angiogenic antithrombin III, signal transduction inhibitors, cartilage-derived inhibitor (CDI), CD59 complement fragment, fibronectin fragment, gro- beta, heparinases, heparin hexasaccharide fragment, human chorionic gonadotropin (hCG), interferon alpha/beta/gamma, interferon inducible protein (IP-10), interleukin-12, kringle 5 (plasminogen fragment), metalloproteinase inhibitors (TIMPs), 2-methoxyestradiol, placental ribonuclease inhibitor, plasminogen activator inhibitor, platelet factor-4 (PF4), prolactin 16 kD fragment, proliferin-related protein (PRP), various retinoids, tetrahydrocortisol-S, thrombospondin-1 (TSP-1), transforming growth factor-beta (TGF- β), vasculostatin, vasostatin (calreticulin fragment) and the like.

In certain embodiments, the additional chemotherapeutic agent is selected from abiraterone acetate, altretamine, anhydrovinblastine, auristatin, bexarotene, bicalutamide, BMS 184476, 2,3,4,5,6-pentafluoro-N-(3-fluoro-4-methoxyphenyl)benzene sulfonamide, bleomycin, N,N-dimethyl-L-valyl-L-valyl-N-methyl-L-valyl-L-proly-I-Lproline-t- butylamide, cachectin, cemadotin, chlorambucil, cyclophosphamide, 3',4'-didehydro-4'- deoxy-8'-norvin-caleukoblastine, docetaxol, doxetaxel, cyclophosphamide, carboplatin, carmustine, cisplatin, cryptophycin, cyclophosphamide, cytarabine, dacarbazine (DTIC), dactinomycin, daunorubicin, decitabine dolastatin, doxorubicin (adriamycin), etoposide, 5- fluorouracil, finasteride, flutamide, hydroxyurea and hydroxyureataxanes, ifosfamide, liarozole, lonidamine, lomustine (CCNU), MDV3100, mechlorethamine (nitrogen mustard), melphalan, mivobulin isethionate, rhizoxin, sertenef, streptozocin, mitomycin, methotrexate, taxanes, nilutamide, onapristone, paclitaxel, prednimustine, procarbazine, RPR109881, stramustine phosphate, tamoxifen, tasonermin, taxol, tretinoin, vinblastine, vincristine, vindesine sulfate, and vinflunine. In certain embodiments, the additional chemotherapeutic agent is platinum, cisplatin, carboplatin, oxaliplatin, mechlorethamine, cyclophosphamide, chlorambucil, azathioprine, mercaptopurine, vincristine, vinblastine, vinorelbine, vindesine, etoposide and teniposide, paclitaxel, docetaxel, irinotecan, topotecan, amsacrine, etoposide, etoposide phosphate, teniposide, 5-fluorouracil, leucovorin, methotrexate, gemcitabine, taxane, leucovorin, mitomycin C, tegafur-uracil, idarubicin, fludarabine, mitoxantrone, ifosfamide and doxorubicin. Additional agents include inhibitors of mTOR (mammalian target of rapamycin), including but not limited to rapamycin, everolimus, temsirolimus and deforolimus.

In still other embodiments, the additional chemotherapeutic agent can be selected from those delineated in U.S. Patent 7,927,613, which is incorporated herein by reference in its entirety. In yet another embodiment, the methods can further include administering one or both of: (i) one or more anti-fungal agents (e.g., selected from the group of bifonazole, butoconazole, clotrimazole, econazole, ketoconazole, luliconazole, miconazole, omoconazole, oxiconazole, sertaconazole, sulconazole, tioconazole, albaconazole, efinaconazole, epoziconazole, fluconazole, isavuconazole, itraconazole, posaconazole, propiconazole, ravusconazole, terconazole, voriconazole, abafungin, amorolfin, butenafine, naftifine, terbinafine, anidulafungin, caspofungin, micafungin, benzoic acid, ciclopirox, flucytosine, 5-fluorocytosine, griseofulvin, haloprogin, tolnaflate, undecylenic acid, and balsam of peru) and (ii) one or more antibiotics (e.g., selected from the group of amikacin, gentamicin, kanamycin, neomycin, netilmicin, tobramycin, paromomycin, streptomycin, spectinomycin, geldanamycin, herbimycin, rifaximin, loracarbef, ertapenem, doripenem, imipenem, cilastatin, meropenem, cefadroxil, cefazolin, cefalotin, cefalothin, cefalexin, cefaclor, cefamandole, cefoxitin, cefprozil, cefuroxime, cefixime, cefdinir, cefditoren, cefoperazone, cefotaxime, cefpodoxime, ceftazidime, ceftibuten, ceftizoxime, ceftriaxone, cefepime, ceftaroline fosamil, ceftobiprole, teicoplanin, vancomycin, telavancin, dalbavancin, oritavancin, clindamycin, lincomycin, daptomycin, azithromycin, clarithromycin, dirithromycin, erythromycin, roxithromycin, troleandomycin, telithromycin, spiramycin, aztreonam, furazolidone, nitrofurantoin, linezolid, posizolid, radezolid, torezolid, amoxicillin, ampicillin, azlocillin, carbenicillin, cloxacillin, dicloxacillin, flucloxacillin, mezlocillin, methicillin, nafcillin, oxacillin, penicillin G, penicillin V, piperacillin, penicillin G, temocillin, ticarcillin, amoxicillin, calvulanate, ampicillin, subbactam, piperacillin, tazobactam, ticarcillin, clavulanate, bacitracin, colistin, polymyxin B, ciprofloxacin, enoxacin, gatifloxacin, gemifloxacin, levofloxacin, lomefloxacin, moxifloxacin, nalidixic acid, norfloxacin, ofloxacin, trovafloxacin, grepafloxacin, sparfloxacin, temafloxacin, mafenide, sulfacetamide, sulfadiazine, silver sulfadiazine, sulfadimethoxine, sulfamethoxazole, sulfanamide, sulfasalazine, sulfisoxazole, trimethoprim-sulfamethoxazole, sulfonamideochrysoidine, demeclocycline, minocycline, oytetracycline, tetracycline, clofazimine, dapsone, dapreomycin, cycloserine, ethambutol, ethionamide, isoniazid, pyrazinamide, rifampicin, rifabutin, rifapentine, streptomycin, arsphenamine, chloramphenicol, fosfomycin, fusidic acid, metronidazole, mupirocin, platensimycin, quinupristin, dalopristin, thiamphenicol, tigecycyline, tinidazole, trimethoprim, and teixobactin).

In certain embodiments, the second therapeutic agent or regimen is administered to the subject prior to contacting with or administering the compound of the invention (e.g., about one hour prior, or about 6 hours prior, or about 12 hours prior, or about 24 hours prior, or about 48 hours prior, or about 1 week prior, or about 1 month prior).

In other embodiments, the second therapeutic agent or regimen is administered to the subject at about the same time as contacting with or administering the compound of the invention. By way of example, the second therapeutic agent or regimen and the compound of the invention are provided to the subject simultaneously in the same dosage form. As another example, the second therapeutic agent or regimen and the compound of the invention are provided to the subject concurrently in separate dosage forms.

In still other embodiments, the second therapeutic agent or regimen is administered to the subject after contacting with or administering the compound of the invention (e.g., about one hour after, or about 6 hours after, or about 12 hours after, or about 24 hours after, or about 48 hours after, or about 1 week after, or about 1 month after).

In some embodiments, the methods described herein further include the step of identifying a subject (e.g., a patient) in need of such treatment (e.g., by way of biopsy, endoscopy, or other conventional method known in the art). In certain embodiments, the NLRP3 protein can serve as a biomarker for certain types of cancer.

In some embodiments, the present compounds, methods, and compositions described herein can be administered to certain treatment-resistant patient populations (e.g., patients resistant to checkpoint inhibitors).

### Uses of the Present Compounds

The compounds of the present invention, or a salt or solvate thereof, can be used as modulators, e.g., agonists, of the NLRP3 receptor in medicaments as well as in the development of biological or diagnostic assays.

In addition, new small molecule NLRP3 activators are useful to study the biology of inflammasome and thus make an important contribution to the fundamental understanding of the activation mechanisms of the NLRP3 inflammasome and enable the targeted development of specific drugs.

The present compounds have been shown to induce typical characteristics of inflammasome activation, including the activation of caspase-1, the release of the biologically active forms of proinflammatory cytokines such as IL-1β (Figures 5A and 5B). Moreover, inflammasome-dependent lytic cell death (pyroptosis) and ASC speck formation are induced by the present compounds (Figure 6 and 7). For the induction of the lytic cell death, a concentration dependence can be observed (Figure 4A -D). Figure 4D shows the EC50 for induction of IL-1β in BMDCs for compounds EN21 and EN22 in comparison to the imidazoquinoline NLRP3 activators imiquimod and CL097, wherein EC50 for EN21 and EN22 is surprisingly 10 times lower than for imiquimod and CL097.

Without wishing to be bound to a theory, it is assumed that the compounds EN21 and EN22 activate the NLRP3 inflammasome, since the observed characteristics of inflammasome activation do not occur in cells isolated from NLRP3 knockout mice (Figure 7) and, in addition, the NLRP3-specific inhibitor MCC950 completely suppresses inflammasome activation (Figure 8).

A further advantage of the present compounds is that the present compounds seem to activate NLRP3 rapidly. Especially with EN22, the inflammasome activation in BMDCs from wild-type mice without inhibitor is almost maximum after only 30 minutes. Thus, a rapid effectiveness of a medicament comprising the compounds of the invention is assumed.

It has further been shown that contrary to other small molecule NLRP3 inflammasome activators like the known activator imiquimod, the present compounds are more specific, since no simultaneous activation of Toll-like receptors (TLR7/ TLR8) occurs and thus the present compounds do not induce the release of further proinflammatory cytokines such as TNF or IL-6 (Figure 9 and 18).

In contrast to other known low molecular weight NLRP3 activators, the present compounds do not inhibit mitochondrial respiration and are therefore less susceptible to unspecific undesired effects (Figure 10).

Moreover, many known NLRP3 activators lead to massive potassium efflux from the cell via different mechanisms, which precedes inflammasome activation. Without wishing to be bound to a theory, it is assumed that the present compounds such as EN21 and EN22 activate the NLRP3 inflammasome independently of potassium efflux from the cell (Figures 11 and 16). It is assumed that reactive oxygen species (ROS) are involved in the mechanism of action of the present compounds, since the ROS scavenger ebselen can inhibit the activation of the inflammasome (Figure 11).

It has further been shown that in contrary to particulate NLRP3 activators such as uric acid crystals (mono sodium urate, MSU) or aluminum hydroxide, the present compounds are not endocyted by immune cells, followed by endolysosomal rupture to induce inflammasome activation. The endocytosis inhibitor cytochalasin D can prevent particle-induced NLRP3 activation, but cannot prevent NLRP3 activation by EN21 and EN22 (Figure 12).

Moreover, an increased lytic cell death and pyroptosis could be observed in BMDCs of wild-type mice treated with 50 µM of the present compounds after incubation 22 hours, in comparison to the solvent only (DMSO), and the known activators nigericin (5µM), imiquimod (R837) and CL097 (each 100µM) (Figure 13).

Finally, an increased IL-1β release from BMDCs of wild-type mice could be observed treated with 50µM of the present compounds, incubation 22 hours, in comparison to the solvent only (DMSO), and the known activators nigericin (5µM), imiquimod (R837) and CL097 (each 100µM) (Figure 14).

Thus, the present compounds are important to the pharmaceutical industry because they can increase or rapidly induce in a selective manner NLRP3 inflammasome.

According to the one aspect, the present invention relates to the present compounds for use as a medicament. In one embodiment, the present compounds for use as a medicament are used in a therapeutically effective amount.

The medicament can be in any physical form, such as a solid, semi-solid, plaster, solution, suspension, lotion, cream, foam, gel, paste, emulsion, or a combination thereof. Examples of the medicament include, but are not limited to, a pharmaceutical composition, an ingestible composition, a personal care composition, and a combination thereof.

According to the one aspect, the present invention relates to the present compounds for use in the prevention or treatment of a disease by activating NLRP3 inflammasome, wherein the disease is selected from a group comprising infectious diseases or cancer.

In one embodiment, the present compounds may be used in the prevention or treatment of a bacterial infection.

In one embodiment, the present compounds may be used in the prevention or treatment of a viral infection. The viral infection may be selected from the group comprising genital warts, cutaneous warts and COVID-19.

In one embodiment, the present compounds may be used in the prevention of an infectious disease by boosting vaccination-mediated immunity against pathogens.

In one embodiment, the present compounds may be used in the prevention or treatment of a cancer. In one embodiment, the present compounds may be used in breaking the immune evasion in cancer diseases. The term "Immune evasion of cancer" or "cancer immune evasion" is the ability of cancer to persist in a host having a functioning immune system. There are a number of factors that contribute to tumor persistence despite having a normal host immune system. Immune editing is one of the key aspects why tumors evade surveillance causing the tumors to lie dormant in patients for years through "equilibrium" and "senescence" before re-emerging.

In one embodiment, the compounds of the present invention may be used as an adjuvant.

In one embodiment, the compounds of the present invention may be used to boost the immune response. The boost of the immune response by a compound of the present invention may be suitable during the prevention or treatment of cancer, an infectious disease or during a vaccination process. Preferably, the compounds of the present invention may be used as an adjuvant in the context of vaccination to boost specific immune responses by activating NLRP3 inflammasome.

In one embodiment, the present compounds may be used in the prevention or treatment of cancer, wherein the cancer is selected from the group comprising solid tumors, skin cancer, wherein skin cancer is selected from the group comprising superficial basal cell carcinoma, genital warts and actinic keratosis, acute myeloid leukemia, adrenocortical carcinoma, Kaposi sarcoma, lymphoma, anal cancer, appendix cancer, teratoid/rhabdoid tumor, basal cell carcinoma, bile duct cancer, bladder cancer, bone cancer, brain cancer, breast cancer, bronchial tumor, carcinoid tumor, cardiac tumor, cervical cancer, chordoma, chronic lymphocytic leukemia, chronic myeloproliferative neoplasm, colon cancer, colorectal cancer, craniopharyngioma, bile duct cancer, endometrial cancer, ependymoma, esophageal cancer, esthesioneuroblastoma, Ewing sarcoma, eye cancer, fallopian tube cancer, gallbladder cancer, gastrointestinal carcinoid tumor, gastrointestinal stromal tumor, germ cell tumor, hairy cell leukemia, head and neck cancer, heart cancer, liver cancer, hypopharngeal cancer, pancreatic cancer, kidney cancer, laryngeal cancer, chronic myelogenous leukemia, lip and oral cavity cancer, lung cancer, melanoma, Merkel cell carcinoma, mesothelioma, mouth cancer, oral cancer, osteosarcoma, ovarian cancer, penile cancer, pharyngeal cancer, prostate cancer, rectal cancer, salivary gland cancer, skin cancer, small intestine cancer, soft tissue sarcoma, testicular cancer, throat cancer, thyroid cancer, urethral cancer, uterine cancer, vaginal cancer, and vulvar cancer.

In one embodiment, the present compounds may be used in the prevention or treatment of disease, wherein the compound activates the NLRP3 inflammasome and is preferably administered to a subject having a disease in which activation of the NLRP3 inflammasome contributes to the treatment of the pathology and/or symptoms of the disease and/or stops or decreases progression of the disease.

In one embodiment of the invention, a subject to be treated is a human or an animal, preferably a human.

In one embodiment of the invention, the present compounds are used in the activation of NLRP3 inflammasome and TLR 7 and/or TLR 8 is not activated.

In one embodiment of the invention, the present compounds are administered in an amount of 0.0005 mg kg⁻¹ to 1000 mg kg⁻¹, 0.005 mg kg⁻¹ to 500 mg kg⁻¹, 0.05 mg kg⁻¹ to 100 mg kg⁻¹, 0.5 mg kg⁻¹ to 100 mg kg⁻¹, 5 mg kg⁻¹ to 75 mg kg⁻¹, preferably about 50 mg kg⁻¹ in a mouse or a human.

In one embodiment of the invention, the present compounds are administered topically, orally or systemically. In one embodiment of the invention, the present compounds are administered topically. In one embodiment of the invention, the present compounds are administered systemically.

According to the one aspect, the present invention relates to a pharmaceutical composition comprising a compound of the present invention and one or more pharmaceutically acceptable excipients.

In one embodiment of the invention, the pharmaceutical composition can be in any physical form, such as a solid, semi-solid, plaster, solution, suspension, lotion, cream, foam, gel, paste, emulsion, or a combination thereof. In one embodiment of the invention, the pharmaceutical composition is in form of a tablet, spray, ointment or gel, preferably an ointment.

In one embodiment of the present invention, the pharmaceutical composition comprises the present compound in a concentration ranging from about 0.0001 ppm to 100,000 ppm. In another embodiment of the composition, the present compound is in a concentration ranging from about 0.001 ppm to 10,000 ppm. In another embodiment of the composition, the present compound is in a concentration ranging from about 0.01 ppm to 1,000 ppm. In another embodiment of the composition, the present compound is in a concentration ranging from about 0.1 ppm to 500 ppm. In another embodiment of the composition, the present compound is in a concentration ranging from about 1 ppm to 500 ppm. In another embodiment of the composition, the present compound is in a concentration ranging from about 10 ppm to 500 ppm. In another embodiment of the composition, the present compound is in a concentration ranging from about 1 ppm to 400 ppm.

According to the one aspect, the present invention relates to the use of the compounds of the present invention for boosting immune responses or breaking immune evasion in cancer diseases in a subject.

According to the one aspect, the present invention relates to a method for modulating NLRP3 activity, the method comprising contacting NLRP3 with a compound of the present invention.

In one embodiment of the present invention, the method for modulating NLRP3 comprises increasing the activity of NLRP3.

In one embodiment of the present invention, the method for modulating NLRP3 comprises that the increase of activity of NLRP3 inflammasome can be measured by the increase of the released amount of IL-1β of 0.25x10⁶ BMDCs, upon administering the compound in a concentration of 50µM of the compound and incubation of after 22 h, wherein the increase is at least 0.1 ng ml⁻¹, 0.5 ng ml⁻¹, 0.75 ng ml⁻¹, 1 ng ml⁻¹ 2 ng ml⁻¹, 10 ng ml⁻¹, 25 ng ml⁻¹, 50 ng ml⁻¹ and more preferably 4 ng ml⁻¹ or more, and still more preferably 6 ng ml⁻¹.

In one embodiment of the present invention, the method for modulating NLRP3 is carried out *in vitro* or *in vivo.*

According to the one aspect, the present invention relates to a method of treating cancer, comprising administering to a subject in need of such treatment an effective amount of a compound of the present invention, or a pharmaceutical composition of the present invention.

In one embodiment of the present invention, the method of treating cancer comprises that the compound is administered in combination with one or more additional cancer therapies.

In one embodiment of the present invention, the method of treating cancer comprises the one or more additional cancer therapies, which comprises surgery, radiotherapy, chemotherapy, toxin therapy, immunotherapy, cryotherapy or gene therapy, or a combination thereof.

In one embodiment of the present invention, the method of treating cancer comprises chemotherapy, which comprises administering one or more additional chemotherapeutic agents.

In one embodiment of the present invention, the method of treating cancer comprises chemotherapy, which comprises administering one or more additional chemotherapeutic agents, signal transduction inhibitors (kinase inhibitors) and immunomodulators (checkpoint inhibitors and STING agonists).

In one embodiment of the present invention, the method of treating cancer comprises chemotherapy, which comprises administering one or more additional chemotherapeutic agents, wherein the one or more additional chemotherapeutic agents is selected from an alkylating agent (e.g., cisplatin, carboplatin, mechlorethamine, cyclophosphamide, chlorambucil, ifosfamide and/or oxaliplatin); an anti-metabolite (e.g.,azathioprine and/or mercaptopurine); a terpenoid (e.g., a vinca alkaloid and/or a taxane; e.g., Vincristine, Vinblastine, Vinorelbine and/or Vindesine Taxol, Pacllitaxel and/or Docetaxel); a topoisomerase (e.g., a type I topoisomerase and/or a type 2 topoisomerase; e.g., camptothecins, such as irinotecan and/or topotecan;. amsacrine, etoposide, etoposide phosphate and/or teniposide); a cytotoxic antibiotic (e.g., actinomycin, anthracyclines, doxorubicin, daunorubicin, valrubicin, idarubicin, epirubicin, bleomycin, plicamycin and/or mitomycin); a hormone (e.g., a lutenizing hormone releasing hormone agonist; e.g., leuprolidine, goserelin, triptorelin, histrelin, bicalutamide, flutamide and/or nilutamide); an antibody (e.g., Abciximab, Adalimumab, Alemtuzumab, Atlizumab, Basiliximab, Belimumab, Bevacizumab, Bretuximab vedotin, Canakinumab, Cetuximab, Ceertolizumab pegol, Daclizumab, Denosumab, Eculizumab, Efalizumab, Gemtuzumab, Golimumab, Golimumab, Ibritumomab tiuxetan, Infliximab, Ipilimumab, Murom onab-CD3, Natalizumab, Ofatumumab, Omalizumab, Palivizumab, Panitumuab, Ranibizumab, Rituximab, Tocilizumab, Tositumomab and/or Trastuzumab); an anti- angiogenic agent; a cytokine; a thrombotic agent; a growth inhibitory agent; an antihelminthic agent; and an immune checkpoint inhibitor that targets an immune checkpoint receptor selected from the group consisting of CTLA-4, PD-1, PD-L1, PD-1 - PD-L1, PD- 1 - PD-L2, T cell immunoglobulin and mucin 3 (TFM3 or HAVCR2), Galectin 9 - TIM3, Phosphatidylserine - TIM3, lymphocyte activation gene 3 protein (LAG3), MHC class II, - LAG3, 4- 1BB-4- 1BB ligand, OX40-OX40 ligand, GITR, GITR ligand - GITR, CD27, CD70-CD27, TNFRSF25, TNFRSF25-TL1 A, CD40L, CD40-CD40 ligand, HVEM- LIGHT-LTA, HVEM, HVEM - BTLA, HVEM - CD 160, HVEM - LIGHT, HVEM- BTLA-CD160, CD80, CD80 - PD-L1, PD-L2 - CD80, CD244, CD48 - CD244, CD244, ICOS, ICOS-ICOS ligand, B7-H3, B7-H4, VISTA, TMIGD2, HHLA2-TMIGD2, Butyrophilins, including BTNL2, Siglec family, TIGIT and PVR family members, K Rs, ILTs and LIRs, NKG2D and NKG2A, MICA and MICB, CD244, CD28, CD86 - CD28, CD86 - CTLA, CD80 - CD28, Phosphatidylserine, TΓM3, Phosphatidylserine - TΓM3, SIRPA-CD47, VEGF, Neuropilin, CD160, CD30, and CD155 (e.g., CTLA-4 or PD-1 or PD-L1) and other immunomodulatory agents, such as interleukin-2 (IL-2), indoleamine, 2,3-dioxygenase (IDO), IL- 10, transforming growth factor-β (TGFp), CD39, CD73 Adenosine-CD39-CD73, and CXCR4-CXCL12.

Additional chemotherapeutic agents might be immune checkpoint inhibitors.

Preferred additional chemotherapeutic agents and/or immune checkpoint inhibitors are selected from the group consisting of Urelumab, PF-05082566, MEDI6469, TRX518, Varlilumab, CP-870893, Pembrolizumab (PD-1), Nivolumab (PD1), Atezolizumab (PD-L1), PDR001 (PD-1), BMS-986016, MGA271, Lirlumab, IPH2201, Emactuzumab, INCB024360, Galunisertib, Ulocuplumab, BKT140, Bavituximab, CC-90002, Bevacizumab, 1VINRP1685A, and MGA271.

According to the one aspect, the present invention relates to a method for analyzing the activity of NLRP3 activation.

NLRP3 inflammasome activation in different cell types like for examples primary murine bone marrow-derived dendritic cells (BMDCs) and macrophages (BMDMs), THP-1 cells, human peripheral blood mononuclear cells (PBMCs). Cells are typically primed with 50 ng ml⁻¹ LPS for 3 hours before inflammasome activation. Markers of inflammasome activation are investigated by measuring release of the cytosolic enzyme lactate dehydrogenase (LDH) from the cells as a readout for lytic cell death and pyroptosis, detecting release of proinflammatory cytokines such as IL-1β into the cell supernatants by enzyme-linked immunosorbent assay (ELISA), looking at caspase 1 activation and cleavage of Gasdermin D and pro IL-1β via Immunoblotting and following ASC speck formation via immunofluorescence imaging.

According to one embodiment, the present invention relates to the method for analyzing the NLRP3 activation, which comprises the steps of: (i) priming cells with LPS, (ii) contacting the cells with present compounds (iii) detecting the release of IL-1β into the cell supernatant by enzyme-linked immunosorbent assay (ELISA) and analyzing the cells by immunoblotting and immunofluorescence imaging.

According to the one aspect, the present invention relates to the use of the present compounds for analyzing the activity of NLRP3 activation in vitro.

### Examples

### 1. Biological Assays

The present compounds are useful as modulators of NLRP3. In a high-throughput screen using a screening method developed by us, new, specific NLRP3 activators out of about 50.000 small molecule compounds have been identified, which differ in their structure and several properties of NLRP3 activation from all previously known and protected NLRP3 activators and are novel and unique in them.

### 1.1 In vitro preliminary data and properties of the activators

All NLRP3 activators presented herein are sufficiently soluble and cell-permeable inflammasome activators: The activators showed sufficient solubility in solvents such as DMSO and have the ability to pass the cell membrane, as only then the detection by the high-throughput screening is possible. Moreover, the present compounds showed good activity *in vitro.* This was tested in cell culture in several different *in vitro* assays classically used to investigate inflammasome activation. These include measuring inflammasome-dependent lytic cell death (pyroptosis) by detecting release of the cytosolic enzyme lactate dehydrogenase (LDH) using a commercial colorimetric assay and release of proinflammatory cytokines like IL-1β by enzyme-linked immunosorbent assay (ELISA), analyzing cleavage and activation of caspase-1 and IL-1β by immunoblotting and immunofluorescence imaging of ASC speck formation. Typical compound concentrations tested were 50 µM, and 25 µM. The present compounds have shown strong activity in inducing NLRP3 inflammasome activation (see **Figure 14****).** Lytic cell death was determined via LDH release. Assay results for compounds are illustrated in Tables 3 and 4 below, wherein the concertation of the compounds tested had been 50 µM, and the concentration of the known activators has been tested with 5 µM of nigericin, 100 µM of each R837 and CL097.

**Table 3**

| **Compound** | | | **LDH release (% of max.)** | | | | **average LDH release (% of max.)** |
|---|---|---|---|---|---|---|---|
| **DMSO** | 8,053 | 7,414 | 8,022 | 8,032 | 10,176 | 6,889 | **8,098** |
| **Nigericin** | 61,933 | 78,845 | 69,982 | 78,413 | 75,764 | 74,003 | **73,157** |
| EN21 | 36,356 | 47,704 | 43,019 | 50,628 | 60,994 | 63,141 | 50,307 |
| **EN22** | 59,410 | 74,624 | 67,018 | 74,225 | 71,324 | 71,181 | **69,630** |
| **R837** | 12,734 | 15,684 | 15,495 | 20,399 | 34,109 | 57,377 | **25,966** |
| **CL097** | 56,511 | 69,145 | 62,611 | 76,051 | 68,018 | 76,242 | **68,096** |
| **L01.110** | 34,845 | 34,294 | 31,850 | | | | **33,663** |
| **L01.041** | 24,371 | 24,119 | 23,567 | | | | **24,019** |
| **L01.004** | 15,163 | 16,970 | 18,507 | | | | **16,880** |
| **L01.007** | 17,823 | 23,721 | 19,371 | | | | **20,305** |
| **L01.104** | 17,190 | 17,671 | 19,727 | | | | **18,196** |
| **L01.116** | 17,956 | 18,328 | 17,892 | | | | **18,058** |
| **L01.113** | 37,377 | 36,390 | 34,335 | | | | **36,034** |
| **L01.101** | 19,384 | 19,564 | 20,661 | | | | **19,870** |
| **L01.092** | 16,396 | 14,952 | 13,605 | | | | **14,985** |
| **L01.124** | 14,119 | 13,508 | 14,589 | | | | **14,072** |
| **L01.016** | 11,582 | 12,903 | 13,299 | | | | **12,595** |
| **L01.028** | 12,612 | 15,129 | 15,770 | | | | **14,503** |
| **L01.047** | 12,065 | 12,727 | 11,451 | | | | **12,081** |
| **L01.026** | 9,228 | 9,969 | 13,232 | | | | **10,810** |
| **L01.130** | 9,387 | 8,722 | 7,450 | | | | **8,520** |
| **L01.049** | 12,648 | 10,774 | 11,897 | | | | **11,773** |
| **L01.035** | 8,461 | 8,303 | 7,473 | | | | **8,079** |

**Table 4**

| **Compound** | | | **IL-1 β (ng/ml)** | | | | **average IL-1 β (ng/ml)** |
|---|---|---|---|---|---|---|---|
| **DMSO** | 0,042 | 0,054 | 0,055 | 0,067 | 0,057 | 0,056 | 0,055 |
| **Nigericin** | 6,834 | 6,400 | 7,872 | 6,518 | 7,890 | 7,496 | 7,168 |
| **EN21** | 8,582 | 7,428 | 8,023 | 6,613 | 8,352 | 7,696 | 7,782 |
| **EN22** | 8,679 | 9,014 | 9,589 | 8,363 | 10,000 | 9,819 | 9,244 |
| **R837** | 5,472 | 4,151 | 1,552 | 1,212 | 0,958 | 0,911 | 2,376 |
| **CL097** | 7,355 | 8,020 | 8,660 | 8,522 | 9,540 | 9,324 | 8,570 |
| **L01.110** | 2,505 | 2,943 | 2,423 | | | | 2,624 |
| **L01.041** | 1,508 | 1,847 | 1,769 | | | | 1,708 |
| **L01.004** | 1,192 | 1,485 | 1,567 | | | | 1,415 |
| **L01.007** | 0,859 | 1,572 | 1,101 | | | | 1,177 |
| **L01.104** | 1,103 | 1,142 | 1,246 | | | | 1,163 |
| **L01.116** | 1,107 | 1,192 | 1,163 | | | | 1,154 |
| **L01.113** | 1,145 | 1,165 | 1,064 | | | | 1,125 |
| **L01.101** | 1,160 | 1,071 | 1,041 | | | | 1,091 |
| **L01.092** | 1,253 | 1,060 | 0,927 | | | | 1,080 |
| **L01.124** | 0,928 | 0,902 | 1,024 | | | | 0,951 |
| **L01.016** | 0,755 | 0,723 | 0,857 | | | | 0,778 |
| **L01.028** | 0,686 | 0,757 | 0,705 | | | | 0,716 |
| **L01.047** | 0,505 | 0,513 | 0,512 | | | | 0,510 |
| **L01.026** | 0,452 | 0,473 | 0,562 | | | | 0,495 |
| **L01.130** | 0,404 | 0,317 | 0,215 | | | | 0,312 |
| **L01.049** | 0,330 | 0,251 | 0,319 | | | | 0,300 |
| **L01.035** | 0,321 | 0,189 | 0,181 | | | | 0,230 |

### 1.2 BMDC isolation and differentiation

Mouse bone marrow-derived dendritic cells (BMDCs) were isolated from femurs and tibias previously described (Gross, Olaf, 2012, "Measuring the inflammasome", Methods in molecular biology, Clifton, N.J., 844, pp. 199-222.). In brief, tibias and femurs were carefully removed and put to sterile 70% ethanol for 1 minute. Using a 22-G needle attached to a 20ml-syringe filled with sterile flushing medium, the bone marrow was pressed through the mesh of a 100 µM cell strainer. The cells were spun down by centrifugation at 400x g, 4°C for 5 minutes and red blood cells lysed by resuspension and incubation for 5 minutes at room temperature in RBC lysis buffer. 9 ml of flushing medium were added and cells were again spun down by centrifugation at 400x g, 4°C for 5 minutes. Cells were then resuspended in BMDC medium (RPMI, 10% fetal bovine serum, 1% penicillin-streptomycin100 U/ml, 20 ng/ml recombinant murine GM-CSF), counted and plated at a density of 0.5x 10⁶ cells ml⁻¹ in10 ml medium and incubated at 37°C, 5% CO₂ in a humidified incubator. After 2 days, 10 ml of fresh medium were added to the cells and after 4 days, the medium was fully exchanged to 20 ml of fresh medium. The cells were allowed to differentiate for 6 to 8 days before usage.

### 1.3 Harvesting of BMDCs and BMDMs

BMDCs after 6 to 8 days of differentiation were harvested by transferring floating cells to 50 ml Falcon tubes and dislodging adherent cells with HBSS containing 5 mM EDTA.

### 1.4 High-throughput screening for inflammasome activators

To screen for new small molecule inflammasome activators, a 2-step semi-automated screening strategy was devised and performed. Small molecules from a Novartis public compound library were tested and access to high-throughput screening equipment was provided via the Novartis Facilitated Access to Screening Technologies (FAST) Lab.

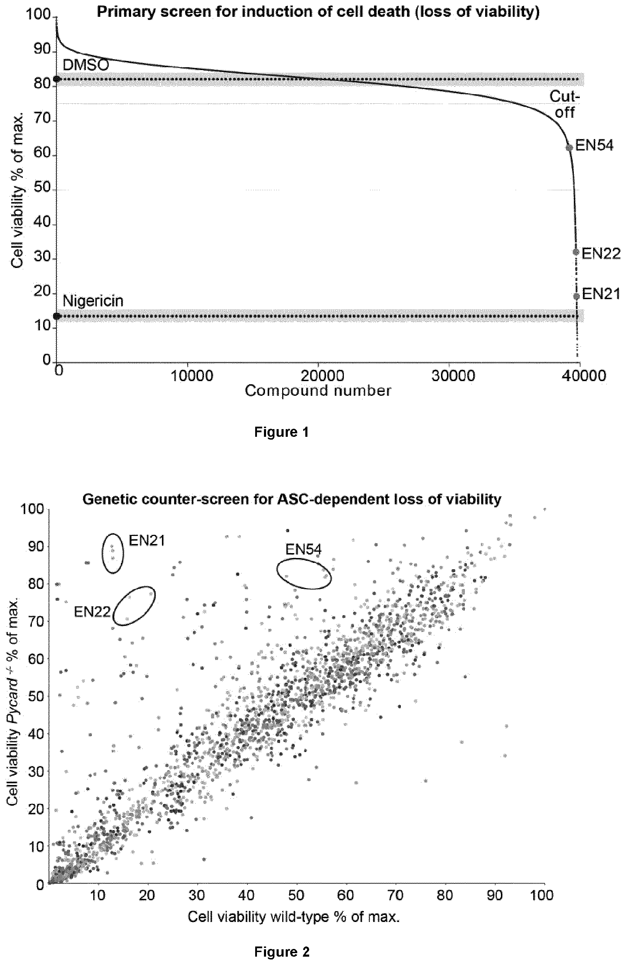

For primary screening (see **Figure 1**), 200 nl of the small molecule stock solutions (10 mM or 5 mM in DMSO) were pre-dispensed to white 384 well flat-bottom microplates with an Echo^{®} 555 Liquid Handler (Labcyte Inc.). The plates were sealed with a PlateLoc Thermal Microplate Sealer (Agilent Technologies Inc.) and stored at 4- 8°C until further use. Plates were brought to ambient temperature before usage.

BMDCs from wild-type mice after 7 days of differentiation were harvested as described above. Depending on the volume of cell suspension needed for the experiment, the cells were resuspended in an appropriate volume of BMDC and counted with a Countess^{™} II FL Automated Cell Counter (Thermo Fisher Scientific). The concentration was adjusted to 0.25x 10⁶ cells ml⁻¹ and the cells were primed with 50 ng ml⁻¹ *E. coli* K12 ultra-pure LPS. Immediately after addition of LPS, 40 µl per well cell suspension were plated to the microplates already containing the small molecules to be tested using a Multidrop^{™} Combi Reagent Dispenser (Thermo Fisher Scientific) resulting in a final small molecule concentration of 50 µM or 25 µM. The cell suspension was mixed on a regular basis to ensure a consistent plating density. 8 wells per plate were filled with nigericin at a final concentration of 5 µM, DMSO at a final concentration of 0.5% or imiquimod and CL097 as references at a final concentration of 100 µM. It was then incubated at 37°C, 5% CO₂ in a humidified incubator for about 20 hours. After the incubation, the plates were removed from the incubator and 10 µl per well CellTiter-Glo^{®} Reagent (Promega Corporation) were added with the Multidrop^{™} Combi Reagent Dispenser. After at least 15 minutes of incubation at room temperature, the luminescence was determined with an EnVision 2105 Multimode Plate Reader (PerkinElmer Inc.) equipped with an EnVision stacker for semi-automated plate loading. Luminescence signals were normalized to nigericin as a positive control for inflammasome activation and DMSO as a negative control. Small molecules were considered active and chosen for subsequent counter screening when they induced a reduction in the luminescence signal that was at least 50% (25% in some cases) of the reduction induced by 5 µM nigericin (see **Figure 1**).

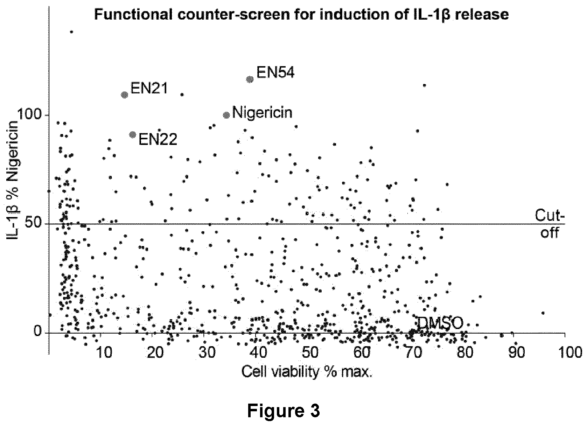

For counter screening (see **Figures 2** **and** **3**), two independent strategies were pursued. To test for inflammasome dependency of cell viability reduction, BMDCs from either wild-type or *Pycard*^{*-*/*-*} mice (Mariathasan, S., Newton, K., Monack, D. et al. Differential activation of the inflammasome by caspase-1 adaptors ASC and Ipaf. Nature 430, 213-218 (2004). https://doi.org/10.1038/nature02664) were used and the same steps were performed as described above for primary screening, whereas the incubation time was reduced to about 16 hours. Three independent replicates of each plate were prepared and measured and the small molecules were either tested at a final concentration of 50 µM or at a dose response of 4 or 8 concentrations per compound going down with a dilution factor of 1/3.16 starting from 50 µM. Small molecules, that induced a reduction in the luminescence signal in the wild-type but not in the *Pycarct*^{*-*/*-*} BMDCs were considered as hits for further evaluation (**see** **Figures2** **and** **4**).

To functionally counter-screen (see **Figure 3**) for induction of IL-1β release, BMDCs from wild-type mice were harvested, counted and the concentration adjusted to 0.25x 10⁶ cells ml⁻¹. The cells were primed with 50 ng ml⁻¹ *E. coli* K12 ultra-pure LPS directly before plating 40 µl per well cell suspension to white 384 well flat-bottom microplates and it was incubated for 3 hours at 37°C, 5% CO₂ in a humidified incubator. 200 nl or 400 nl of the small molecule stock solutions from the libraries that had before been transferred to mother plates for the Echo^{®} 555 Liquid Handler were then added to the microplates with the primed BMDCs to a final compound concentration of 50 µM. After 20 hours of incubation at 37°C, 5% CO₂, IL-β release from the cells was then determined by using HTRF^{®} technology developed by Cisbio from cell-free supernatants. HTRF (Homogeneous Time Resolved Fluorescence) is the most frequently used generic assay technology to measure analytes in a homogenous format, which is the ideal platform used for drug target studies in high-throughput screening. This technology combines fluorescence resonance energy transfer technology (FRET) with time-resolved measurement. The measurements were carried out as described in the manufacturer's manual.

### 1.5 Inflammasome stimulation

For canonical inflammasome activation BMDCs after 7- 9 days of differentiation were harvested and counted with a Neubauer Chamber (Hecht Assistant) and the concentration was set to 1 Mio cells ml⁻¹. 100 µl cell suspension per well were seeded in 96-well plates. The cells were primed with 50 ng ml⁻¹ *E. coli* K12 ultra-pure LPS for 3 hours and subsequently treated with canonical inflammasome activators or EN21, EN22 for 3 hours if not indicated differently (incubation time Figure 12: 4h, Figure 13 and 14: 22 h). Concentrations of the stimuli were as follows if not indicated differently: 5 µM nigericin, 100 µM imiquimod (also known as R837), 100 µM CL097, 100 µM R848. If an inhibitor was used before stimuli treatment, the inhibitor was added after 2.5 hours of priming and it was incubated for 30 minutes before inflammasome activation. Inhibitors were used as follows: 45 mM KCl, 30 µM ebselen (2-Phenyl-1,2-benzoselenazol-3-one), 5 µM cytochalasin D (see **Figures 7, 8****,** **11, 12** **and** **16**).

All inflammasome stimulations were performed in three technical replicates for each condition. Lytic cell death was determined via LDH release (see **Figures 7, 8****, and** **13**), cytokines quantified in cell-free supernatants via ELISA and cell lysates and supernatants analyzed via immunoblotting (see **Figures 5A and 5B**) as previously described in detail (Gross, Olaf, 2012 "Measuring the inflammasome", Methods in molecular biology, Clifton, N.J., 844, pp. 199-222. Schneider, Katharina S.; Thomas, Christina J.; Groß, Olaf, 2013, "Inflammasome activation and inhibition in primary murine bone marrow-derived cells, and assays for IL-1α, IL-1β, and caspase-1", Methods in molecular biology, Clifton, N.J., 1040, pp. 117-135).

### 1.6 Toll-like receptor stimulation

To test for secretion of inflammasome independent cytokines (TNF and IL-6), BMDCs after 7-9 days of stimulation were left unprimed and then stimulated as described in Example 1.4. Cell-free supernatants were analyzed via ELISA (see **Figure 9** **and** **18**).

### 1.7 Determination of cell viability

### 1.7.1 LDH release

To assess lytic cell death, release of the stable cytosolic enzyme lactate dehydrogenase (LDH) was determined using the CytoTox 96^{®} Non-Radioactive Cytotoxicity kit (Promega Corporation) according to the manufacturer's instructions. The volumes of cell-free supernatants and reagent used were reduced to 35µl. Background signal that was determined from medium without cells was subtracted from all values and all results were depicted as % of cells that had been lysed with 0.8% Triton^{®} X-100 45 minutes before performing the assay. Absorbance at 490 nm was determined using a Tecan Infinite M200 plate reader (Tecan Life Sciences) (see **Figures 7, 8****,** **13****,** **15** **and** **17**).

### 1.7.2 Cellular ATP

Cellular ATP levels as were determined with the CellTiter-Glo^{®} Luminescent Cell Viability kit (Promega Corporation) according to the protocol provided by the manufacturer except that one fourth of the recommended amount of reagent was used. Luminescence intensities were normalized as % of the highest signals obtained, usually in the untreated control conditions.

### 1.8 Cytokine quantification

### ELISA

Cytokines were quantified in cell-free supernatants by using enzyme-linked immunosorbent assay (ELISA) kits for murine IL-1β, TNF and IL-6 (Thermo Fisher Scientific). The volumes of cell-free supernatants, antibodies and reagents used were reduced to 50 µl. Each condition was measured as 3 independent technical replicates and data was depicted as mean ± SD. Absorbance at 450 nm and 570 nm was determined using a Tecan Infinite M200 plate reader (Tecan Life Sciences). Absolute values were interpolated from a standard curve that was generated with the Magellan^{™} Data Analysis Software. Depending on the expected cytokine quantity cell-free supernatants were diluted so that the detection values were within the linear range of the standard curve (see **Figures 7- 9****,** **11****,** **12** **and** **14-18**).

The IL-1β release from BMDCs of wild-type mice treated with 50µM of the present compounds, incubation 22 hours, in comparison to the solvent only (DMSO), and the known activators nigericin (5µM), R837 and CL097 (each 100µM) can be derived from Figure 14. The cells were primed for 3 hours with 50 ng ml⁻¹ LPS. Similarly, the IL-1β release from BMDCs of wild-type mice was measured in diagrams of Figures 7, 8, 11, 12, and 15 to 17. However, the incubation time with 50µM of the present compounds was 3 hours (Figures 7, 8, 11, 15 to 17) or 4 hours (Figure 12).

### 1.9 Immunoblotting

Cell lysates for immunoblotting were generated by lysing the cells with 1x reducing SDS-PAGE loading buffer followed by 1 freeze-thaw cycle. Cell-free supernatants were diluted with 3x reducing SDS-PAGE loading buffer. Samples from 3 independent technical replicates were pooled and proteins were separated via SDS-PAGE (sodium dodecyl sulfate polyacrylamide gel electrophoresis) using 12% polyacrylamide gels and applying 80-120 V for 1-2.5 hours. Afterwards, proteins were transferred to nitrocellulose membranes by application of 100 V for 100 minutes. Membranes were rinsed with PBS-tween and success of protein transfer checked via Ponceau S staining. The membranes were then washed with PBS-tween again until Ponceau was completely removed and blocked with 2% skim milk powder in PBS-tween for 1 hour at room temperature. Membranes were rinsed with PBS-tween again and primary antibodies applied overnight at 4°C (diluted in PBS-tween, 2% skim milk powder, 0.05% sodium azide). Before adding HRP-conjugated secondary antibodies (diluted 1:5,000 in PBS-tween, 2% skim milk powder) for 1 hour at room temperature, membranes were washed with PBS-tween for 2 hours, replacing with fresh PBS-tween every 15 minutes. Enhanced chemiluminescence substrate was applied before imaging with a cooled charge-coupled device. Depending on the signal intensity (16 bit, 64K grayscale) it was exposed for 2-15 minutes.

### 1.10 Immunofluorescence imaging for ASC specks

BMDCs from either wild-type or ASC-citrine mice expressing a ASC fluorescent protein were seeded in chambered coverslips for cell imaging (µ-Slide, Ibidi) and primed with 50 ng ml⁻¹ LPS for 3 hours. The cells were then incubated with the inflammasome activator of interest at the respective conditions. After the treatment, the cells were washed with PBS, fixed with 4% paraformaldehyde for 10 minutes and permeabilized with 0.1% (v/v) TritonTM X-100) for 5 minutes. The cells were then stained with polyclonal anti-ASC antibody derived from rabbit (Adipogen) and FITC anti-mouse CD45 antibody (Biolegend) overnight at 4°C. The next day, the cells were washed with blocking buffer and stained with a fluorescence labelled secondary antibody specific for rabbit for 30 minutes at room temperature, washed and subsequently resuspended in Vectashield^{®} Antifade Mounting Medium with DAPI (H-1200) (Vector Laboratories) diluted 1:10 in blocking buffer for at least 10 minutes. Confocal microscopy of the immunostained cells was then performed. Immunostaining for ASC is dispensable if BMDCs from ASC-citrine mice are used.

### 1.11 Metabolic Analysis (extracellular flux analysis)

Oxygen consumption rate (OCR) and extracellular acidification rate (ECAR) were measured by extracellular flux analysis with a Seahorse XFe96 Analyzer. One day before the experiment, BMDCs were plated to XFe cell culture microplates at a density of 80,000 cells per well and a XFe sensor cartridge was rehydrated using XF Calibrant and put to 37°C in a CO₂-free incubator. On the day of the experiment, the cells were first primed with 50 ng ml⁻¹ *E. coli* K12 ultra-pure LPS for 2-3 hours. The medium was then exchanged to bicarbonate- and phenol red-free seahorse base medium supplemented with 2 mM L-glutamine, 10 mM glucose and 5 mM HEPES, pH 7.4 and the cells were incubated at 37°C in a CO₂-free incubator for 1 hour. In the meantime, 10x dilutions of compounds and inhibitors to be tested were prepared in seahorse base medium and loaded to the injection ports of the rehydrated XFe sensor cartridge. Small molecules were used as indicated and respiratory chain inhibitors (piericidin, antimycin A) and mitochondrial uncouplers (FCCP) were used as follows after an initial titration: 10 µM piericidin, 2 µM antimycin A, 1 µM FCCP.

### 1.12 Materials

- 1x PBS (GibcoTM, Thermo Fisher Scientific)
- 3x reducing SDS-PAGE loading buffer: 37.5ml 1M Tris-HCl pH 7.0 (=187.5 mM), 12g SDS (= 6% w/v), 60mg phenol red (= 0.03% w/v) (Sigma Aldrich), 69g glycerol (=30% w/v); adjust pH with 0.1N HCl to pH 6.8, 66,7 mM DTT (Carl Roth)
- 4% Paraformaldehyde solution (Sigma Aldrich)
- Blocking buffer: PBS (GibcoTM, Thermo Fisher Scientific), 5% FCS (GibcoTM, Thermo Fisher Scientific), 0.1% TritonTM X-100 (Sigma Aldrich)
- BMDC medium: RPMI (GibcoTM, Thermo Fisher Scientific), 10% FCS (GibcoTM, Thermo Fisher Scientific), 100 units ml-1 penicillin/ streptomycin (GibcoTM, Thermo Fisher Scientific), 50 ng ml-1 recombinant murine GM-CSF (Immunotools)
- CellTiter-Glo^{®} Luminescent Cell Viability Assay (Promega Corporation)
- CytoTox 96^{®} Non-Radioactive Cytotoxicity Assay (Promega Corporation)
- *E. coli* K12 ultra-pure LPS (Invivogen)
- IL-1β, TNF and IL-6 Mouse Uncoated ELISA Kit (Thermo Fisher Scientific)
- PBS-tween: 4g KH2PO4 (Potassium phosphate monobasic, Sigma Aldrich), 4g KCl (Potassium chloride, Sigma Aldrich), 160g NaCl (>99.8 %, Carl Roth), 28.4g Na2HPO4 x 2 H2O pH 7.2 - 7.4 (di-Natriumhydrogenphosphat Dihydrat, ≥99.5 %, Carl Roth), 20 ml Tween-20 (Sigma Aldrich) in 20 I H2O
- Primary antibodies for immunoblotting and immunostaining:
   Anti-Asc, pAb (AL177) (Adipogen)
   Anti-Caspase-1 (p20) (mouse), mAb (Casper-1) (Adipogen)
   Mouse IL-1β/IL-1F2 Antibody (R&D)
- XFe flux analysis consumables and reagents (Agilent Technologies)
- Nigericin sodium salt (Sigma Aldrich)
- Imiquimod (R837) (Invivogen)
- CL097 (Invivogen)
- R848 (Invivogen)

### 1.12 Devices

- Countess^{™} II FL Automated Cell Counter (Thermo Fisher Scientific)
- Echo^{®} 555 Liquid Handler (Labcyte Inc.)
- EnVision 2105 Multimode Plate Reader (PerkinElmer Inc.)
- Humidified CO2 incubator (HeracellTM 150, Thermo Fisher Scientific)
- Intas Chemostar ECL Imager (Intas Science Imaging)
- Leica SP8 confocal microscope equipped with a 63×/1.40 and 40x/1.25 oil objectives (Leica Microsystems)
- Multidrop^{™} Combi Reagent Dispenser (Thermo Fisher Scientific)
- Neubauer chamber (Hecht Assistent)
- Seahorse XFe96 Flux Analyzer (Agilent Technologies)
- Tecan Infinite M200 plate reader (Tecan Life Sciences)

### 2. Synthesis

Synthesis was carried out following the scheme given below:

### 2.1 Preparation of compound EN21

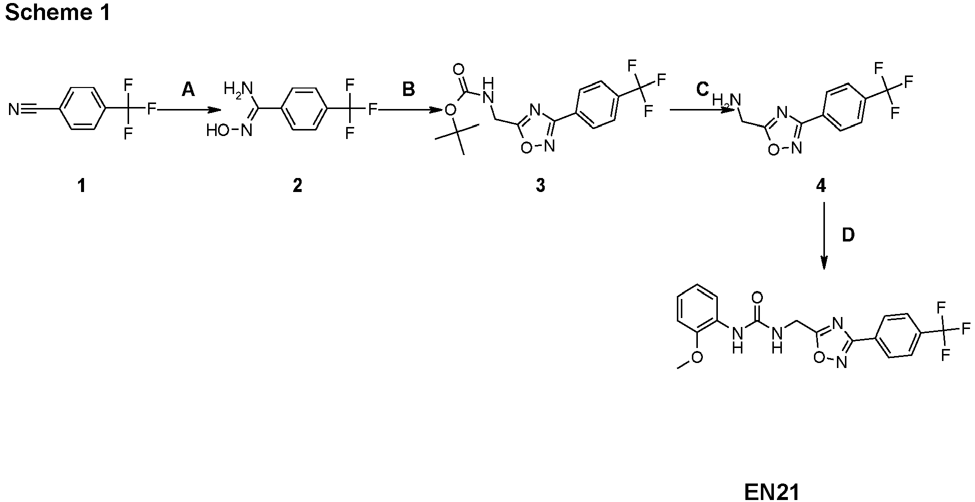

### Step A:

To a stirred suspension of 4-(trifluoromethyl)benzonitrile (1) (15 g, 87.7 mmol, 1 eq.) and; sodium hydrogencarbonate (8.8 g, 105.2 mmol, 1.2 eq.) in methanol (150 ml), hydroxylamine hydrochloride (7.3 g, 105.2 mmol, 1.2 eq.) was added portion wise and stirred overnight. After completion of the reaction (TLC data), the mixture was filtrated and the filtrate was carefully evaporated in vacuo at 35°C. The resulting residue was suspended in water (200 mL), stirred for 15 min and then filtrated to afford N'-hydroxy-4-(trifluoromethyl)benzimidamide (**2**) (16.2 g, 91 % yield) as white solid.

### Step B:

To a stirred solution of 2-((*tert*-butoxycarbonyl)amino)acetic acid (15.3 g, 87.3 mmol, 1.1 eq.) in dry 1,4-dioxane (180 mL), 1,1'-carbonyldiimidazole (CDI) (12.9 g, 79.4 mmol, 1 eq.) was added by portion at 60°C and the resulting mixture was stirred for 30 min at 60°C. N'-hydroxy-4- (trifluoromethyl)benzimidamide (**2**) (16.2 g, 79.4 mmol, 1 eq.) was added at once and the resulting mixture was stirred at 60°C until full consumption of 4-(trifluoromethyl)benzimidamide (**2**) was being achieved (TLC data, approximately 30 min for this scale). Then the reaction mixture was heated at 90°C for 12 h, cooled to r.t. and evaporated in vacuo. The resulting residue was diluted with 30 % aq. solution of potassium carbonate (200 mL) and ethyl acetate (200 mL), the water layer was separated and extracted with ethyl acetate (2×100 mL). The combined organic layers were dried over anhydrous sodium sulfate and evaporated in vacuo. The crude material was purified by column chromatography (chloroform : ethyl acetate, v/v= 10:1, rf = 0.8) to afford *tert*-butyl((3-(4-(trifluoromethyl)phenyl)-1,2,4-oxadiazol-5-yl)methyl)carbamae (3) (16.1 g, 59 % yield) as white solid.

### Step C, D :

To a stirred trifluoroacetic acid (10 mL), *tert*-butyl ((3-(4-(trifluoromethyl)phenyl)-1,2,4-oxadiazol-5-yl)methyl)carbamate (**3**) (2 g, 5.8 mmol) was added by portion at r.t. and the resulting mixture was stirred for 30 min. The solvent was removed in vacuo and the residue was diluted with dichloromethane and threated with excess of DIPEA (3.1 mL, 17.5 mmol, 3 eq.). The resulting mixture was stirred at r.t. for 5 min and then, 1-isocyanato-2-methoxybenzene (0.9 g, 5.8 mmol, 1 eq.) was added at once. The resulting mixture was stirred for additional 30 min. The resulting precipitate was filtrated and washed with dichloromethane (2×10 mL) to afford 1-(2- methoxyphenyl)-3-((3-(4-(trifluoromethyl)phenyl)-1,2,4-oxadiazol-5-yl)methyl)urea (**EN21**, also called **EN02-01**) (1.1 g, 48 % summary yield) as white solid.

EN21: 1H NMR (400 MHz, dmso) δ 3.86 (s, 3H), 4.72 (d, 2H), 6.83 (t, 1H), 6.91 (t, 1H), 6.99 (d, 1H), 7.67 (t, 1H), 7.94 (d, 2H), 8.02 (d, 1H), 8.22 (d, 2H), 8.28 (s, 1H).

### 2.2 Preparation of compound EN22

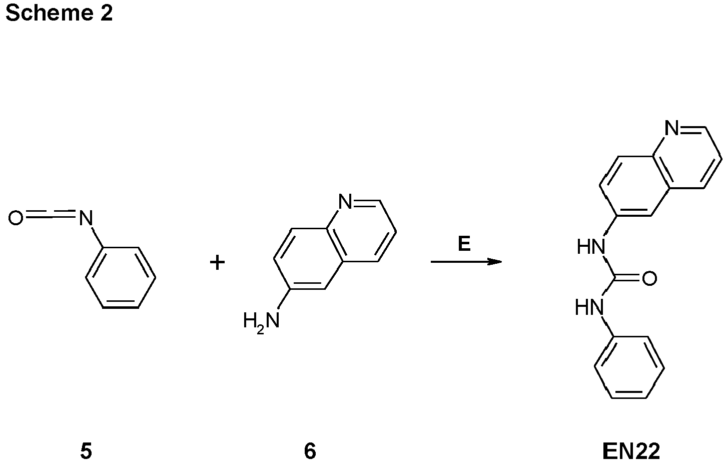

### Step E:

To a solution of amine **6** (3.29 g, 22.8 mmol) in dry dichloromethane (50 mL) was added isocyanate **5** (2.72 g, 22.8 mmol). The reaction mixture was stirred for 2 h at r.t. and the precipitated solid was collected, washed with dichloromethane and hexane, and dried in vacuum to afford 5.15 g of compound **EN22** (also called **EN02-02**) (85 % yield).

**EN22:** 1H NMR (400 MHz, dmso+ccl4) δ 6.99 (t, 1H), 7.30 (t, 2H), 7.45 (dd, 3.9 Hz, 1H), 7.50 (d, 2H), 7.70 (d, 1H), 7.94 (d, 1H), 8.18 (s, 1H), 8.25 (d, 1H), 8.74 (d, 1H), 8.79 (s, 1H), 9.03 (s, 1H).

### 2.3 Preparation of compound EN54

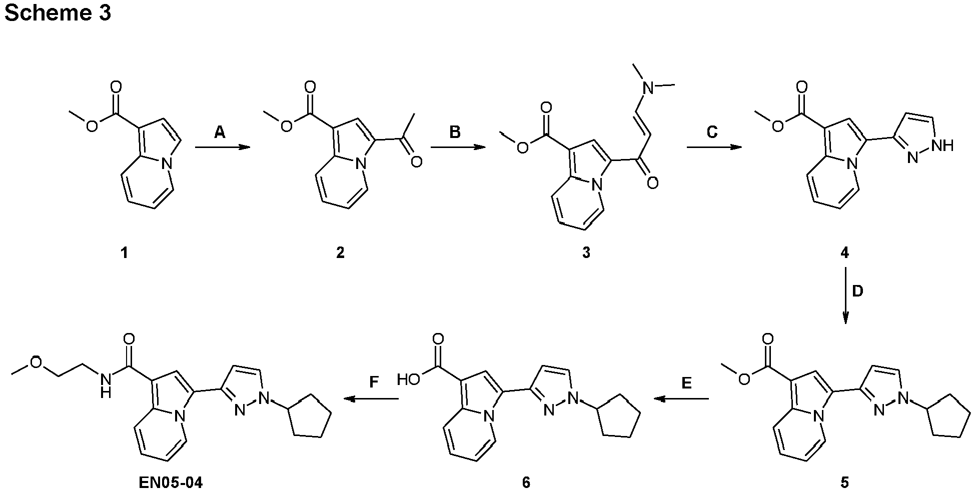

### Step A:

To a stirred solution of acetyl chloride (4.7 g, 60 mmol) in dichloromethane (60 ml) aluminum chloride (8.0 g, 60 mmol) was added. The mixture was cooled to 0 °C and methyl 1-indolizinecarboxylate (**1**) (7.0 g, 40 mmol) in dichloromethane (40 ml) was added dropwise. The resulting mixture was stirred overnight at room temperature, poured in ice water and diluted with HCl. The organic phase was separated, the water phase was extracted with dichloromethane twice, and the combined organic phase was washed with saturated aqueous sodium bicarbonate and dried over potassium sulphate. The solvent was removed under reduced pressure to give methyl 3-acetyl-1-indolizinecarboxylate **(2)** without further purification as yellow solid. Yield 7.2 g, 84%.

### Step B:

A mixture of methyl 3-acetyl-1-indolizinecarboxylate **(2)** (7.2 g, 33.6 mmol) and DMF-DMA (8.0 g, 67.2 mmol) in dimethylformamide (10 ml) was heated to 110 °C overnight. The solution was cooled to room temperature, and the formed solid product was collected by filtration and washed with hexane and dried to obtain compound **3.** Yield 7.0 g, 78%.

### Step C:

A mixture of methyl 3-[3-(dimethylamino)acryloyl]indolizine-1-carboxylate **(3)** (7.0 g, 26 mmol) and hydrazine hydrate (6.5 g, 130 mmol) in methanol (50 ml) was refluxed for 3 h. The solution was cooled to room temperature and diluted with water (100 ml). The solid product was collected by filtration and dried to obtain compound **4.** Yield 4.2 g, 67%.

### Step D:

To a stirred solution of methyl 3-(1H-pyrazol-3-yl)indolizine-1-carboxylate **(4)** (4.2g, 17.4 mmol) in DMF (40 ml) sodium hydride (0.85 g, 20.9 mmol as suspension in mineral oil) was added. The mixture was stirred for 30 min., cooled to 0 °C and bromocyclopenthane (3.9 g, 26.1 mmol) in DMF (10 ml) was added dropwise. The resulting mixture was stirred overnight at room temperature and poured in ice water. The solid product was collected by filtration and dried to obtain compound **5.** Yield 4.8 g, 89%.

### Step E:

To a stirred suspension of ethyl 3-(1-cyclopentyl-1H-pyrazol-3-yl)indolizine-1-carboxylate **(5)** (4.8 g, 15.5 mmol) in methanol (50 ml) potassium hydroxide (8.7 g, 155 mmol) in water (40 ml) was added and the resulting mixture was refluxed for 24 h. The clear solution was cooled to room temperature, diluted with water (50 ml) and diluted HCI was added. The solid product was collected by filtration and dried to obtain compound **6.** Yield 4.2 g, 67%.

### Step F:

To a stirred suspension of 3-(1-cyclopentyl-1H-pyrazol-3-yl)indolizine-1-carboxylic acid **(6)** (3.2 g, 10.8 mmol) in dichloromethane (40 ml) thionyl chloride (2.6 g, 21.7 mmol) was added and the resulting mixture was stirred at room temperature to obtain of the clear dark green solution (6-8 h). The solvent and the excess of thionyl chloride were removed under reduced pressure, and the residue was dissolved in dichloromethane (40 ml) and added dropwise to a stirred solution of 2-methoxyethylamine (1.2 g, 16.3 mmol) and DIPEA (2.8 g, 21.7 mmol) in dichloromethane (40 ml) at room temperature. The resulting mixture was stirred overnight, washed with diluted HCl, saturated aqueous sodium bicarbonate and dried over potassium sulphate. The solvent was removed under reduced pressure to give 3.1 g (82%) of the title product. The analytical sample of compound **EN54** (also called **EN05-04**) was obtained by column chromatography on silica gel using ethyl acetate as an eluent.

**EN54:** 1H NMR (400 MHz, dmso) δ 1,68 (m, 2H), 1,83 (m, 2H), 2,02 (m, 2H), 2,16 (m, 2H), 3.28 (s, 3H), 3,46 (m, 4H), 4,8 (m, 1H), 6.57 (d, 1H), 6.92 (t, 1H), 7.07 (t, 1H), 7.73 (s, 1H), 7.90 (d, 1H), 8.01 (t, 1H), 8.33 (d, 1H), 9.40 (d, 1H).

The measurements of the NMR spectra were done with Bruker AVANCE DRX 500 Mhz and Bruker AVANCE III 400 Mhz.

The invention is defined in the following clauses:
1. A compound for use in the prevention or treatment of a disease by activating NLRP3 inflammasome, wherein the disease is selected from a group comprising infectious diseases or cancer, and wherein the compound is a compound of Formula (I) 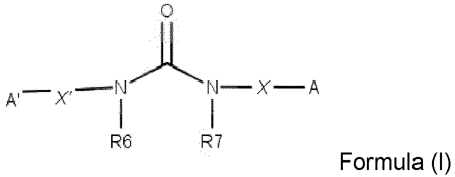 or a pharmaceutically acceceptable salt thereof, wherein:
A is an optionally substituted C₆-C₁₈ aryl; optionally substituted 5-20 membered heteroaryl; optionally substituted C₃-C₂₀ cycloalkyl; optionally substituted 3-20 membered cycloheteroalkyl, and wherein X and X' are each independently present or not, wherein X and X' are each independently an optionally substituted C₁-C₁₀ alkyl group;
R⁶ and R⁷ are each independently hydrogen, an optionally substituted C₁-C₁₀ alkyl or R⁶ and R⁷ when taken together form an optionally substituted five- or six-membered heterocyclic ring.
2. The compound for use according to clause 1, wherein X and X' are each independently - (CH₂)ₘ-, wherein each m is independently an integer from 0 to 4; and wherein R⁶ and R⁷ are each independently hydrogen, or C₁-C₁₀ alkyl, preferably wherein R⁶ and R⁷ are each hydrogen.
3. The compound for use according to any of clause 1 or 2, wherein A and A' are each independently optionally substituted benzene, imidazolidine, morpholine, piperazine, piperidine, pyrazolidine, pyrrolidone, quinuclidine, tetrahydrofuran, furan, pyrrolidine, pyridine, pyrazol, indolizine, quinoline, thiadiazol, oxadiazol, 1,2,4-oxadiazol, acridine, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, cyclopropyl, cyclobutyl, cyclopentyl, cyclohexyl; cyclopropenyl, cyclobutenyl, cyclopentenyl, cyclohexenyl; cyclobutadienyl, cyclohexadienyl, carbazole, benzimidazole, imidazopyridine, benzoisoxazole, tetrahydrothiophene, benzofuran, benzoxazole, or benzthiozole, preferably wherein A and A' are each independently optionally substituted quinoline, isoquinoline, indole, benzene, pyridine, pyrazole, pyrrol, furan or thiophene, more preferably wherein A and A' are each independently optionally substituted quinoline or benzene.
4.The compound for use according to any of clauses 2 to 3, wherein each m is independently an integer from 0 to 2, preferably 0 or 1, more preferably 0.
5. The compound for use according to any of clauses 1 to 4, wherein the compound is a compound of Formula (Ic): 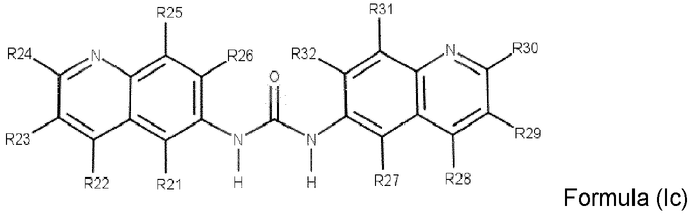 or 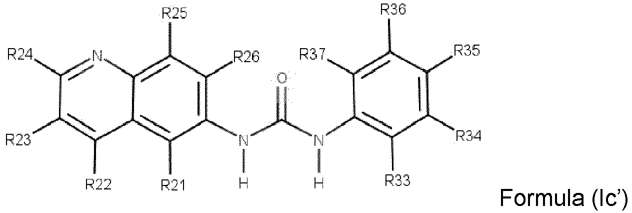
wherein R²¹ to R³⁷ are each independently selected from the group consisting of hydrogen, -F, -CI, -Br, -NO₂,-O-N=O, -N=O, -OH, -NH₂, -CH=NH, -N₃, -SH, -SO₃H, -SO₂H, -S(O)₂CH₃ -CN, - NC,-OCN, -NCO, -SCN, -NCS, -(P=O)(OH)₂, -O(P=O)(OH)₂, -(C=O)OH, -(C=O)NHOH, - NH(C=O)NH₂, -NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or - NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or preferably wherein R²¹ to R³⁷ are each independently selected from the group consisting of hydrogen, C₁-C₆ alkoxy, C₁-C₅ alkyl, C₁-C₅ alkyl(SO₂)C₁-C₅ alkyl, preferably -CH₂(SO₂)CH₃, - CH₂(SO₂)CH₂CH₃, -CH₂CH₂(SO₂)CH₂CH₃; -(SO₂)C₁-C₅ alkyl, preferably -(SO₂)CH₃, - (SO₂)CH₂CH₃, -CF₃, -F, -Cl, or -Br; or more preferably wherein R²¹ to R³⁷ are each hydrogen.
6. A compound as defined in clause 1, wherein the compound is a compound of Formula (Ia) 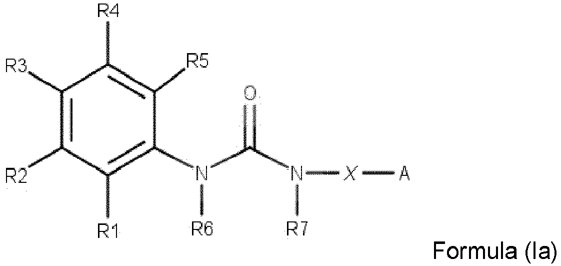 or a pharmaceutically acceceptable salt thereof, wherein:
A is preferably an optionally substituted C₆-C₁₈ aryl, and more preferably the following optionally substituted aryl group: 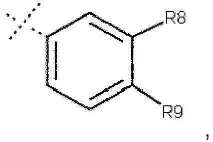 wherein X is a C₁-C₁₀ alkyl group or preferably X is not present; or preferably X is not present; or A is an optionally substituted five- or six-membered heterocyclic ring, and more preferably wherein A is 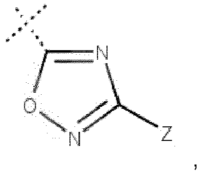
wherein Z is hydrogen, an optionally substituted C₁-C₁₀ alkyl, optionally substituted 2-20 membered heteroalkyl, optionally substituted C₃-C₂₀ cycloalkyl, optionally substituted 3-20 membered cycloheteroalkyl, optionally substituted C₆-C₁₈ aryl or optionally substituted 5-20 membered heteroaryl, preferably an optionally substituted C₆-C₁₄ aryl or 5-20 membered heteroaryl; and
wherein X is a C₁-C₁₀ alkyl group; and
wherein R¹, R², R³, R⁴, R⁵, R⁸ and R⁹ are each independently hydrogen, -F, -Cl, -Br, -NO₂, -ON=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, - NH(C=S)NH₂, -(C=O)NH₂,-CHO,-CHS, -OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², -NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, -CHF₂, -CF₃, -CHCl₂, and -CCl₃; or
R¹ and R², R² and R³, R³ and R⁴, R⁴ and R⁵, or R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring.
7. The compound according to clause 6, wherein A is the following optionally substituted aryl group: 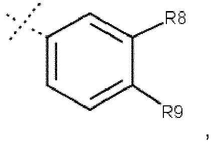 wherein R⁸ and R⁹ are as defined in clause 1 and X is not present; or
wherein X is a C₁-C₁₀ alkyl group; and wherein A is 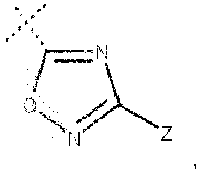 wherein Z is hydrogen, an optionally substituted C₁-C₁₀ alkyl, optionally substituted 2-20 membered heteroalkyl, optionally substituted C₆-C₁₈ aryl or optionally substituted 5-20 membered heteroaryl, preferably an optionally substituted C₆-C₁₄ aryl or 5-20 membered heteroaryl.
8. The compound according to any of clause 6 or 7, wherein R¹, R², R³, R⁴ and R⁵ are each independently hydrogen, C₁-C₆ alkoxy, C₁-C₅ alkyl, -F, -Cl, or -Br.
9.The compound according to any of clauses 6 to 8, wherein R⁶ and R⁷ are each independently hydrogen, or R⁶ and R⁷ when taken together form a heterocyclic ring selected from: 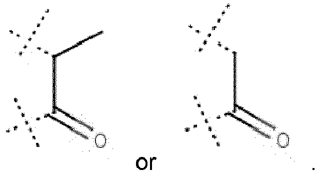 10.The compound of any of clauses 6 to 9, wherein R¹ is hydrogen, C₁-C₂ alkoxy, C₁-C₃ alkyl, R² is hydrogen; R³ is hydrogen or methyl; R⁴ is hydrogen or -F, -Cl, or -Br; and R⁵ is hydrogen, methyl or ethyl.
11. The compound according to any of clauses 6 to 10, wherein, R² and R³; or R², R³ and R⁵; or R², R³, R⁴ and R⁵ are each hydrogen.
12.The compound according to any of clauses 6 to 11, wherein the compound is a compound of Formula (Ia') 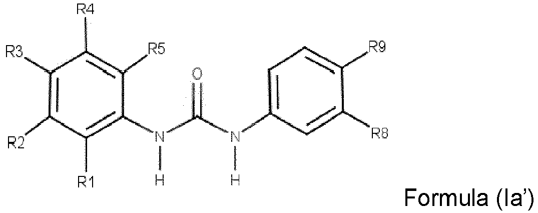 wherein R¹, R², R³, R⁴, R⁵, R⁸, and R⁹ are defined as in the preceding clauses.
13. The compound according to clause 12, wherein R¹, R², R³, R⁴ and R⁵ are each hydrogen.
14. The compound according to any of clauses 6 to 13, wherein R⁸ and R⁹ are each independently hydrogen, substituted C₁-C₁₀ alkyl, substituted 2-10 membered heteroalkyl, substituted C₂-C₁₄ aryl, substituted 5-20 membered heteroaryl, substituted five- or six-membered cycloheteroalkyl, -S(O)₂CH₃, -F, -Cl, -Br, -NHC(O)R¹¹, or -NHC(O)NHR¹¹, wherein R¹¹ is selected from C₁-C₅ alkyl, C₃-C₆ cycloalkyl, -CHF₂, -CF₃, -CHCl₂, -CCl₃; or five-membered heterocyclic ring; with the proviso that R⁸ is not identical to R⁹, or R⁸ and R⁹, when taken together form an optionally substituted five- or six-membered cycloalkyl, optionally substituted five- or six-membered cycloheteroalkyl or optionally substituted five or six-membered aryl, optionally substituted five- or six-membered heteroaryl, preferably the heterocyclic ring is selected from: 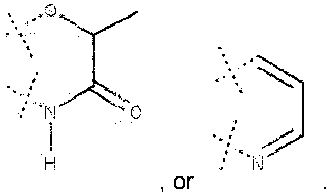 15. The compound according to any of clauses 6 to 14, wherein R⁸ and R⁹ are each independently defined as above, with the provisio that if one of R⁸ or R⁹ is -F, -Cl, or -Br, the other R⁸ or R⁹ is neither -F, -Cl, -Br nor hydrogen.
16. The compound according to any of clauses 6 to 15, wherein R⁹ is selected from C₁-C₃ alkyl, five-membered heterocyclic ring, -OCH₃, -S(O)₂CH₃, -NHC(O)R¹¹, or -NHC(O)NHR¹¹, wherein R¹¹ is selected from C₂-C₅ alkyl, C₃-C₆ cycloalkyl, -CHF₂, -CF₃, -CHCl₂, -CCl₃; or five-membered heterocyclic ring.
17. The compound according to any of clauses 6 to 16, wherein R⁸ is -S(O)₂CH₃, -NHC(O)R¹¹, or -NHC(O)NHR¹¹, wherein R¹¹ is selected from C₂-C₅ alkyl, C₃-C₅ cycloalkyl, -CHF₂; or five-membered heterocyclic ring.
18. The compound according to any of clauses 6 to 15, wherein R⁸ and R⁹, when taken together form a heterocyclic ring is selected from: 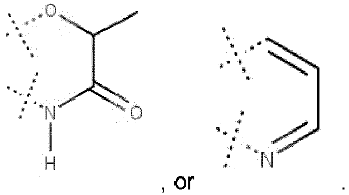 19. The compound according to any of clauses 6 to 11, wherein the -X-A group is 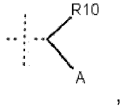 wherein R¹⁰ is C₁-C₃ alkyl, preferably -CH (CH₃)₂ or -(CH₂)ₙ-A, wherein n is an inter from 1-3, preferably 1,
and wherein A is 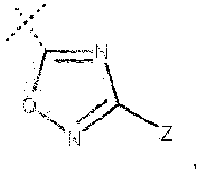 forming Formula (Ib) 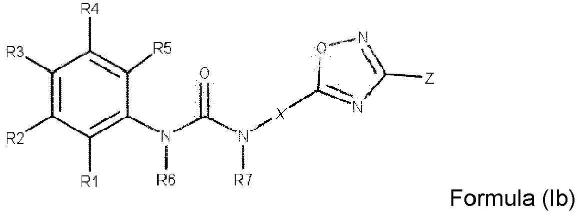 wherein Z is hydrogen, an optionally substituted C₁-C₁₀ alkyl, optionally substituted 2-20 membered heteroalkyl, optionally substituted C₆-C₁₈ aryl or optionally substituted 5-20 membered heteroaryl, preferably an optionally substituted C₆-C₁₄ aryl or 5-20 membered heteroaryl.
20. The compound of any of clauses 6 to 11 or 19, wherein Z is an optionally substituted C₆-C₁₄ aryl or 5-10 membered heteroaryl, preferably Z is pyridyl or substituted C₆ aryl, more preferably 3-pyridyl or para substituted phenyl, wherein the substituent is selected from the group consisting of -F, -Cl, and -CF₃.
21. The compound of clause 19 or 20, wherein R² and R⁵ are each hydrogen, R¹ is hydrogen or methoxy, R³, is hydrogen or methyl and R⁴ is hydrogen or -F, wherein at least one of R¹ to R⁵ is not hydrogen.
22. The compound of clause 6, wherein the compound is selected from the compounds delineated in Table 1.

**Table 1:**

| No. | Structure Formula (Ia') |
|---|---|
| L01.110 | 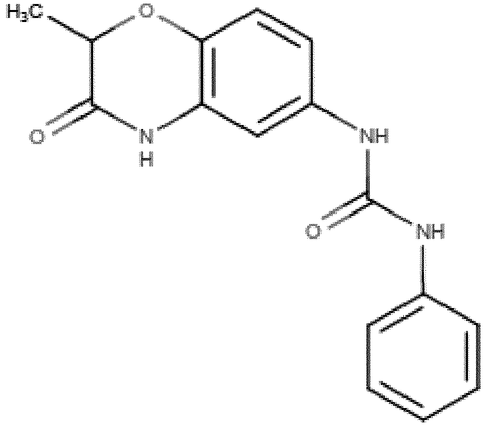 |
| L01.041 | 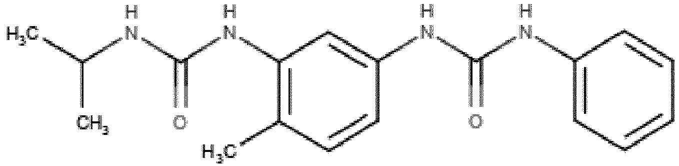 |
| L01.004 | 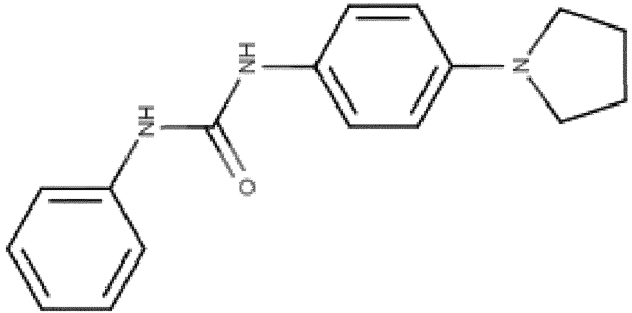 |
| L01.104 | 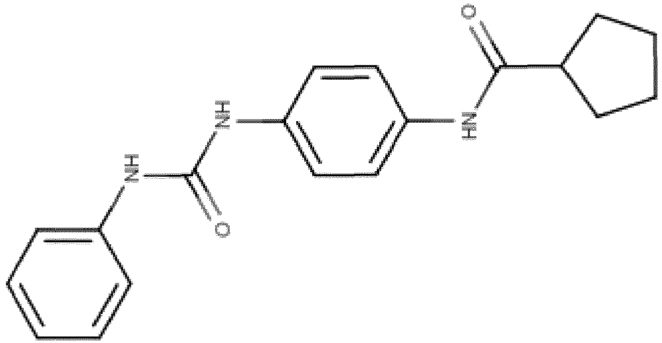 |
| L01.116 | 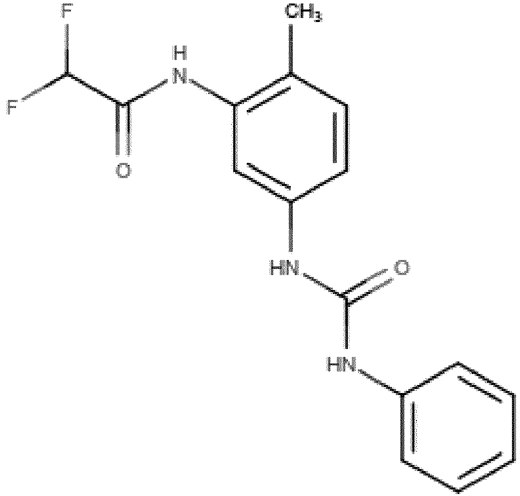 |
| L01.101 | 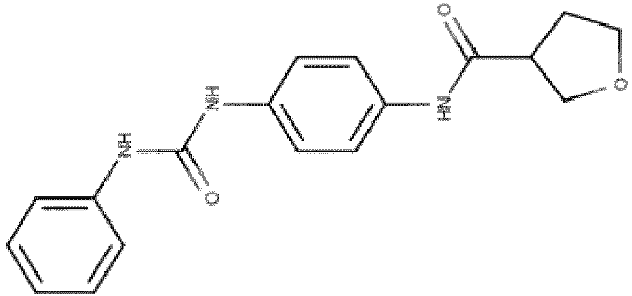 |
| L01.092 | 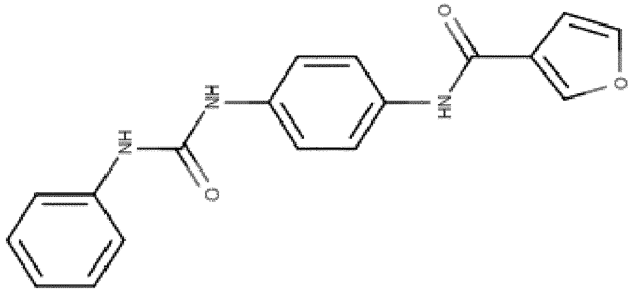 |
| L01.124 | 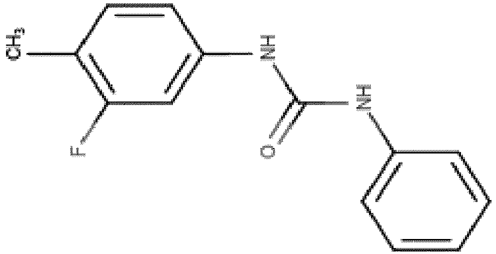 |
| L01.016 | 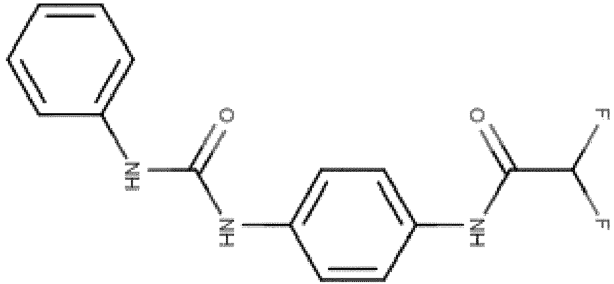 |
| L01.028 | 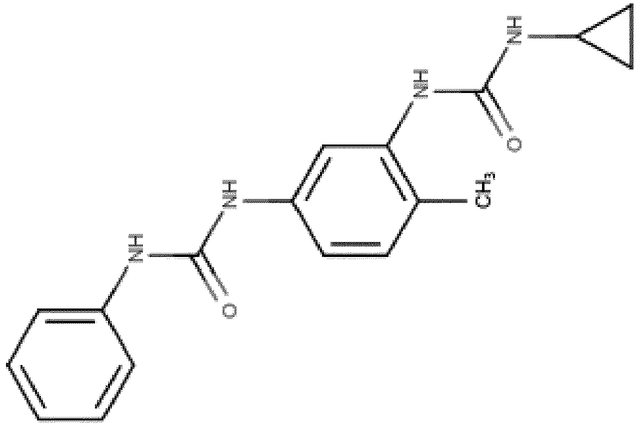 |
| L01.047 | 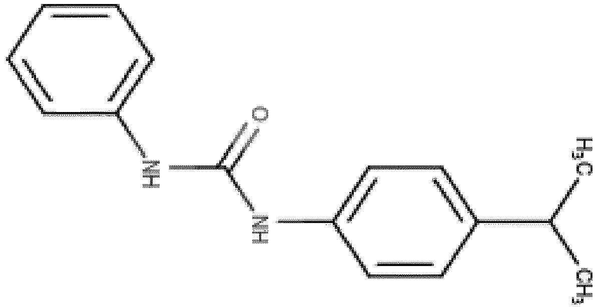 |
| L01.026 | 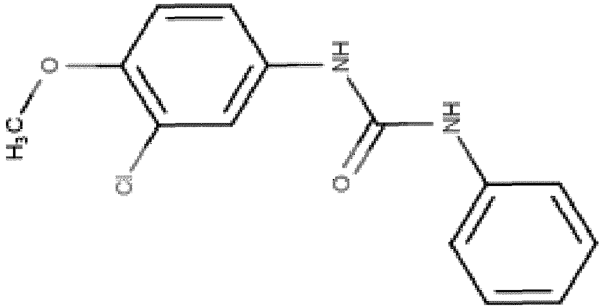 |
| L01.007 | 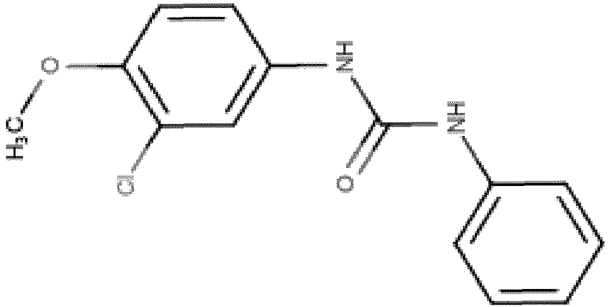 |
| EN22 | 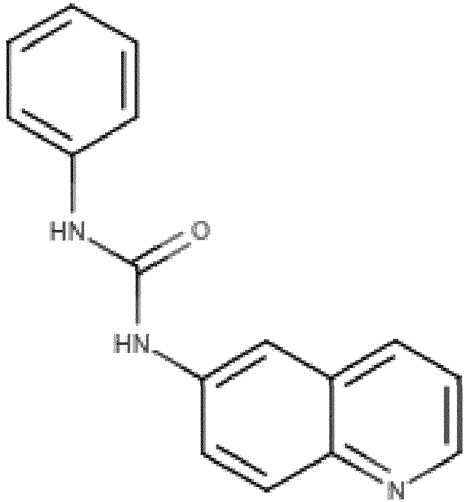 |
| L01.035 | 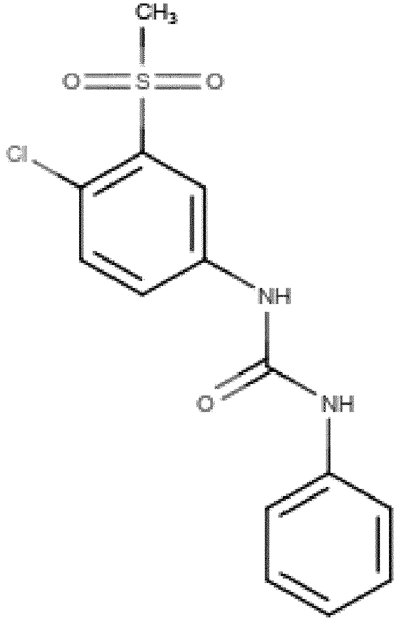 |
| EN23 | 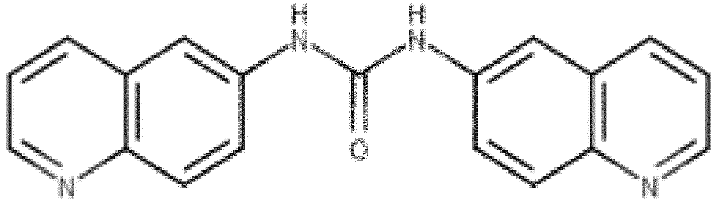 |

| No. | Structure Formula (Ib) |
|---|---|
| L01.113 | 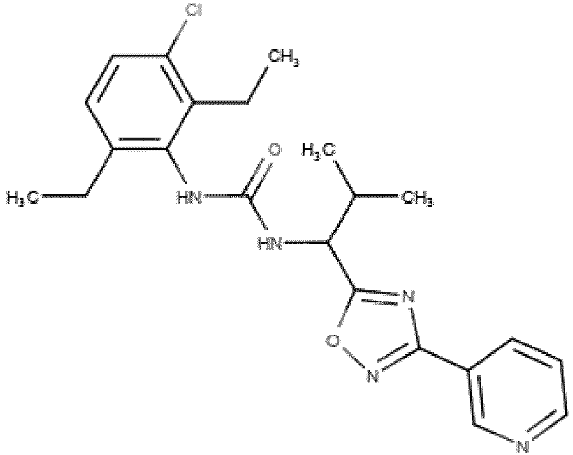 |
| L01.130 | 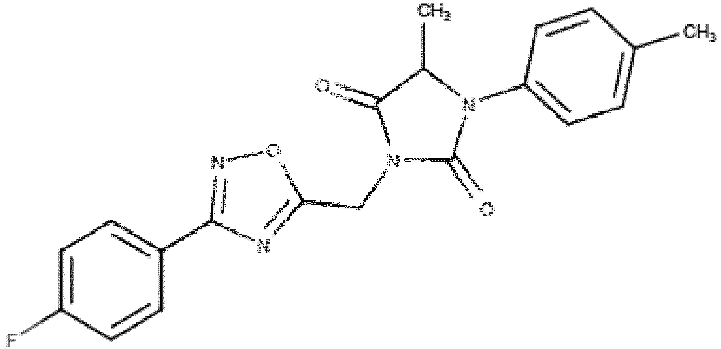 |
| L01.049 | 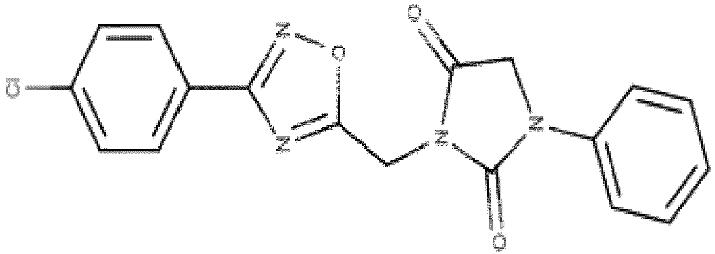 |
| EN21 | 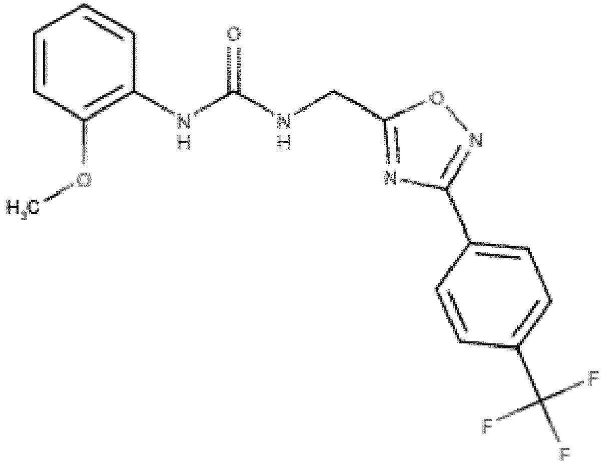 |

23. The compound of any of the preceding clauses, wherein the compound increases the activity of NLRP3 inflammasome, thereby preferably increasing the release of IL-1β of 0.1x10⁶ BMDCs upon administering the compound in a concentration of 50µM of the compound and incubation of 2 hours, wherein the increase is at least 0.1 ng/mL, 0.5 ng/mL, preferably 2 ng/mL, 10 ng/mL, 25 ng/mL, 50 ng/mL, and more preferably 4 ng/mL.
24. A compound of any of clauses 6 to 23 for use as a medicament.
25. A compound of any of clauses 6 to 24 for use in the prevention or treatment of a disease by activating NLRP3 inflammasome, wherein the disease is selected from a group comprising infectious diseases or cancer.
26. A method of treating cancer, comprising administering to a subject in need of such treatment an effective amount of a compound as claimed in any one of clauses 1 to 23.
27. Use of the compounds of clauses 1 to 23 for analyzing the activity of NLRP3 activation *in vitro.*
28. Use of the compounds of clauses 1 to 23 for boosting immune responses or breaking immune evasion in cancer diseases in a subject.

## Claims

1. A compound for use in the prevention or treatment of a disease by activating NLRP3 inflammasome, wherein the disease is selected from a group comprising infectious diseases or cancer, and
wherein the compound is a compound of Formula (I) 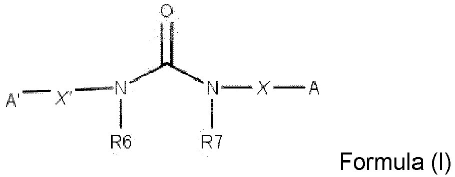 or a pharmaceutically acceceptable salt thereof, wherein:
A is an optionally substituted C₆-C₁₈ aryl; optionally substituted 5-20 membered heteroaryl; optionally substituted C₃-C₂₀ cycloalkyl; optionally substituted 3-20 membered cycloheteroalkyl,
and wherein X and X' are each independently present or not, wherein X and X' are each independently an optionally substituted C₁-C₁₀ alkyl group;
R⁶ and R⁷ are each independently hydrogen, an optionally substituted C₁-C₁₀ alkyl or R⁶ and R⁷ when taken together form an optionally substituted five- or six-membered heterocyclic ring.

2. The compound for use according to claim 1, wherein X and X' are each independently -(CH₂)ₘ-, wherein each m is independently an integer from 0 to 4; and wherein
R⁶ and R⁷ are each independently hydrogen, or C₁-C₁₀ alkyl, preferably wherein R⁶ and R⁷ are each hydrogen.

3. The compound for use according to any of claim 1 or 2, wherein A and A' are each independently optionally substituted benzene, imidazolidine, morpholine, piperazine, piperidine, pyrazolidine, pyrrolidone, quinuclidine, tetrahydrofuran, furan, pyrrolidine, pyridine, pyrazol, indolizine, quinoline, thiadiazol, oxadiazol, 1,2,4-oxadiazol, acridine, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, thiophene, triazole, xanthene, cyclopropyl, cyclobutyl, cyclopentyl, cyclohexyl; cyclopropenyl, cyclobutenyl, cyclopentenyl, cyclohexenyl; cyclobutadienyl, cyclohexadienyl, carbazole, benzimidazole, imidazopyridine, benzoisoxazole, tetrahydrothiophene, benzofuran, benzoxazole, or benzthiozole,
preferably wherein A and A' are each independently optionally substituted quinoline, isoquinoline, indole, benzene, pyridine, pyrazole, pyrrol, furan or thiophene,
more preferably wherein A and A' are each independently optionally substituted quinoline or benzene.

4. The compound for use according to any of claims 1 to 3, wherein the compound is a compound of Formula (Ic): 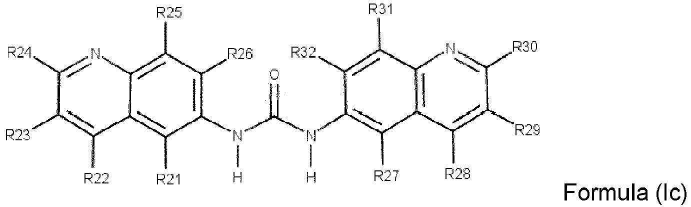 or 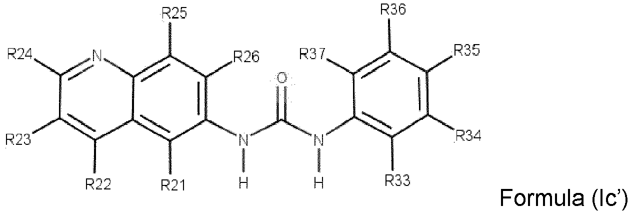
wherein R²¹ to R³⁷ are each independently selected from the group consisting of hydrogen, -F, -Cl, -Br, -NO₂,-O-N=O, -N=O, -OH, -NH₂, -CH=NH, -N₃, -SH, -SO₃H, - SO₂H, -S(O)₂CH₃ -CN, -NC,-OCN, -NCO, -SCN, -NCS, -(P=O)(OH)₂, -O(P=O)(OH)₂, -(C=O)OH, -(C=O)NHOH, -NH(C=O)NH₂, -NH(C=S)NH₂, -(C=O)NH₂,-CHO,-CHS, - OCHO, -NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl, optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², - NHC(O)R¹¹, and -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, - CHF₂, -CF₃, -CHCl₂, and -CCl₃; or
preferably wherein R²¹ to R³⁷ are each independently selected from the group consisting of hydrogen, C₁-C₆ alkoxy, C₁-C₅ alkyl, C₁-C₅ alkyl(SO₂)C₁-C₅ alkyl, preferably -CH₂(SO₂)CH₃, -CH₂(SO₂)CH₂CH₃, -CH₂CH₂(SO₂)CH₂CH₃; -(SO₂)C₁-C₅ alkyl, preferably -(SO₂)CH₃, -(SO₂)CH₂CH₃, -CF₃, -F, -Cl, and -Br; or
more preferably wherein R²¹ to R³⁷ are each hydrogen.

5. A compound as defined in claim 1, wherein the compound is a compound of Formula (Ia) 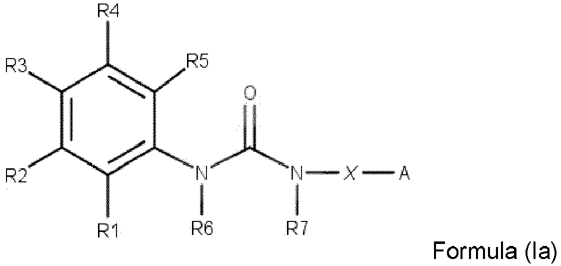 or a pharmaceutically acceceptable salt thereof, wherein:
A is preferably an optionally substituted C₆-C₁₈ aryl, and more preferably the following optionally substituted aryl group: 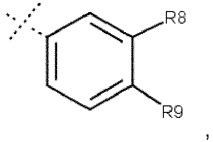 wherein X is a C₁-C₁₀ alkyl group or preferably X is not present; or preferably X is not present;
or A is an optionally substituted five- or six-membered heterocyclic ring, and more preferably wherein A is 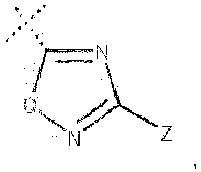
wherein Z is hydrogen, an optionally substituted C₁-C₁₀ alkyl, optionally substituted 2-20 membered heteroalkyl, optionally substituted C₃-C₂₀ cycloalkyl, optionally substituted 3-20 membered cycloheteroalkyl, optionally substituted C₆-C₁₈ aryl or optionally substituted 5-20 membered heteroaryl, preferably an optionally substituted C₆-C₁₄ aryl or 5-20 membered heteroaryl; and
wherein X is a C₁-C₁₀ alkyl group; and
wherein R¹, R², R³, R⁴, R⁵, R⁸ and R⁹ are each independently hydrogen, -F, -Cl, -Br, -NO₂,-O-N=O, -N=O, -OH, -NH₂, -CH=NH, -N₃, -SH, -SO₃H, -SO₂H, -S(O)₂CH₃ -CN, -NC,-OCN, -NCO, -SCN, -NCS, -(P=O)(OH)₂, -O(P=O)(OH)₂, -(C=O)OH, - (C=O)NHOH, -NH(C=O)NH₂, -NH(C=S)NH₂, -(C=O)NH₂,-CHO,-CHS, -OCHO, - NCHO, -B(OH)₂, optionally substituted C₁-C₁₀ alkyl, optionally substituted C₁-C₁₀ alkoxy, optionally substituted C₂-C₁₀ alkenyl, optionally substituted C₃-C₂₀ cycloalkyl, optionally substituted C₃-C₂₀ cycloalkyl C₁-C₁₀ alkyl, optionally substituted 3-20 membered cycloheteroalkyl or 3-20 membered cycloheteroalkyl C₁-C₁₀ alkyl,
optionally substituted C₆-C₁₈ aryl, optionally substituted C₆-C₁₈ aryl C₁-C₁₀ alkyl, optionally substituted 5-20 membered heteroaryl, optionally substituted 5-20 membered heteroaryl C₁-C₁₀ alkyl, (C=O)R¹¹, (C=O)OR¹¹, (C=O)SR¹¹, (C=O)NR¹¹R¹², (SO₂)R¹¹, (SNO)R¹¹, (P=O)R¹¹R¹², (P=O)OR¹¹OR¹² or C(NR¹¹)R¹², - NHC(O)R¹¹, or -NHC(O)NHR¹¹,
wherein R¹¹ and R¹² are independently selected from the group consisting of optionally substituted C₁-C₁₀ alkyl, optionally substituted C₂-C₁₀ alkenyl, optionally substituted C₃-C₂₀ cycloalkyl, optionally substituted C₆-C₁₈ aryl, optionally substituted 5-20 membered heteroaryl, optionally substituted 2-20 membered heteroalkyl, - CHF₂, -CF₃, -CHCl₂, and -CCl₃; or
R¹ and R², R² and R³, R³ and R⁴, R⁴ and R⁵, or R⁸ and R⁹, when taken together form a optionally substituted five- or six-membered cycloalkyl, optionally substituted five- or six-membered cycloheteroalkyl, optionally substituted five- or six-membered aryl, or optionally substituted five- or six-membered heteroaryl, and
R⁶ and R⁷ are each independently hydrogen, an optionally substituted C₁-C₁₀ alkyl or R⁶ and R⁷ when taken together form an optionally substituted five- or six-membered heterocyclic ring.

6. The compound according to any of claim 5, wherein R¹, R², R³, R⁴ and R⁵ are each independently hydrogen, C₁-C₆ alkoxy, C₁-C₅ alkyl, -F, -Cl, or -Br.

7. The compound according to any of claim 5 or 6, wherein R⁶ and R⁷ are each independently hydrogen, or R⁶ and R⁷ when taken together form a heterocyclic ring selected from:

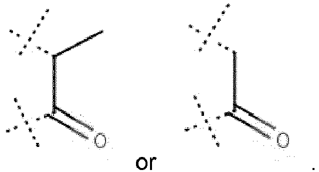

8. The compound according to claims 5 to 7, wherein R¹, R², R³, R⁴ and R⁵ are each hydrogen and/or wherein R⁸ and R⁹ are each independently hydrogen, substituted C₁-C₁₀ alkyl, substituted 2-10 membered heteroalkyl, substituted C₂-C₁₄ aryl, substituted 5-20 membered heteroaryl, substituted five- or six-membered cycloheteroalkyl, -S(O)₂CH₃, -F, -Cl, -Br, -NHC(O)R¹¹, or -NHC(O)NHR¹¹,
wherein R¹¹ is selected from C₁-C₅ alkyl, C₃-C₆ cycloalkyl, -CHF₂, -CF₃, -CHCl₂, - CCl₃; or five-membered heterocyclic ring;
with the proviso that R⁸ is not identical to R⁹, or
R⁸ and R⁹, when taken together form an optionally substituted five- or six-membered cycloalkyl, optionally substituted five- or six-membered cycloheteroalkyl or optionally substituted five or six-membered aryl, optionally substituted five- or six-membered heteroaryl, preferably the heterocyclic ring is selected from:

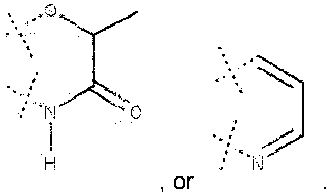

9. The compound according to any of claims 5 to 8, wherein R⁸ and R⁹ are each independently defined as above, with the provisio that if one of R⁸ or R⁹ is -F, -Cl, or -Br, the other R⁸ or R⁹ is neither -F, -Cl, -Br nor hydrogen.

10. The compound according to any of claims 5 to 9, wherein
R⁹ is selected from C₁-C₃ alkyl, five-membered heterocyclic ring, -OCH₃, -S(O)₂CH₃, - NHC(O)R¹¹, or -NHC(O)NHR¹¹, and
wherein R¹¹ is selected from C₂-C₅ alkyl, C₃-C₆ cycloalkyl, -CHF₂, -CF₃, - CHCl₂, -CCl₃; or five-membered heterocyclic ring; and/or
wherein R⁸ is -S(O)₂CH₃, -NHC(O)R¹¹, or -NHC(O)NHR¹¹, and
wherein R¹¹ is selected from C₂-C₅ alkyl, C₃-C₅ cycloalkyl, -CHF₂; or five-membered heterocyclic ring;
or wherein R⁸ and R⁹, when taken together form a heterocyclic ring is selected from:

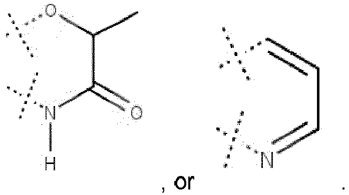

11. The compound according to any of claims 5 to 7, wherein the -X-A group is (CH₃)₂ 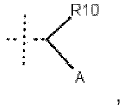 wherein R¹⁰ is C₁-C₃ alkyl, preferably -CH or -(CH₂)ₙ-A, wherein n is an inter from 1-3, preferably 1, and wherein A is 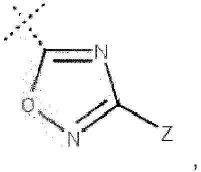 forming Formula (Ib) 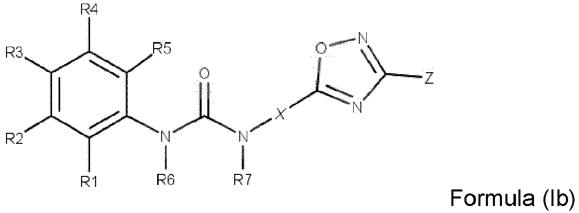 wherein Z is hydrogen, an optionally substituted C₁-C₁₀ alkyl, optionally substituted 2-20 membered heteroalkyl, optionally substituted C₆-C₁₈ aryl or optionally substituted 5-20 membered heteroaryl, preferably an optionally substituted C₆-C₁₄ aryl or 5-20 membered heteroaryl, more preferably wherein Z is an optionally substituted C₆-C₁₄ aryl or 5-10 membered heteroaryl, preferably a pyridyl or substituted C₆ aryl, more preferably 3-pyridyl or para substituted phenyl, wherein the substituent is selected from the group consisting of -F, -Cl, and -CF₃.

12. The compound of claim 5, wherein the compound is selected from the compounds delineated in Table 1.
**Table 1:**
| No. | Structure Formula (Ia') |
|---|---|
| L01.110 | 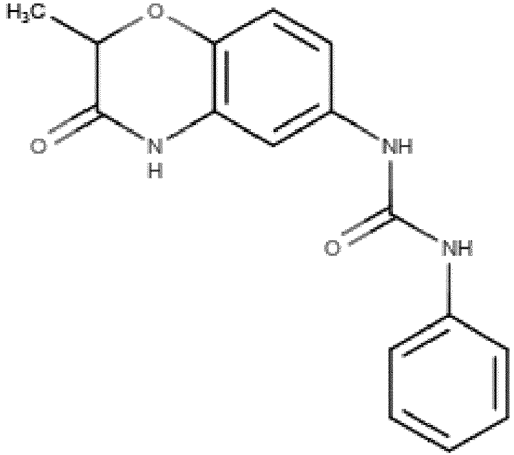 |
| L01.041 | 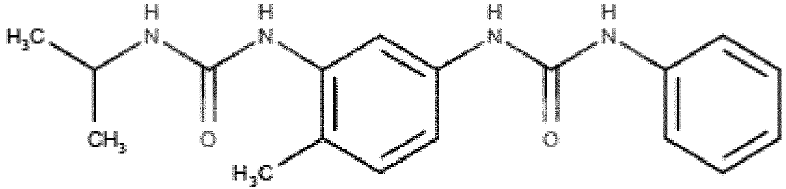 |
| L01.004 | 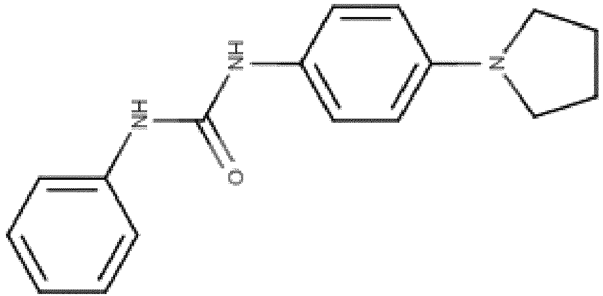 |
| L01.104 | 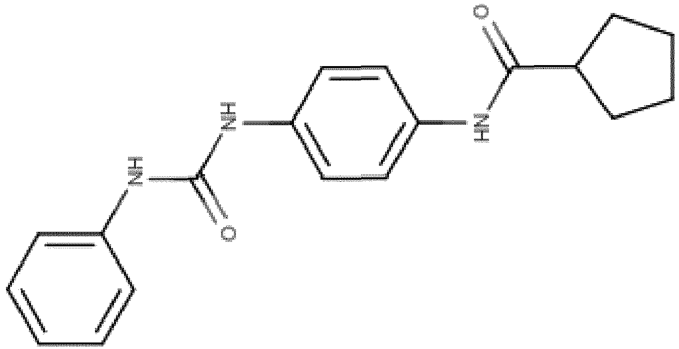 |
| L01.116 | 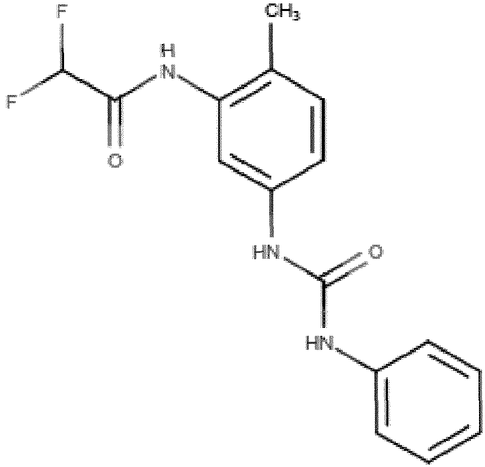 |
| L01.101 | 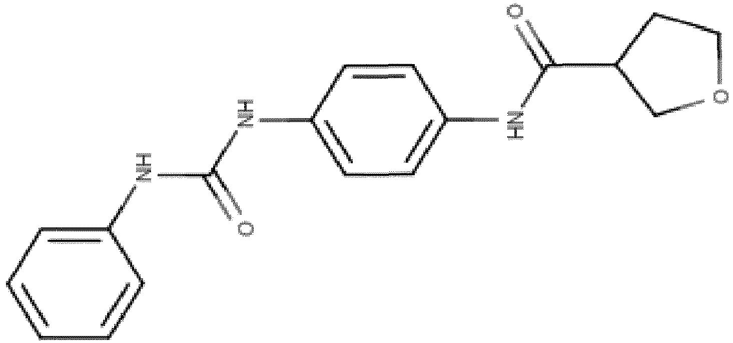 |
| L01.092 | 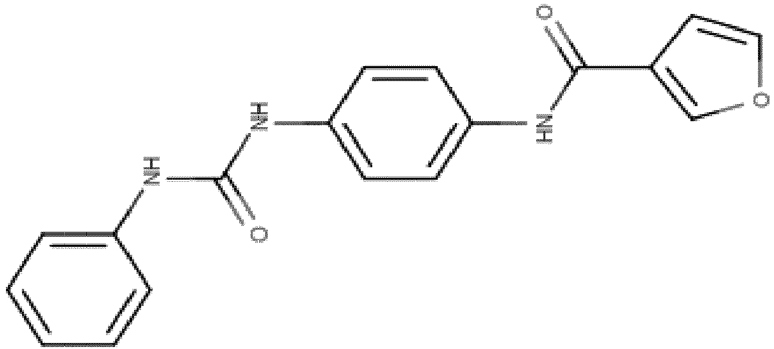 |
| L01.124 | 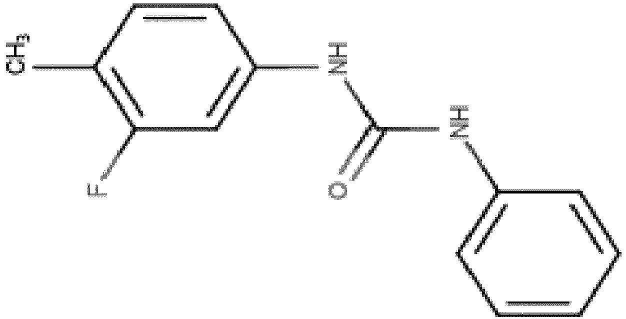 |
| L01.016 | 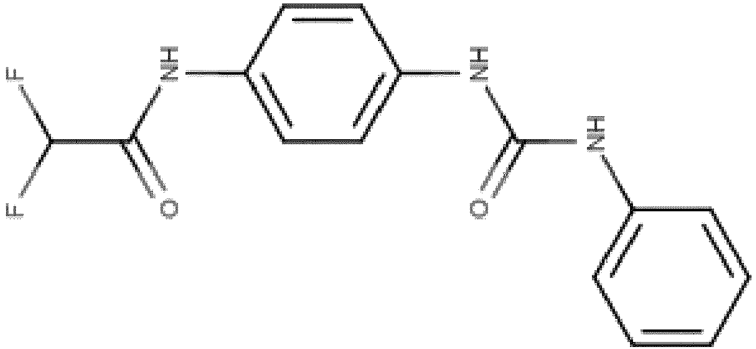 |
| L01.028 | 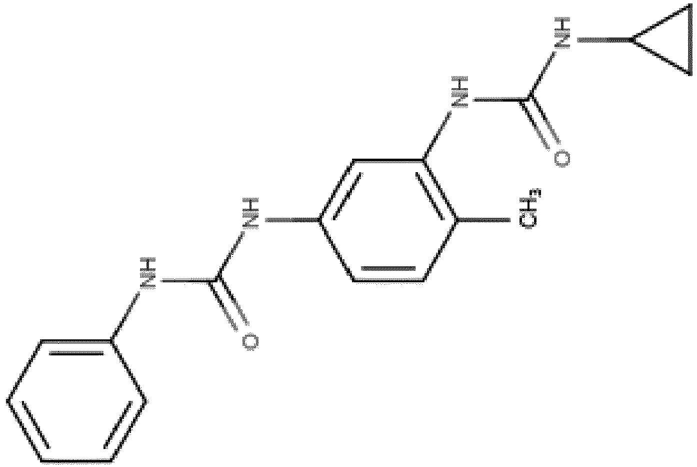 |
| L01.047 | 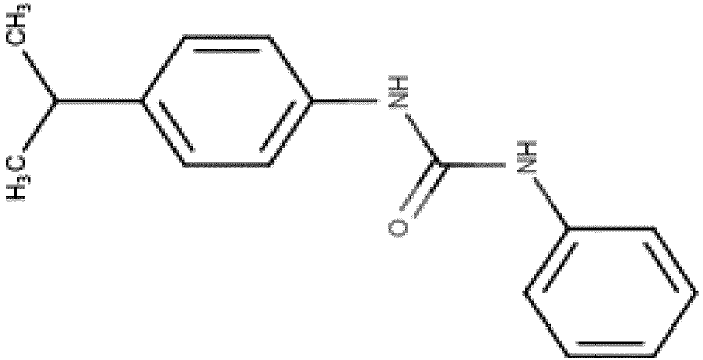 |
| L01.026 | 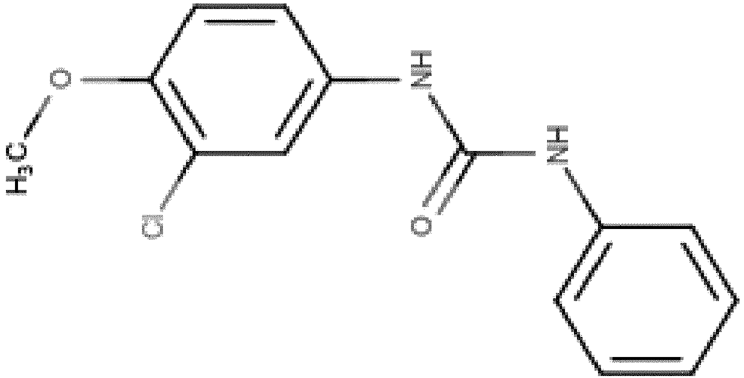 |
| L01.007 | 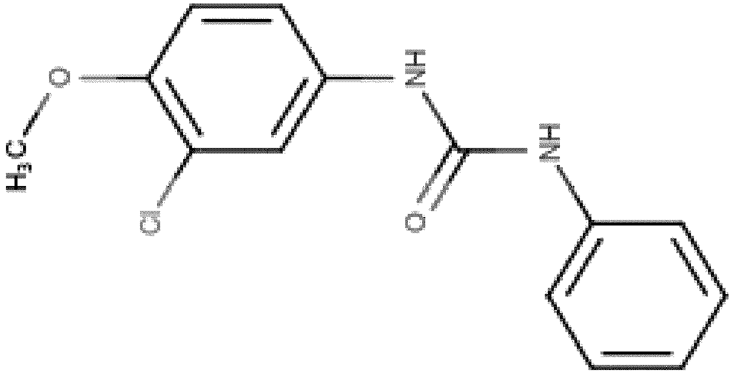 |
| EN22 | 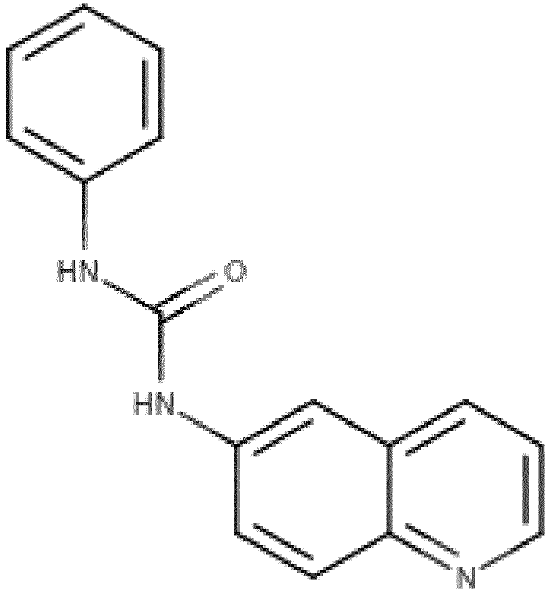 |
| L01.035 | 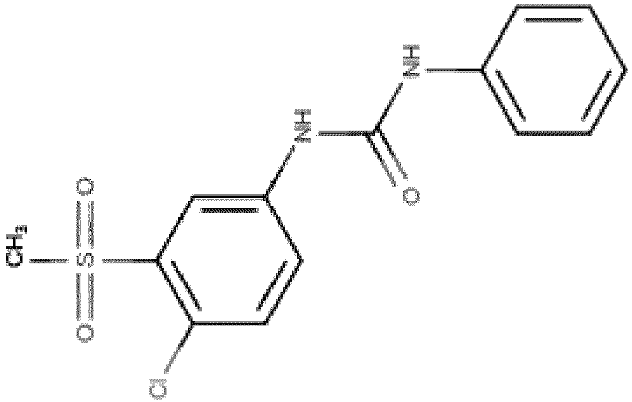 |
| EN23 | 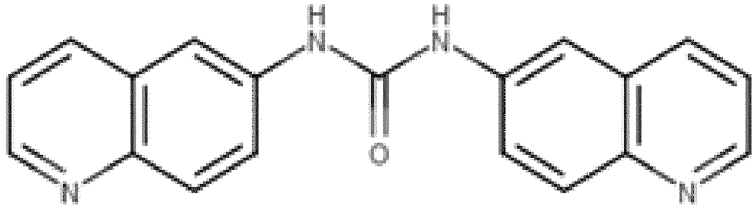 |
| No. | Structure Formula (Ib) |
|---|---|
| L01.113 | 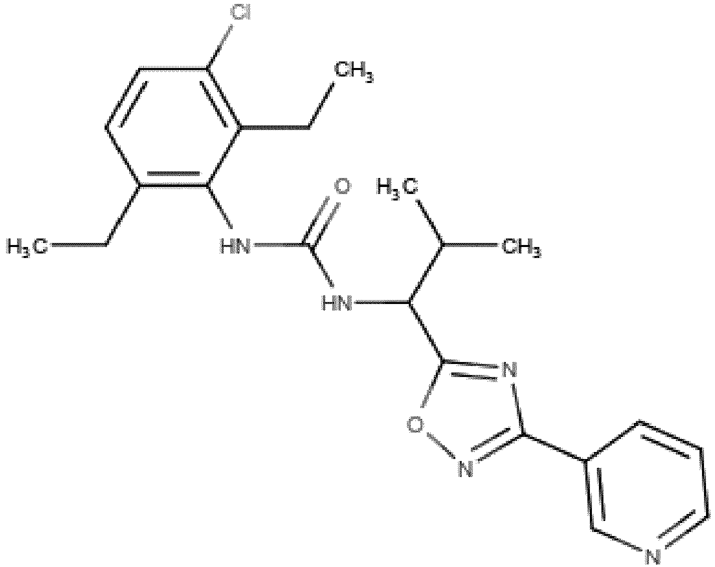 |
| L01.130 | 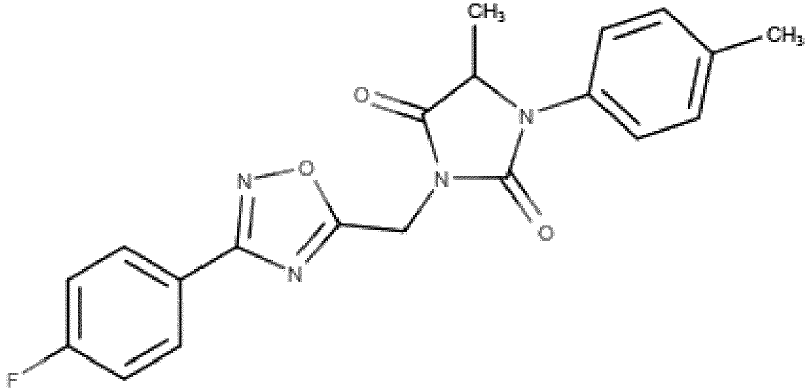 |
| L01.049 | 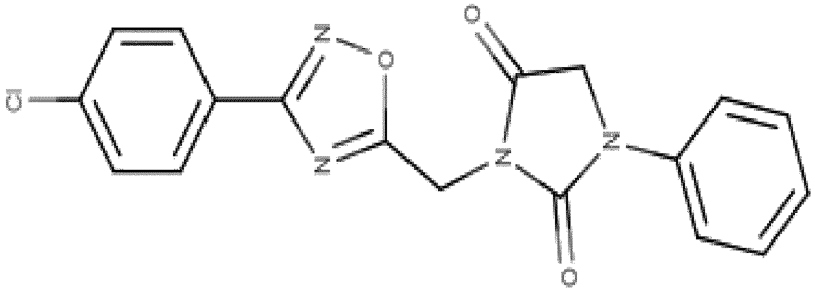 |
| EN21 | 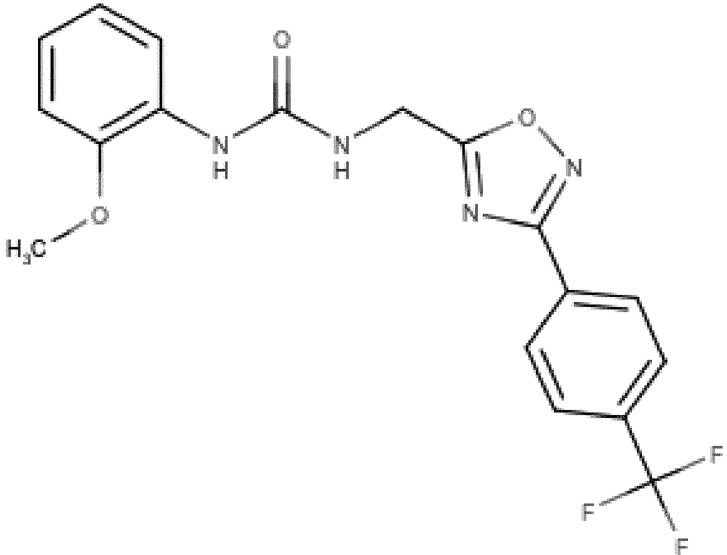 |

13. A compound of any of claims 5 to 12 for use as a medicament.

14. A compound of any of claims 5 to 13 for use in the prevention or treatment of a disease by activating NLRP3 inflammasome, wherein the disease is selected from a group comprising infectious diseases or cancer, or for use for boosting immune responses or breaking immune evasion in cancer diseases in a subject, or for use as an adjuvant in the context of vaccination to boost specific immune responses by activating NLRP3 inflammasome.

15. Use of the compounds of claims 1 to 12 for analyzing the activity of NLRP3 activation *in vitro.*
